(19) Europäisches Patentamt
European Patent Office
Office européen des brevets

(11) EP 4 524 140 A1

(12) EUROPEAN PATENT APPLICATION
published in accordance with Art. 153(4) EPC

(43) Date of publication:
19.03.2025 Bulletin 2025/12

(21) Application number: 23802728.8

(22) Date of filing: 04.05.2023

(51) International Patent Classification (IPC):
*C07D 491/22* (2006.01) *C07D 491/147* (2006.01)
*A61K 31/437* (2006.01) *A61P 35/00* (2006.01)

(52) Cooperative Patent Classification (CPC):
A61K 31/437; A61P 35/00; C07D 491/147; C07D 491/22

(86) International application number:
PCT/CN2023/092019

(87) International publication number:
WO 2023/216956 (16.11.2023 Gazette 2023/46)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 13.05.2022 CN 202210521215

(71) Applicant: Sichuan Kelun-Biotech Biopharmaceutical Co., Ltd.
Chengdu, Sichuan 611138 (CN)

(72) Inventors:
- TIAN, Qiang
Chengdu, Sichuan 611138 (CN)
- ZHANG, Yitao
Chengdu, Sichuan 611138 (CN)
- YE, Jian
Chengdu, Sichuan 611138 (CN)
- LI, Yile
Chengdu, Sichuan 611138 (CN)
- SONG, Hongmei
Chengdu, Sichuan 611138 (CN)
- GE, Junyou
Chengdu, Sichuan 611138 (CN)

(74) Representative: Cabinet Nony
11 rue Saint-Georges
75009 Paris (FR)

# (54) CAMPTOTHECIN COMPOUND, PREPARATION METHOD THEREFOR AND USE THEREOF

(57) The present invention relates to a camptothecin compound with anti-tumor activity, a preparation method therefor and use thereof. In particular, the present invention relates to a compound of the following formula or a pharmaceutically acceptable form thereof, a pharmaceutical composition thereof, a preparation method thereof and use thereof. The compound can be used as a medicine for treating diseases in abnormal cell proliferation,

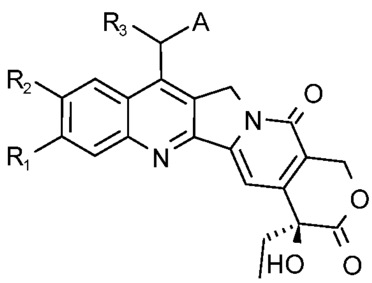
.

EP 4 524 140 A1

## Description

The present application is based on CN application with application number 202210521215.2 and the filing date of May 13, 2022, and claims its priority. The disclosure of the CN application is hereby incorporated into the present application in its entirety.

## Technical Field

The present invention relates to a class of camptothecin compounds with anti-tumor activity and conjugates thereof, as well as preparation methods therefor and medical applications thereof.

## Background Art

Camptothecin (CPT, Formula 1) is a compound having a five-ring quinoline core isolated from *Camptotheca acuminata*, a plant of the *Davidia* family. It consists of quinoline rings A and B, pyrrole ring C, pyridone ring D, and $\alpha$-hydroxylactone ring E, in which the 20-position is in S configuration (see, the structural represented by the following Formula). It was introduced into clinical practice in the early 1970s due to its excellent anti-cancer activity, but the clinical trials were terminated later due to severe side effects such as diarrhea and hemorrhagic cystitis.

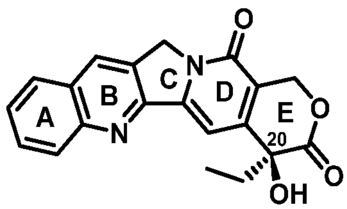

Camptothecin

Research data shows that camptothecin can form a ternary complex with cellular DNA topoisomerase I, thereby inhibiting the unwinding of DNA, causing DNA replication to be blocked, and thus causing cell death (Cancer Res. 1989, 49, 6365). Camptothecin and derivatives thereof have strong anti-tumor activity in animal models of lung cancer, breast cancer, colorectal cancer, ovarian cancer, etc. (Nature Review Cancer. 2006, 6, 789).

At present, several camptothecin drugs have been approved for use in tumor treatment (Med. Res. Rev. 2015, 35, 753). Irinotecan is used for the treatment of colorectal cancer; Topotecan is used for the treatment of ovarian cancer; Belotecan is used for the treatment of ovarian cancer and small cell lung cancer. Camptothecin derivatives further include Exatecan, Rubitecan, Karenitecan, Diflomotecan, Lurtotecan, Gimatecan, Namitecan, Simmitecan, Silatecan, Chimmitecan, Elomotecan, etc.

Camptothecin drugs or derivatives often have blood toxicity caused by bone marrow suppression, such as neutropenia, leukopenia, thrombocytopenia, anemia, etc., as well as gastrointestinal side effects, such as nausea, vomiting, diarrhea, etc. Clinical studies have found that measures to improve the safety and effectiveness of camptothecin compounds include improving their pharmacokinetic properties, adjusting activity, reducing dosage, or using their conjugates to form antibody-conjugated drugs with antibodies. Therefore, it still has high clinical demand and application value to develop camptothecin compounds and conjugates thereof with novel structures, enhanced effectiveness and improved safety.

## Contents of the present invention

The present invention provides a novel camptothecin compound and conjugate thereof, the camptothecin compound has good anti-tumor activity, excellent permeability, low excretion rate, and good metabolic stability, and is promising to be used for the treatment of tumor diseases; and its conjugate has broad application prospects as antibody-conjugated drugs.

A first aspect of the present invention provides a compound or a pharmaceutically acceptable salt, ester, stereoisomer, polymorph, solvate, N-oxide, isotopically labeled compound, metabolite and prodrug thereof, wherein the compound has the following structure:

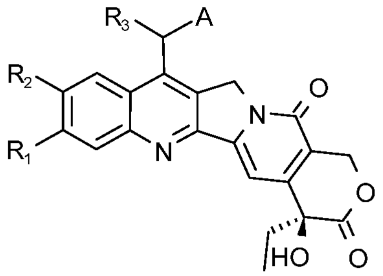

Formula (I)

wherein,

$R_1$ is selected from the group consisting of hydrogen, fluorine, and chlorine;

$R_2$ is selected from the group consisting of hydrogen, methyl, fluorine, chlorine, and hydroxyl; or $R_1$ and $R_2$ together with the carbon atom to which they are attached are connected to form a 5-to 6-membered oxygen-containing heterocycle;

$R_3$ is selected from the group consisting of hydrogen, $C_{1-6}$ alkyl, deuterated $C_{1-6}$ alkyl, $C_{1-6}$ haloalkyl, $C_{1-6}$ alkoxyalkyl, $C_{3-6}$ cycloalkyl, and 3- to 6-membered heterocyclyl, or $R_3$ is connected to an adjacent benzene ring carbon atom to form a 6-membered carbocycle;

A is selected from the group consisting of

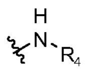, 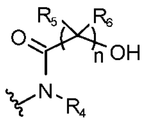, 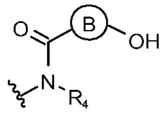, 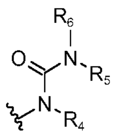,

and

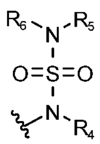;

Ring B is a 3- to 6-membered carbocycle or a 3- to 6-membered heterocycle;

$R_4$ is selected from the group consisting of hydrogen, $C_{1-6}$ alkyl, $C_{1-6}$ haloalkyl, $C_{1-6}$ alkoxyalkyl, $C_{3-6}$ cycloalkyl, and 3- to 6-membered heterocyclyl;

$R_5$ and $R_6$ are each independently selected from the group consisting of hydrogen, $C_{1-6}$ alkyl, $C_{2-6}$ alkenyl, $C_{3-6}$ cycloalkyl, and $C_{1-6}$ haloalkyl; or $R_5$ or $R_6$ together with the carbon atom to which they are attached form a 3- to 6-membered ring;

or, $R_4$ and $R_5$ are connected to form a 4- to 6-membered ring;

n = 1 to 6, such as 1, 2, 3, 4, 5 or 6.

In some embodiments, $R_3$ is connected to an adjacent benzene ring carbon atom to form a 6-membered carbocycle, and the carbon atom is the carbon atom at the meta position of $R_1$ and at the ortho position of $R_2$.
In some embodiments, in Formula (I),

$R_1$ is selected from the group consisting of hydrogen, fluorine and chlorine;

$R_2$ is selected from the group consisting of hydrogen, methyl, fluorine, chlorine and hydroxyl; or $R_1$ and $R_2$ together with the carbon atom to which they are attached are connected to form a 5-to 6-membered oxygen-containing heterocycle;

$R_3$ is selected from the group consisting of hydrogen, $C_{1-6}$ alkyl, $C_{1-6}$ haloalkyl, and $C_{1-6}$ alkoxyalkyl, or connected to an adjacent benzene ring carbon atom to form a 6-membered carbocycle;

A is selected from the group consisting of

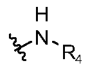 , 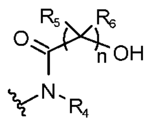 , 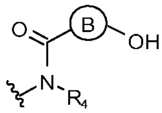 , 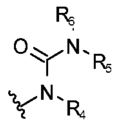

and

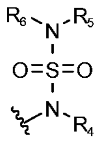 ;

Ring B is a 3- to 6-membered carbocycle or a 3- to 6-membered heterocycle;

$R_4$ is selected from the group consisting of hydrogen, $C_{1-6}$ alkyl, $C_{1-6}$ haloalkyl, $C_{1-6}$ alkoxyalkyl, $C_{3-6}$ cycloalkyl and 3- to 6-membered heterocyclyl;

$R_5$ and $R_6$ are each independently selected from the group consisting of hydrogen, $C_{1-6}$ alkyl, $C_{3-6}$ cycloalkyl, and $C_{1-6}$ haloalkyl; or $R_5$ or $R_6$ together with the carbon atom to which they are attached form a 3- to 6-membered ring;

or, $R_4$ and $R_5$ are connected to form a 4- to 6-membered ring;

n = 1 or 2.

In some embodiments, the compound has the structure of Formula (II):

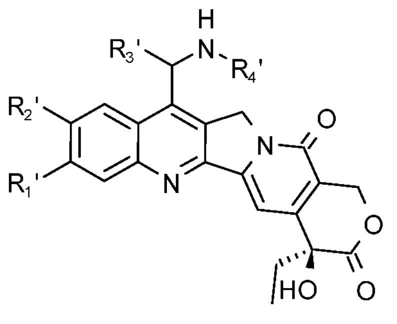

Formula (II)

in Formula (II),

$R_1$' is selected from the group consisting of hydrogen, fluorine, and chlorine;

$R_2$' is selected from the group consisting of methyl, fluorine, chlorine, and hydroxyl;

$R_3$' is selected from the group consisting of hydrogen, $C_{1-6}$ alkyl, deuterated $C_{1-6}$ alkyl, $C_{1-6}$ haloalkyl, $C_{1-6}$ alkoxyalkyl, $C_{3-6}$ cycloalkyl, and 3- to 6-membered heterocyclyl; $R_3$' is preferably hydrogen, $C_{1-6}$ alkyl, deuterated $C_{1-6}$ alkyl, $C_{1-6}$

haloalkyl, or $C_{1-6}$ alkoxyalkyl;

$R_4$' is selected from the group consisting of hydrogen, $C_{1-6}$ alkyl, and $C_{1-6}$ haloalkyl; $R_4$' is preferably hydrogen;

when $R_1$' is fluorine and $R_2$' is methyl, $R_3$' and $R_4$' are not hydrogen at the same time; and

when $R_1$' is fluorine and $R_2$' is methyl, $R_4$' is not $C_{1-6}$ alkyl.

In some embodiments, in Formula (II),

$R_1$' is selected from the group consisting of hydrogen, fluorine and chlorine;

$R_2$' is selected from the group consisting of methyl, fluorine, chlorine and hydroxyl;

$R_3$' is selected from the group consisting of hydrogen, $C_{1-6}$ alkyl, $C_{1-6}$ haloalkyl, $C_{1-6}$ alkoxyalkyl, $C_{3-6}$ cycloalkyl and 3- to 6-membered heterocyclyl; $R_3$' is preferably hydrogen, $C_{1-6}$ alkyl, $C_{1-6}$ haloalkyl or $C_{1-6}$ alkoxyalkyl;

$R_4$' is selected from the group consisting of hydrogen, $C_{1-6}$ alkyl and $C_{1-6}$ haloalkyl; $R_4$' is preferably hydrogen;

when $R_1$' is fluorine and $R_2$' is methyl, $R_3$' and $R_4$' are not hydrogen at the same time; and

when $R_1$' is fluorine and $R_2$' is methyl, $R_4$' is not $C_{1-6}$ alkyl.

In some embodiments, in Formula (II),

$R_1$' is selected from the group consisting of hydrogen, fluorine and chlorine;

$R_2$' is chlorine or methyl;

$R_3$' is hydrogen, methyl or deuterated methyl;

$R_4$' is hydrogen.

In some embodiments, in Formula (II),

$R_1$' is fluorine;

$R_2$' is chlorine;

$R_3$' is selected from the group consisting of hydrogen, $C_{1-6}$ alkyl, $C_{1-6}$ haloalkyl, $C_{1-6}$ alkoxyalkyl, $C_{3-6}$ cycloalkyl and 3- to 6-membered heterocyclyl; $R_3$' is preferably hydrogen, $C_{1-6}$ alkyl, $C_{1-6}$ haloalkyl or $C_{1-6}$ alkoxyalkyl; preferably hydrogen, $C_{1-6}$ alkyl or $C_{1-6}$ haloalkyl; for example, hydrogen, methyl or deuterated methyl;

$R_4$' is selected from the group consisting of hydrogen, $C_{1-6}$ alkyl and $C_{1-6}$ haloalkyl; $R_4$' is preferably hydrogen.

In some embodiments, in Formula (II),

$R_1$' is chlorine;

$R_2$' is methyl;

$R_3$' is selected from the group consisting of hydrogen, $C_{1-6}$ alkyl, $C_{1-6}$ haloalkyl, $C_{1-6}$ alkoxyalkyl, $C_{3-6}$ cycloalkyl and 3- to 6-membered heterocyclyl; $R_3$' is preferably hydrogen, $C_{1-6}$ alkyl, $C_{1-6}$ haloalkyl or $C_{1-6}$ alkoxyalkyl; preferably hydrogen, $C_{1-6}$ alkyl or $C_{1-6}$ haloalkyl; for example, hydrogen, methyl or deuterated methyl;

$R_4$' is selected from the group consisting of hydrogen, $C_{1-6}$ alkyl and $C_{1-6}$ haloalkyl; $R_4$' is preferably hydrogen.

In some embodiments, the compound has the structure of Formula (III):

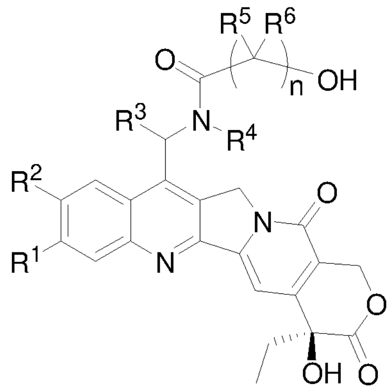

Formula (III)

in Formula (III),

$R^1$ is selected from the group consisting of hydrogen, fluorine, and chlorine;

$R^2$ is selected from the group consisting of hydrogen, methyl, fluorine, chlorine, and hydroxyl;

$R^3$ is selected from the group consisting of hydrogen, $C_{1-6}$ alkyl, $C_{1-6}$ haloalkyl, $C_{1-6}$ alkoxyalkyl, $C_{3-6}$ cycloalkyl, and 3- to 6-membered heterocyclyl, or $R^3$ is connected to an adjacent benzene ring carbon atom to form a 6-membered carbocycle; $R^3$ is preferably hydrogen;

$R^4$ is hydrogen or $C_{1-6}$ alkyl;

$R^5$ and $R^6$ are independently selected from the group consisting of hydrogen, $C_{1-6}$ alkyl, $C_{2-6}$ alkenyl, $C_{1-6}$ haloalkyl, $C_{3-6}$ cycloalkyl and 3- to 6-membered heterocyclyl, or $R^5$ and $R^6$ together with the carbon atom to which they are attached form a 3- to 6-membered ring;

or, $R^4$ and $R^5$ are connected to form a 4- to 6-membered ring;

n = 1 or 2;

when $R^1$ is fluorine, $R^2$ is methyl, $R^3$ is hydrogen, and $R^4$ is hydrogen, $R^5$ and $R^6$ are not hydrogen at the same time, nor are they together with the carbon atom to which they are attached form a cyclopropyl; and

when $R^1$ is fluorine, $R^2$ is methyl, and $R^3$ is hydrogen, $R^4$ is not alkyl.

In some embodiments, $R^3$ is connected to an adjacent benzene ring carbon atom to form a 6-membered carbocycle, and the carbon atom is the carbon atoms at the meta position of $R^1$ and at the ortho position of $R^2$.

In some embodiments, the compound has the structure represented by Formula (III-1),

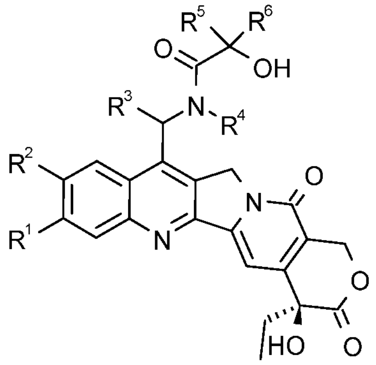

Formula (III-1)

$R^1$ is selected from the group consisting of hydrogen, fluorine and chlorine;

$R^2$ is selected from the group consisting of hydrogen, methyl, fluorine, chlorine and hydroxyl;

$R^3$ is selected from the group consisting of hydrogen, $C_{1-6}$ alkyl, $C_{1-6}$ haloalkyl, $C_{1-6}$ alkoxyalkyl, $C_{3-6}$ cycloalkyl and 3- to 6-membered heterocyclyl; $R^3$ is preferably hydrogen;

$R^4$ is hydrogen or $C_{1-6}$ alkyl;

$R^5$ and $R^6$ are independently selected from the group consisting of hydrogen, $C_{1-6}$ alkyl, $C_{1-6}$ haloalkyl, $C_{3-6}$ cycloalkyl and 3- to 6-membered heterocyclyl, or $R^5$ and $R^6$ together with the carbon atom to which they are attached form a 3- to 6-membered ring;

when $R^1$ is fluorine, $R^2$ is methyl, $R^3$ is hydrogen, and $R^4$ is hydrogen, $R^5$ and $R^6$ are not hydrogen at the same time, nor do they together with the carbon atom to which they are attached form a cyclopropyl; and

when $R^1$ is fluorine, $R^2$ is methyl, and $R^3$ is hydrogen, $R^4$ is not alkyl.

In some embodiments, $R^2$ is fluorine or chlorine.
In some embodiments, $R^2$ is selected from the group consisting of hydrogen, methyl, fluorine, and chlorine.
In some embodiments, $R^2$ is methyl or chlorine.
In some embodiments, $R^3$ is selected from the group consisting of hydrogen, $C_{1-6}$ alkyl, $C_{1-6}$ haloalkyl, $C_{1-6}$ alkoxyalkyl, $C_{3-6}$ cycloalkyl, and 3- to 6-membered heterocyclyl.
In some embodiments, $R^4$ is hydrogen.
In some embodiments, $R^5$ and $R^6$ are independently selected from the group consisting of hydrogen, $C_{1-6}$ alkyl, $C_{1-6}$ haloalkyl, $C_{3-6}$ cycloalkyl, and $C_{2-6}$ alkenyl, or $R^5$ and $R^6$ together with the carbon atom to which they are attached form a 3- to 6-membered ring.
In some embodiments, $R^5$ and $R^6$ are independently selected from the group consisting of hydrogen, $C_{1-6}$ alkyl, $C_{2-6}$ alkenyl, $C_{3-6}$ cycloalkyl, or $R^5$ and $R^6$ together with the carbon atom to which they are attached form a 3- to 6-membered carbocycle.
In some embodiments, $R^5$ and $R^6$ are independently selected from the group consisting of hydrogen, methyl, vinyl, allyl, cyclopropyl, or $R^5$ and $R^6$ together with the carbon atom to which they are attached form a 3-membered carbocycle.
In some embodiments, $R^5$ and $R^6$ are independently selected from the group consisting of hydrogen, $C_{1-6}$ alkyl and $C_{3-6}$ cycloalkyl, or $R^5$ and $R^6$ together with the carbon atom to which they are attached form a 3- to 6-membered carbocycle.
In some embodiments, $R^5$ and $R^6$ are independently selected from the group consisting of hydrogen, methyl, and cyclopropyl, or $R^5$ and $R^6$ together with the carbon atom to which they are attached form a 3-membered carbocycle.
In some embodiments, when n = 2, in Formula (III)

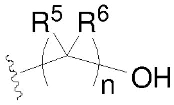 is 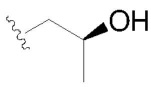

or

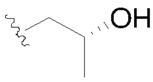.

In some embodiments, $R^4$ and $R^5$ are connected to form a 5-membered ring.
In some embodiments, $R^4$ and $R^5$ are connected to form a 5-membered ring, and $R^6$ is hydrogen.
In some embodiments, in Formula (III) or Formula (III-1),

$R^1$ is selected from the group consisting of fluorine and chlorine;

$R^2$ is selected from the group consisting of chlorine and methyl;

$R^3$ is selected from the group consisting of hydrogen and $C_{1-6}$ alkyl, or $R^3$ is connected to an adjacent benzene ring carbon atom to form a 6-membered carbon ring;

$R^4$ is hydrogen;

$R^5$ and $R^6$ are independently selected from the group consisting of hydrogen, $C_{1-6}$ alkyl, $C_{1-6}$ haloalkyl, $C_{3-6}$ cycloalkyl and $C_{2-6}$ alkenyl, or $R^5$ and $R^6$ together with the carbon atom to which they are attached form a 3- to 6-membered ring;

or, $R^4$ and $R^5$ are connected to form a 4- to 6-membered ring.

In some embodiments, $R^3$ is connected to an adjacent benzene ring carbon atom to form a 6-membered carbocycle, and the carbon atom is the carbon atom at the meta position of $R^1$ and at the ortho position of $R^2$.
In some embodiments, in Formula (III) or Formula (III-1),

$R^1$ is fluorine;

$R^2$ is chlorine;

$R^3$ is hydrogen or $C_{1-2}$ alkyl, or $R^3$ is connected to an adjacent benzene ring carbon atom to form a 6-membered carbocycle;

$R^4$ is hydrogen;

$R^5$ and $R^6$ are independently selected from the group consisting of hydrogen, $C_{1-2}$ alkyl, $C_{1-2}$ haloalkyl, $C_{3-6}$ cycloalkyl and $C_{2-4}$ alkenyl, or $R^5$ and $R^6$ together with the carbon atom to which they are attached form a 3- to 6-membered ring;

or, $R^4$ and $R^5$ are connected to form a 4- to 6-membered ring.

In some embodiments, $R^3$ is connected to an adjacent benzene ring carbon atom to form a 6-membered carbocycle, and the carbon atom is the carbon atom at the meta position of $R^1$ and at the ortho position of $R^2$.
In some embodiments, in Formula (III) or Formula (III-1),

$R^1$ is chlorine;

$R^2$ is methyl;

$R^3$ is hydrogen or $C_{1-2}$ alkyl, or $R^3$ is connected to an adjacent benzene ring carbon atom to form a 6-membered carbon ring;

$R^4$ is hydrogen;

$R^5$ and $R^6$ are independently selected from the group consisting of hydrogen, $C_{1-2}$ alkyl, $C_{1-2}$ haloalkyl, $C_{3-6}$ cycloalkyl and $C_{2-4}$ alkenyl, or $R^5$ and $R^6$ together with the carbon atom to which they are attached form a 3- to 6-membered ring;

or, $R^4$ and $R^5$ are connected to form a 4- to 6-membered ring.

In some embodiments, $R^3$ is connected to an adjacent benzene ring carbon atom to form a 6-membered carbocycle, and the carbon atom is the carbon atom at the meta position of $R^1$ and at the ortho position of $R^2$.

In some embodiments, in Formula (III) or Formula (III-1),

$R^1$ is fluorine;

$R^2$ is methyl;

$R^3$ is hydrogen or $C_{1-2}$ alkyl, or $R^3$ is connected to an adjacent benzene ring carbon atom to form a 6-membered carbon ring;

$R^4$ is hydrogen;

$R^5$ and $R^6$ are independently selected from the group consisting of hydrogen, $C_{1-2}$ alkyl, $C_{1-2}$ haloalkyl, $C_{3-6}$ cycloalkyl and $C_{2-4}$ alkenyl, or $R^5$ and $R^6$ together with the carbon atom to which they are attached form a 3- to 6-membered ring; preferably, $R^5$ and $R^6$ are not hydrogen at the same time;

or, $R^4$ and $R^5$ are connected to form a 4- to 6-membered ring.

In some embodiments, $R^3$ is connected to an adjacent benzene ring carbon atom to form a 6-membered carbocycle, and the carbon atom is the carbon atom at the meta position of $R^1$ and at the ortho position of $R^2$.

In some embodiments, in Formula (III) or Formula (III-1),

$R^1$ is fluorine or chlorine;

$R^2$ is methyl or chlorine; preferably chlorine;

$R^3$ is hydrogen, or $R^3$ is connected to an adjacent benzene ring carbon atom to form a 6-membered carbon ring;

$R^4$ is hydrogen;

$R^5$ and $R^6$ are independently selected from the group consisting of hydrogen, $C_{1-6}$ alkyl, $C_{1-6}$ haloalkyl, $C_{3-6}$ cycloalkyl and $C_{2-6}$ alkenyl, or $R^5$ and $R^6$ together with the carbon atom to which they are attached form a 3- to 6-membered ring;

or, $R^4$ and $R^5$ are connected to form a 4- to 6-membered ring.

In some embodiments, $R^3$ is connected to an adjacent benzene ring carbon atom to form a 6-membered carbocycle, and the carbon atom is the carbon atom at the meta position of $R^1$ and at the ortho position of $R^2$.

In some embodiments, in Formula (III) or Formula (III-1),

$R^1$ is fluorine or chlorine;

$R^2$ is chlorine;

$R^3$ is hydrogen, or $R^3$ is connected to an adjacent benzene ring carbon atom to form a 6-membered carbon ring;

$R^4$ is hydrogen;

$R^5$ and $R^6$ are independently selected from the group consisting of hydrogen, methyl, trifluoromethyl, cyclopropyl and vinyl, or $R^5$ and $R^6$ together with the carbon atom to which they are attached form a 3-membered ring;

or, $R^4$ and $R^5$ are connected to form a 5-membered ring.

In some embodiments, $R^3$ is connected to an adjacent benzene ring carbon atom to form a 6-membered carbocycle, and the carbon atom is the carbon atom at the meta position of $R^1$ and at the ortho position of $R^2$.

In some embodiments, the compound has the structure represented by Formula (IV):

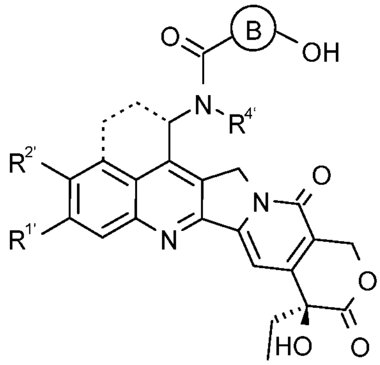

Formula (IV)

in Formula (IV),

$R^{1'}$ is selected from the group consisting of hydrogen, fluorine, and chlorine;

$R^{2'}$ is selected from the group consisting of hydrogen, methyl, fluorine, chlorine, and hydroxyl;

$R^{4'}$ is selected from the group consisting of hydrogen, $C_{1-6}$ alkyl, $C_{3-6}$ cycloalkyl, and $C_{1-6}$ haloalkyl; $R^{4'}$ is preferably hydrogen;

B is selected from the group consisting of $C_{1-6}$ alkylene, 3- to 6-membered carbocycle, and 3- to 6-membered heterocycle;

when $R^{1'}$ is fluorine, $R^{2'}$ is methyl, and the dashed carbocycle exists, $R^{4'}$ is neither hydrogen nor $C_{1-6}$ alkyl; and

when $R^{2'}$ is methyl, $R^{1'}$ is fluorine, and the dashed carbocycle does not exist, Ring B is not a 3-membered carbocycle.

In some embodiments, in Formula (IV), $R^{1'}$ is selected from the group consisting of hydrogen, fluorine, and chlorine;

$R^{2'}$ is selected from the group consisting of hydrogen, methyl, fluorine, chlorine and hydroxyl;

$R^{4'}$ is selected from the group consisting of hydrogen, $C_{1-6}$ alkyl, $C_{3-6}$ cycloalkyl and $C_{1-6}$ haloalkyl; $R^{4'}$ is preferably hydrogen;

B is selected from the group consisting of 3- to 6-membered carbocycle or 3- to 6-membered heterocyclic group;

when $R^{1'}$ is fluorine, $R^{2'}$ is methyl, and the dashed carbocycle exists, $R^{4'}$ is neither hydrogen nor alkyl; and

when $R^{2'}$ is methyl, $R^{1'}$ is fluorine, and the dashed carbocycle does not exist, Ring B is not a 3-membered carbocycle.

In some embodiments, $R^{1'}$ is fluorine or chlorine.

In some embodiments, $R^{2'}$ is selected from the group consisting of hydrogen, methyl, fluorine, and chlorine.

In some embodiments, $R^{2'}$ is methyl or chlorine.

In some embodiments, $R^{4'}$ is hydrogen.

In some embodiments, B is $C_{1-6}$ alkylene; preferably, B is methylene, ethylene, propylene, isopropylene; more preferably, B is propylene or isopropylene.

In some embodiments, Ring B is a 3- to 6-membered carbocycle; preferably, Ring B is a 3-to 6-membered saturated carbocycle.

In some embodiments, the present invention provides the following compounds:

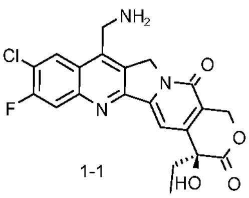
1-1

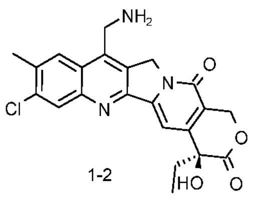
1-2

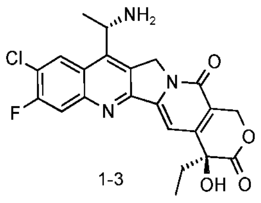
1-3

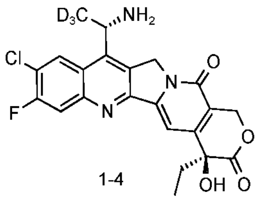
1-4

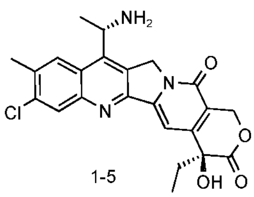
1-5

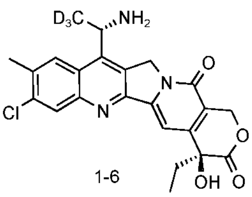
1-6

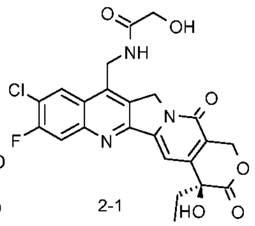
2-1

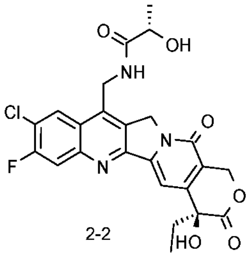
2-2

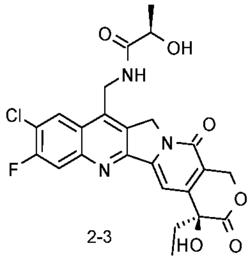
2-3

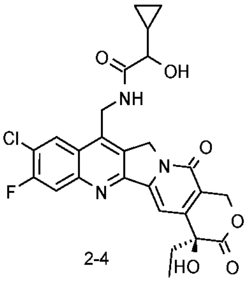
2-4

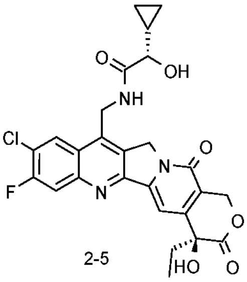
2-5

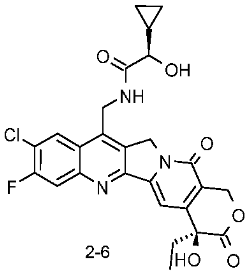
2-6

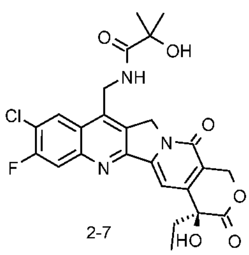
2-7

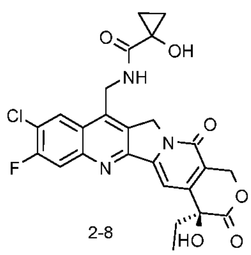
2-8

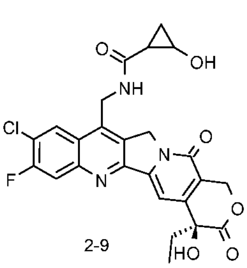
2-9

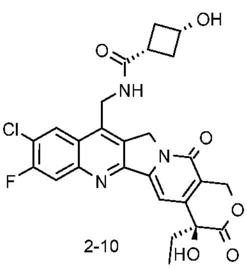
2-10

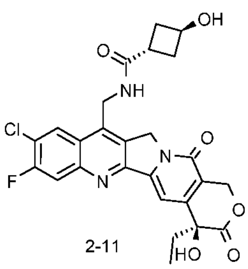
2-11

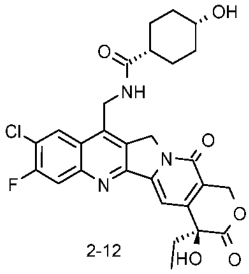
2-12

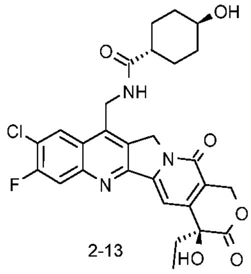
2-13

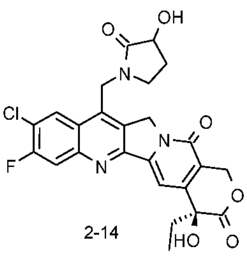
2-14

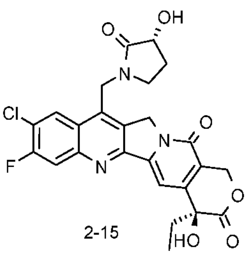
2-15

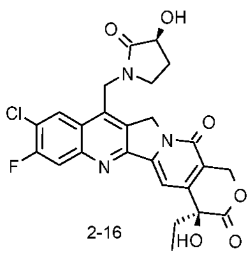
2-16

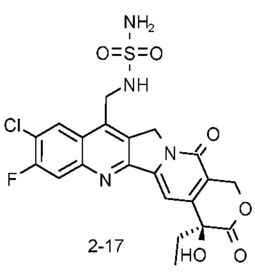
2-17

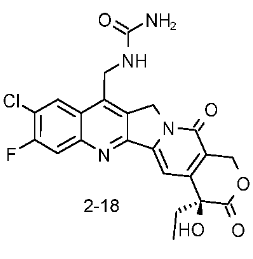
2-18

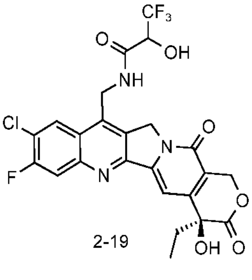
2-19

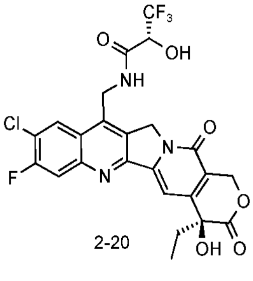
2-20

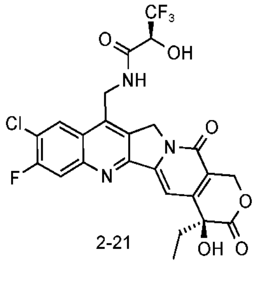
2-21

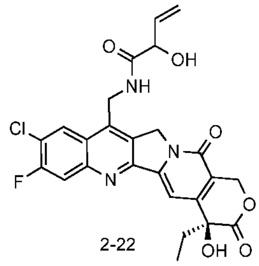
2-22

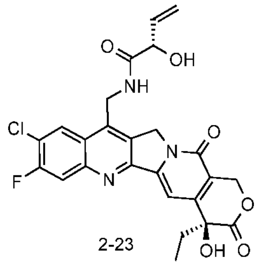
2-23

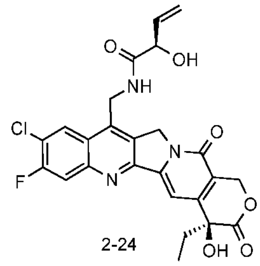
2-24

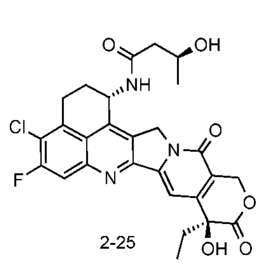
2-25

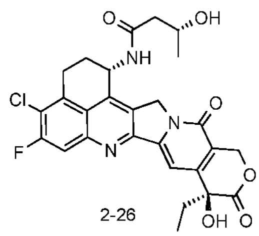
2-26

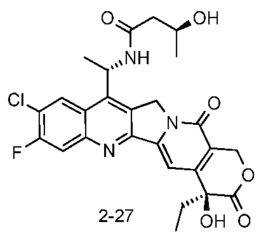
2-27

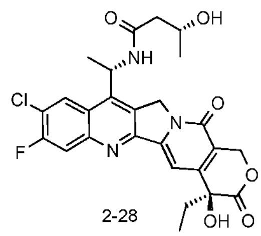
2-28

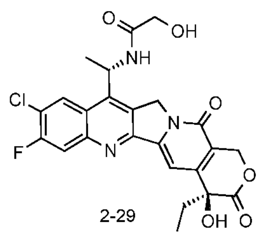
2-29

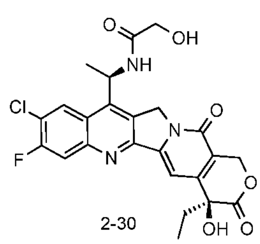
2-30

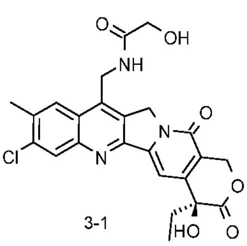
3-1

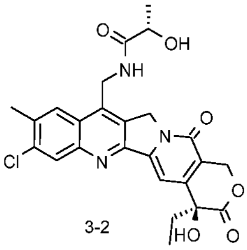
3-2

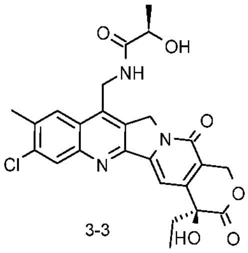
3-3

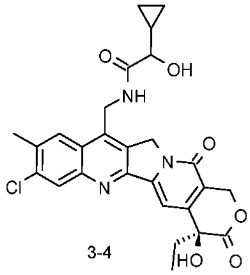
3-4

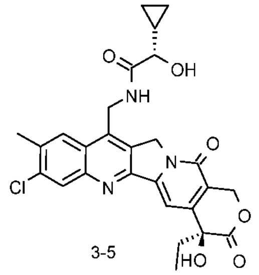
3-5

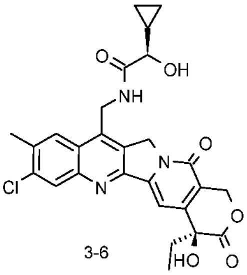
3-6

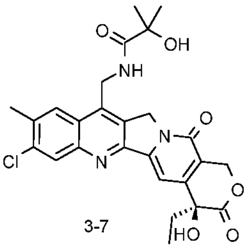
3-7

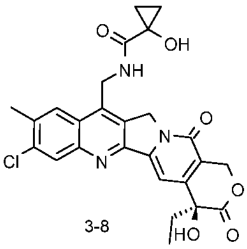
3-8

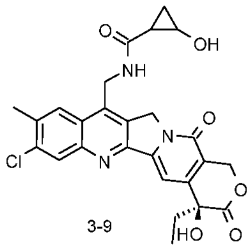
3-9

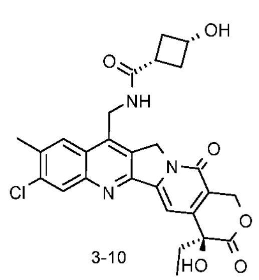
3-10

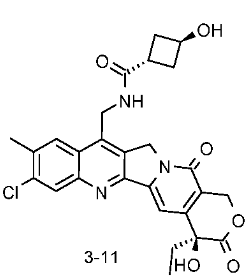
3-11

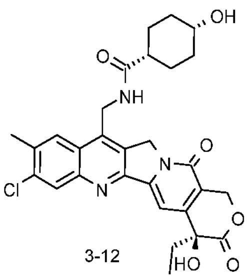
3-12

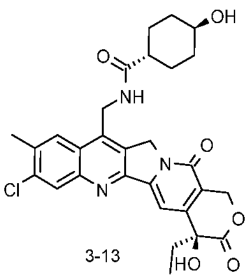
3-13

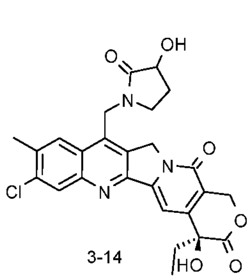
3-14

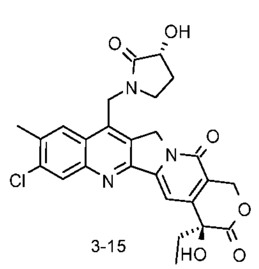
3-15

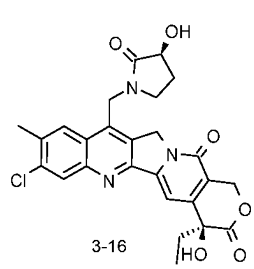
3-16

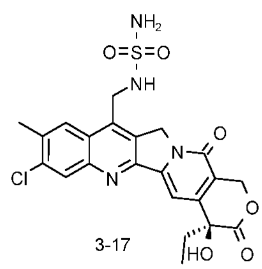
3-17

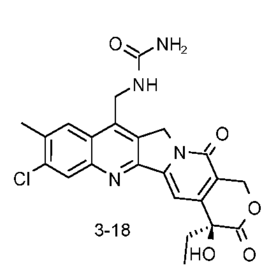
3-18

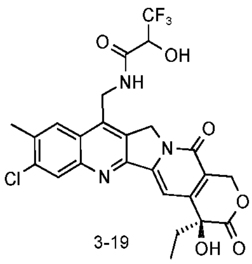
3-19
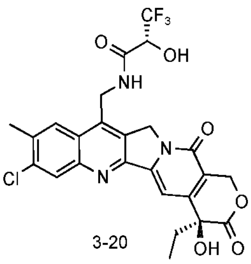
3-20
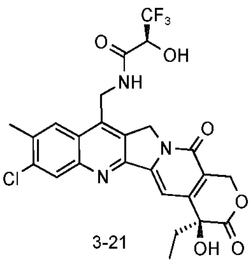
3-21
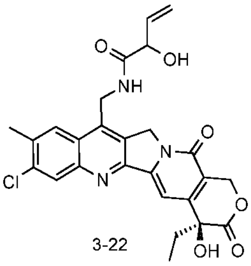
3-22
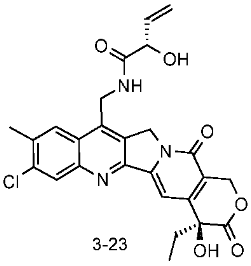
3-23
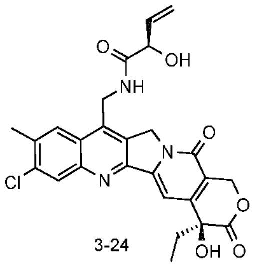
3-24
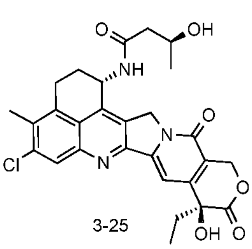
3-25
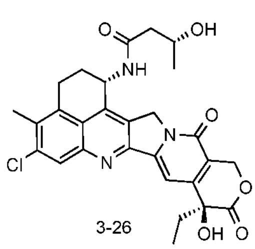
3-26
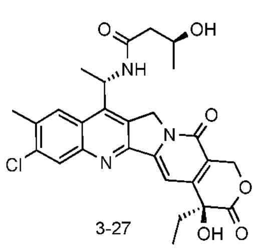
3-27
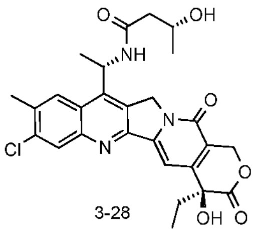
3-28
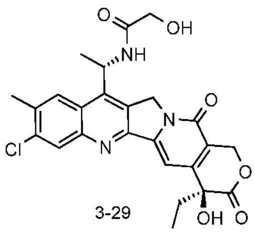
3-29
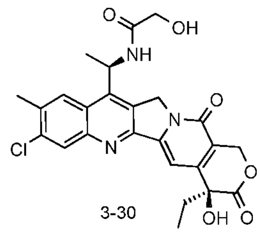
3-30
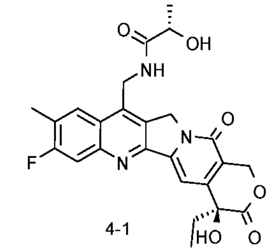
4-1
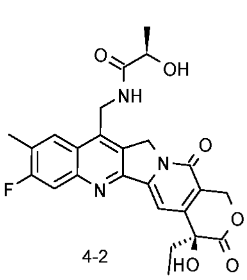
4-2
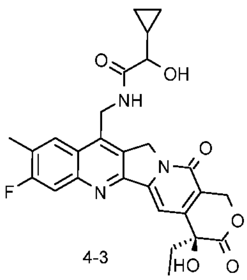
4-3
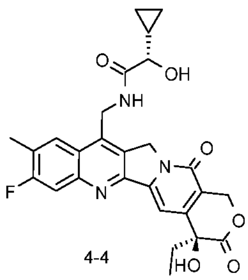
4-4
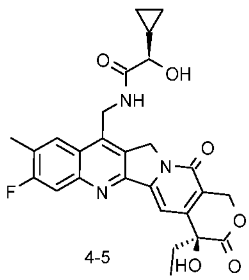
4-5
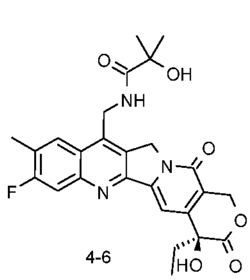
4-6
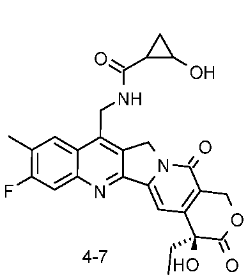
4-7
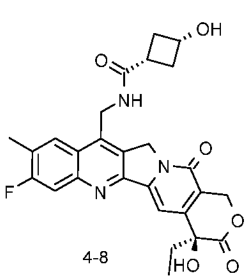
4-8
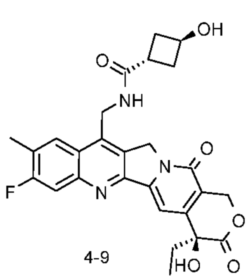
4-9
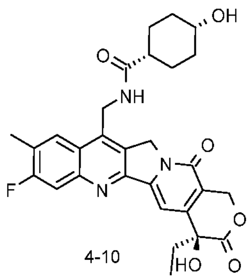
4-10
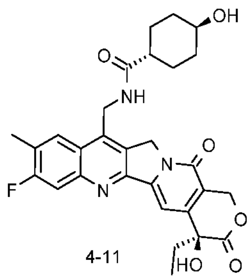
4-11
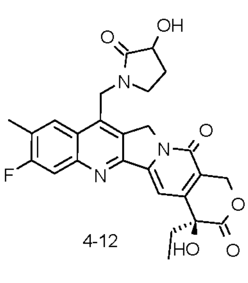
4-12
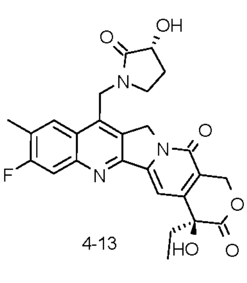
4-13
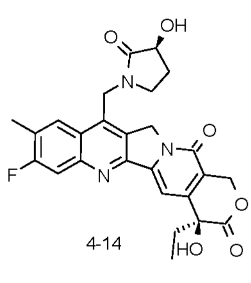
4-14
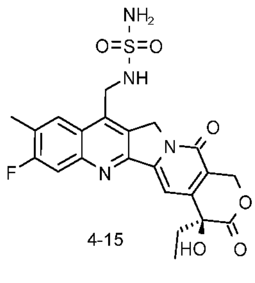
4-15

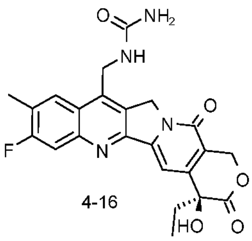
4-16

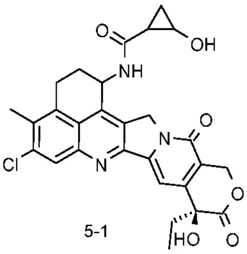
5-1

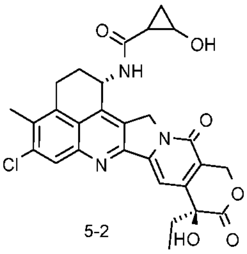
5-2

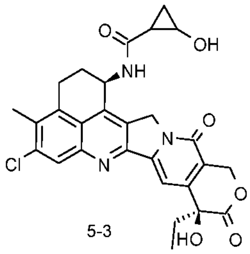
5-3

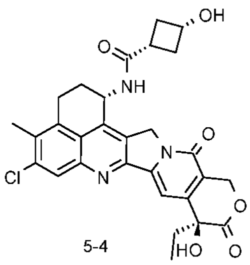
5-4

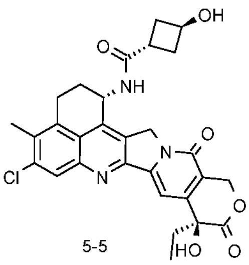
5-5

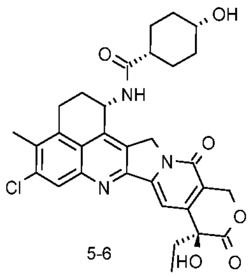
5-6

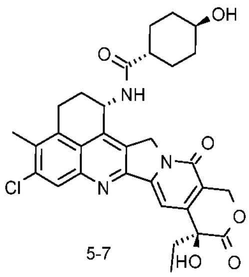
5-7

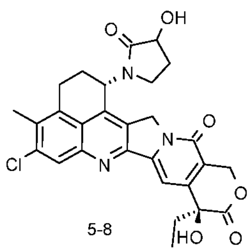
5-8

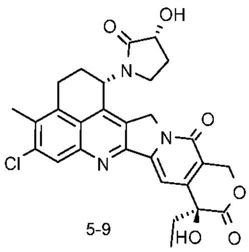
5-9

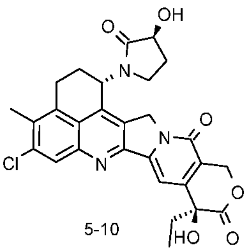
5-10

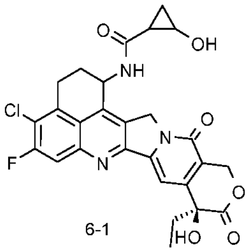
6-1

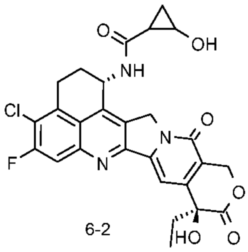
6-2

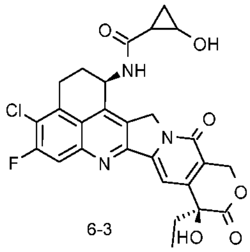
6-3

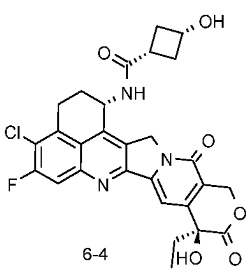
6-4

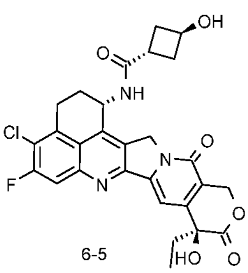
6-5

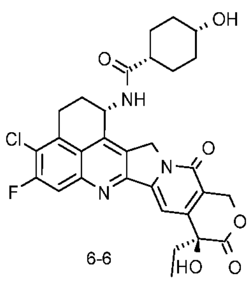
6-6

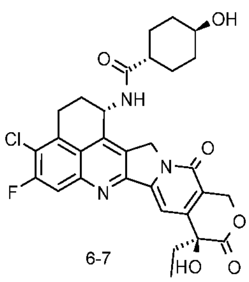
6-7

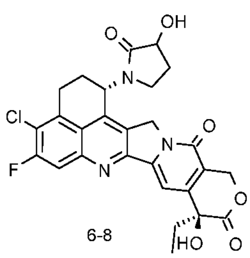
6-8

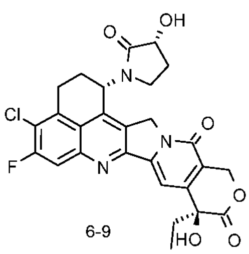
6-9

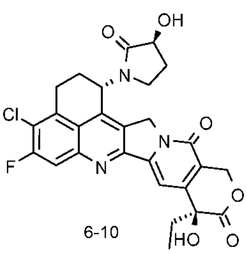
6-10

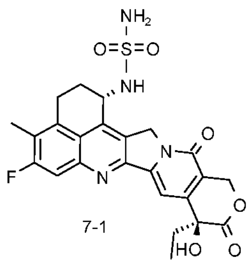
7-1

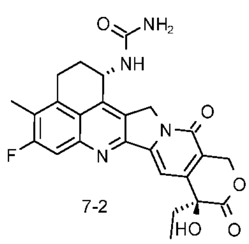
7-2

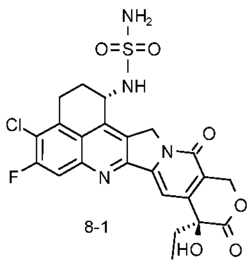
8-1

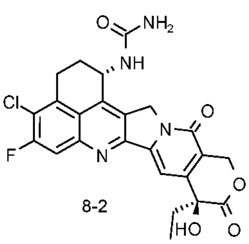
8-2

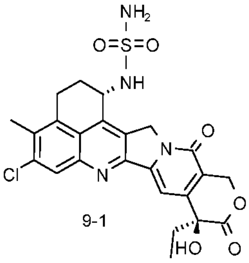
9-1

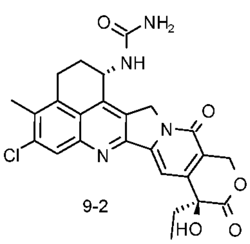
9-2

On the other hand, the present invention further provides a compound of Formula (V) or a pharmaceutically acceptable salt, ester, stereoisomer, polymorph, solvate, N-oxide, isotopically labeled compound, metabolite and prodrug thereof:

M-L-E-D Formula(V)

wherein,

M represents a linking site connecting an antibody or antigen-binding fragment thereof;

L represents a linker connecting the linking site M and E;

E represents a structural fragment connecting L and D;

D represents a structural fragment of a cytotoxic drug.

In some embodiments, M is selected from the group consisting of the following structures:

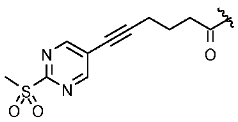, 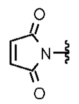, 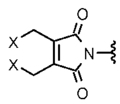, 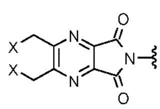, 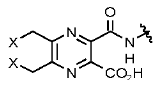 and 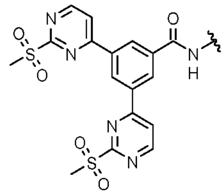,

wherein, X is selected from leaving groups, such as chlorine, bromine, -OMs, OTs, and OTf.

In some embodiments, M is selected from the group consisting of the following structures:

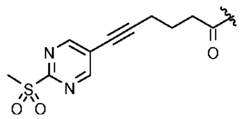 and 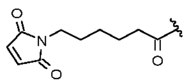.

In some embodiments, L is a structure constituted of one or more selected from the group consisting of: $C_{1-6}$ alkylene, -N(R')-, carbonyl, -O-, Val, Cit, Phe, Lys, D-Val, Leu, Gly, Ala, Asn, Val-Cit, Val-Ala, Val-Lys, Val-Lys(Ac), Phe-Lys, Phe-Lys(Ac), D-Val-Leu-Lys, Gly-Gly-Arg, Ala-Ala-Asn, Ala-Ala-Ala, Val-Lys-Ala, Val-Lys-Gly, Gly-Gly-Gly, Gly-Gly-Phe-Gly, Gly-Gly-Gly-Gly-Gly,

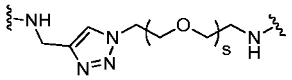, 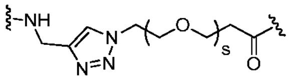, 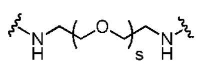, 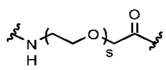,

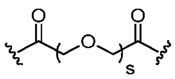, 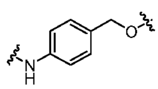, 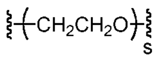, 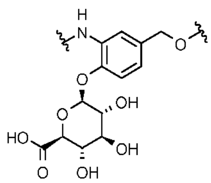 and 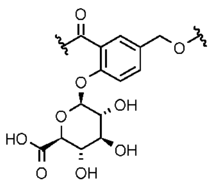;

wherein R' represents hydrogen, $C_{1-6}$ alkyl or an alkyl containing $-(CH_2CH_2O)_r-$; r is an integer selected from 1 to 10; s is an integer selected from 1 to 20.

In some embodiments, L is selected from the group consisting of the following structures:

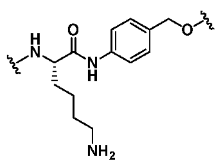,
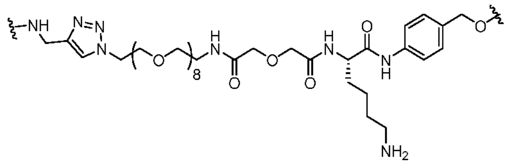,
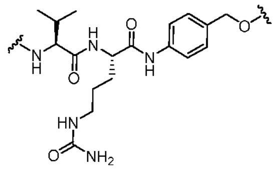,

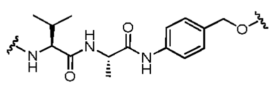,,
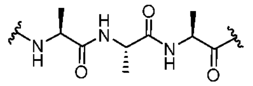,
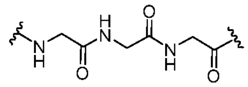,
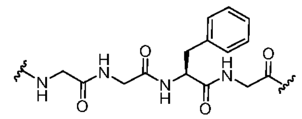,

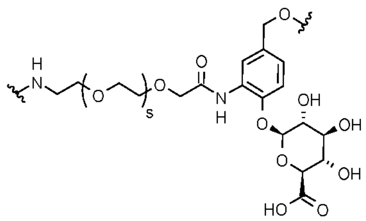 and 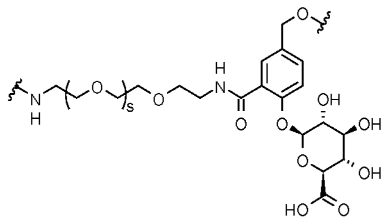;

wherein s is an integer selected from 1 to 20.

In some embodiments, E is selected from the group consisting of a single bond, -NH-$CH_2$-,

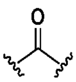, 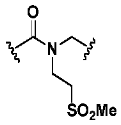, 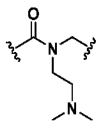 and 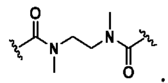.

In some embodiments, E is -NH-$CH_2$-.

In some embodiments, the cytotoxic drug is selected from the group consisting of the compounds described in any one of items of the first aspect of the present invention.

In some embodiments, the cytotoxic drug is selected from the group consisting of Compounds **1-1** to **1-2; 2-1** to **2-23; 3-1 to 3-4;** and **4-1** to **4-12.**

In some embodiments, D is selected from the group consisting of the structure formed by removing a hydrogen atom from the compound described in the first aspect of the present invention.

In some embodiments, D is selected from the group consisting of the structure formed by removing a hydrogen atom from Compound **1-1** to **1-2; 2-1** to **2-18; 3-1** to **3-18; 4-1** to **4-16; 5-1** to **5-10; 6-1** to **6-10; 7-1** to **7-2; 8-1** to **8-2** or **9-1** to **9-2.**

In some embodiments, D is selected from the group consisting of the following structures:

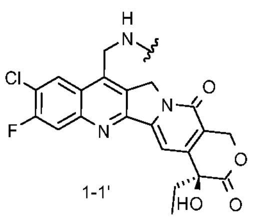
1-1'

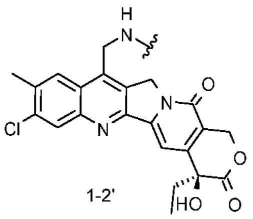
1-2'

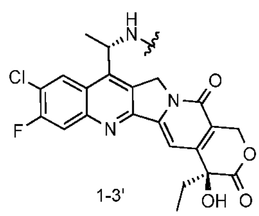
1-3'

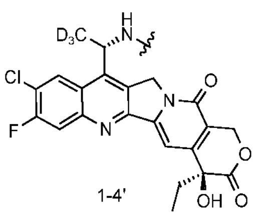
1-4'

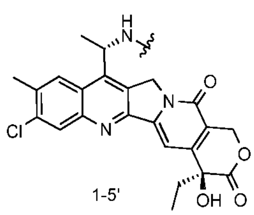
1-5'

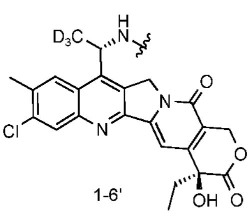
1-6'

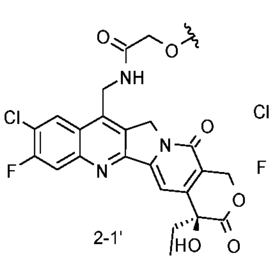
2-1'

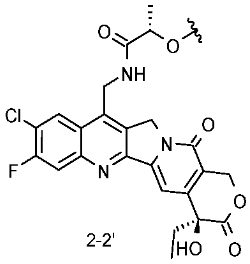
2-2'

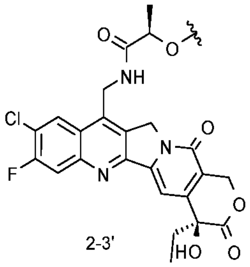
2-3'

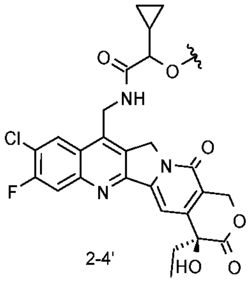
2-4'

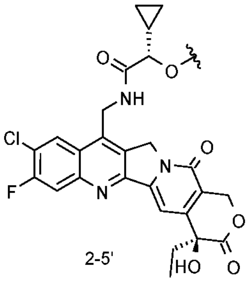
2-5'

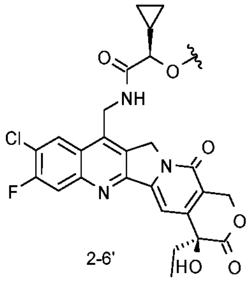
2-6'

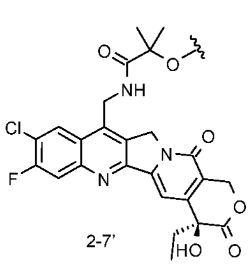
2-7'

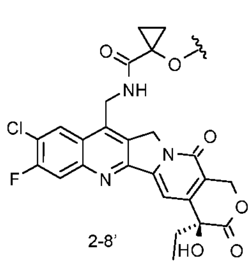
2-8'

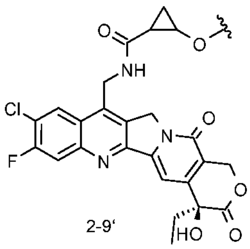
2-9'

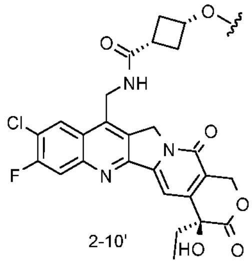
2-10'

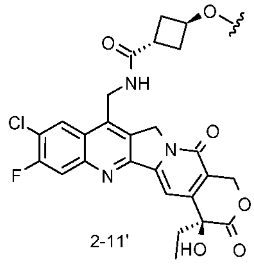
2-11'

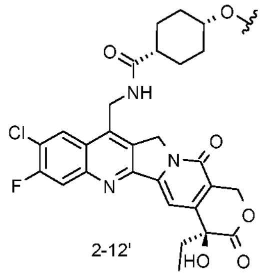
2-12'

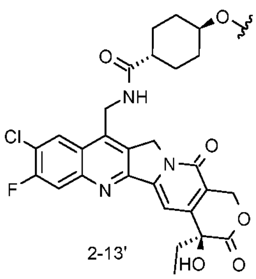
2-13'

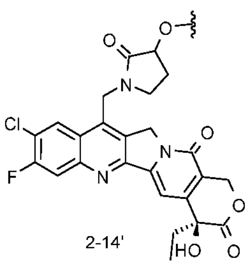
2-14'

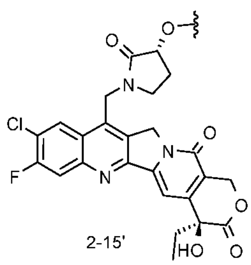
2-15'

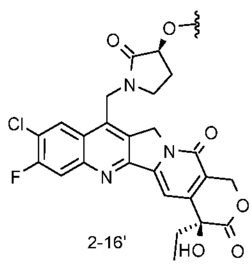
2-16'

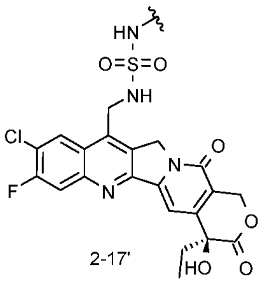
2-17'

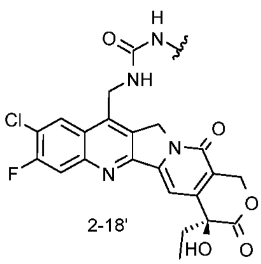
2-18'

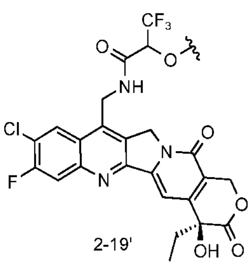
2-19'

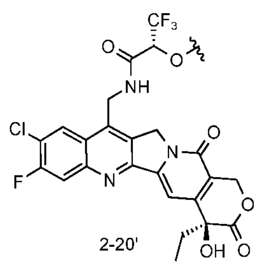
2-20'

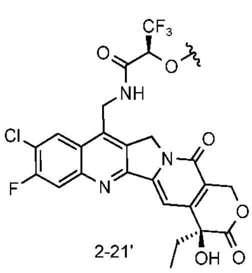
2-21'

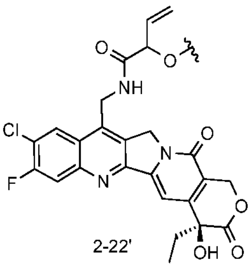
2-22'

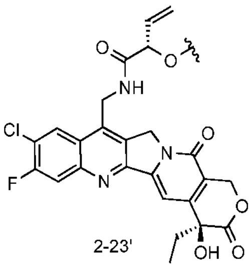
2-23'

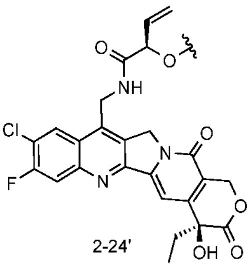
2-24'

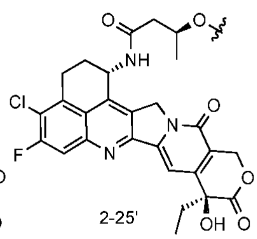
2-25'

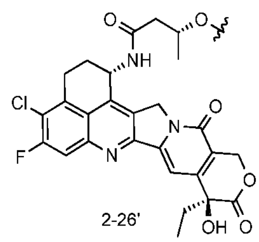
2-26'

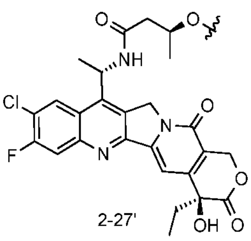
2-27'

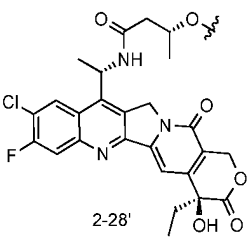
2-28'

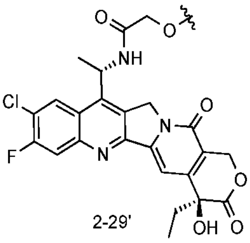
2-29'

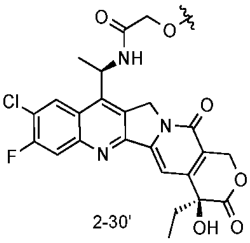
2-30'

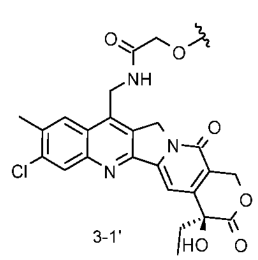
3-1'

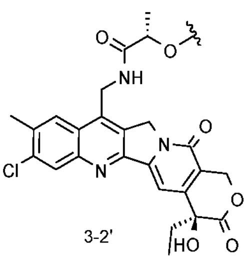
3-2'

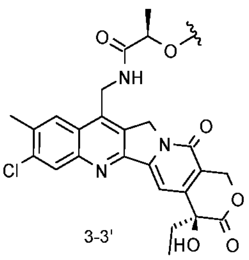
3-3'

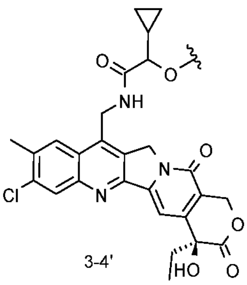
3-4'

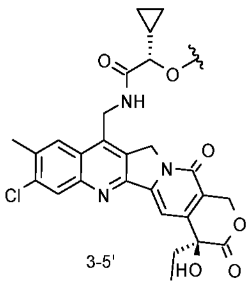
3-5'

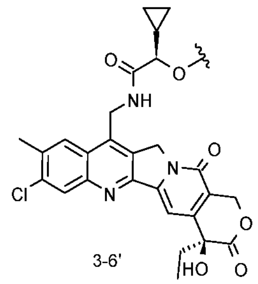
3-6'

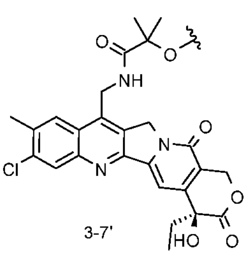
3-7'

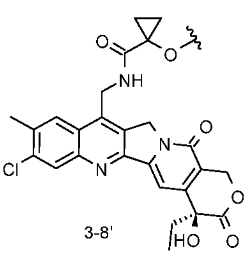
3-8'

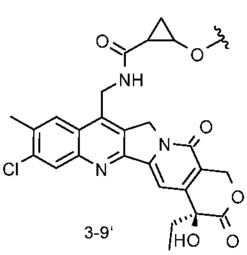
3-9'

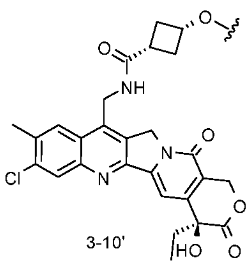
3-10'

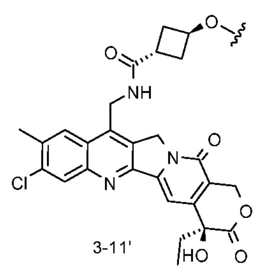
3-11'

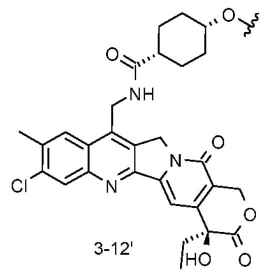
3-12'

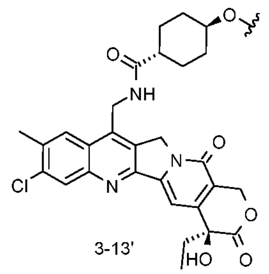
3-13'

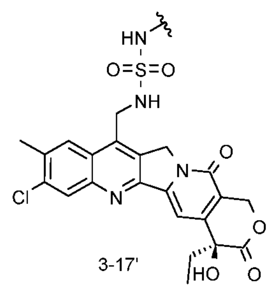
3-17'

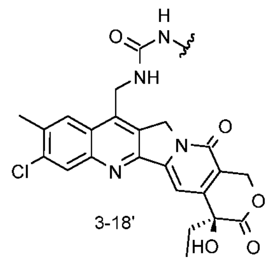
3-18'

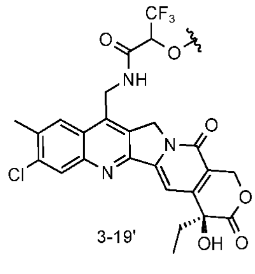
3-19'

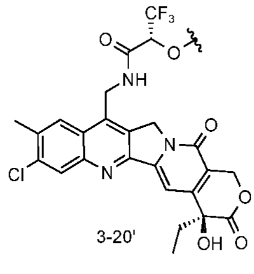
3-20'

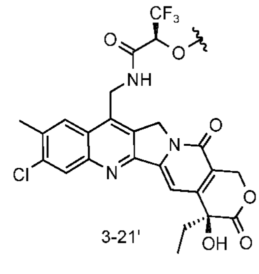
3-21'

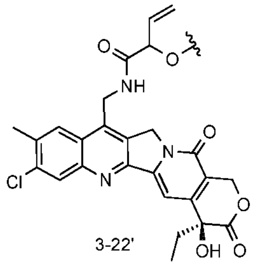
3-22'

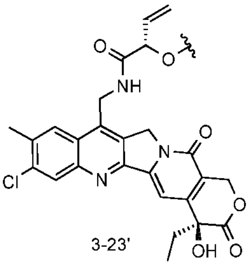
3-23'

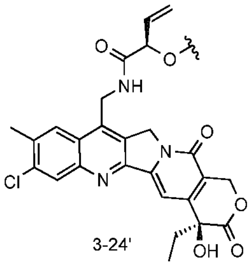
3-24'

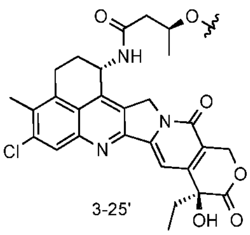
3-25'

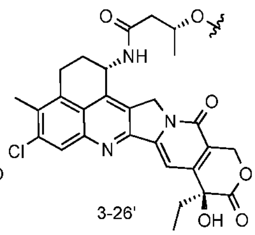
3-26'

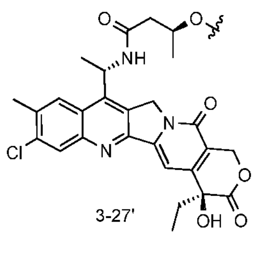
3-27'

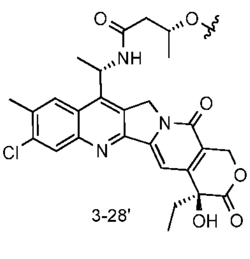
3-28'

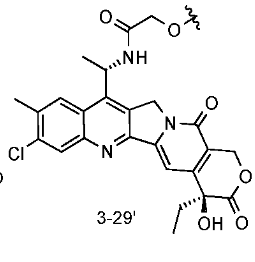
3-29'

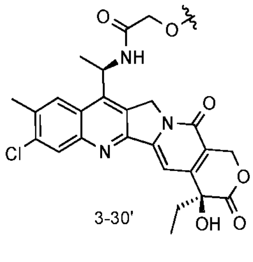
3-30'

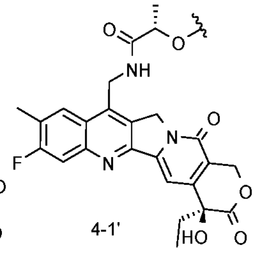
4-1'

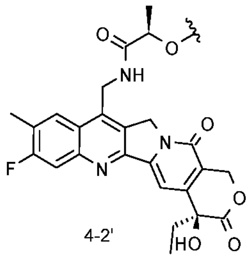
4-2'

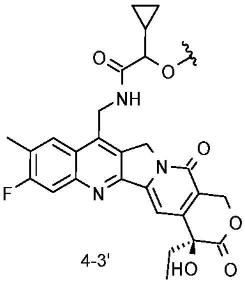
4-3'

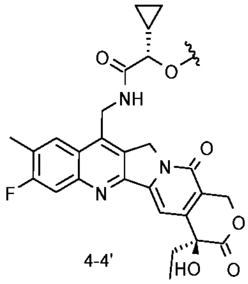
4-4'

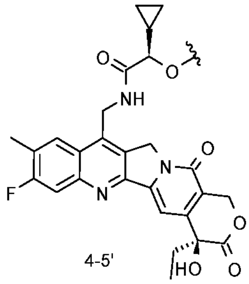
4-5'

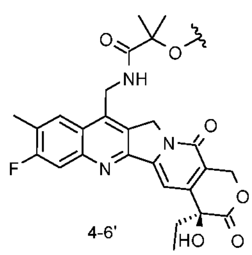
4-6'

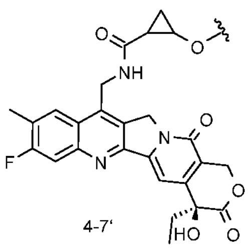
4-7'

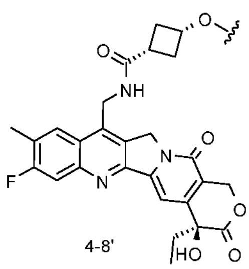
4-8'

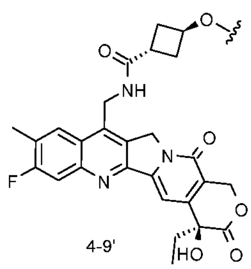
4-9'

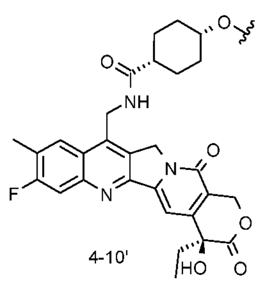
4-10'

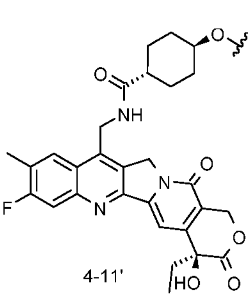
4-11'

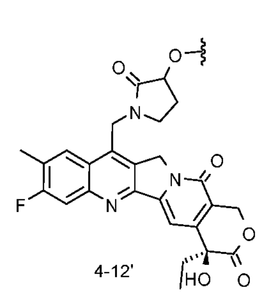
4-12'

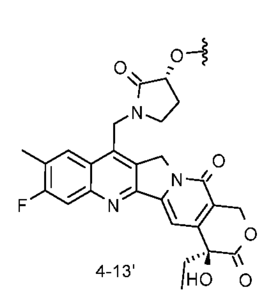
4-13'

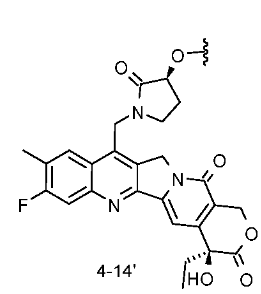
4-14'

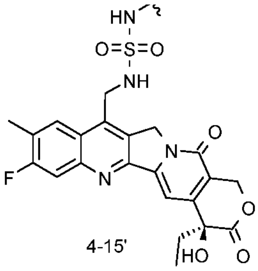
4-15'

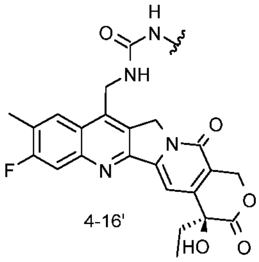
4-16'

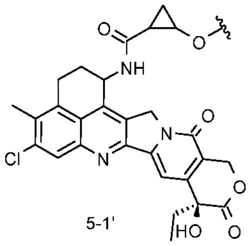
5-1'

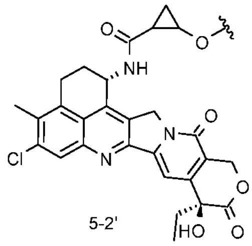
5-2'

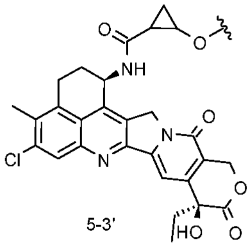
5-3'

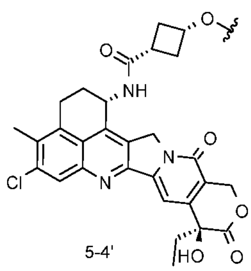
5-4'

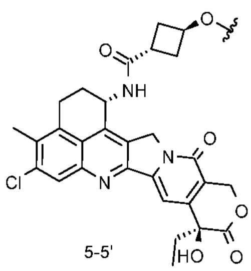
5-5'

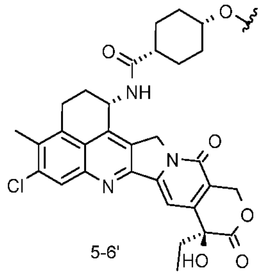
5-6'

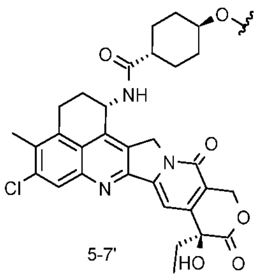
5-7'

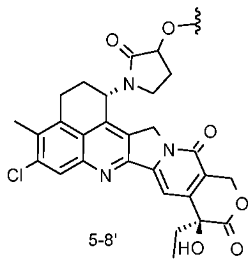
5-8'

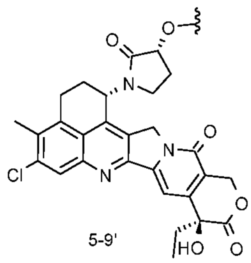
5-9'

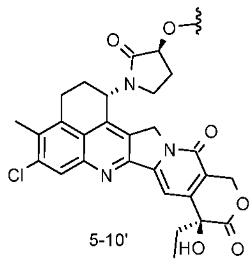
5-10'

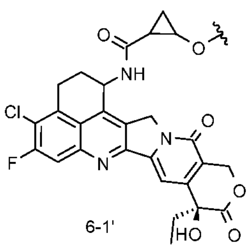
6-1'

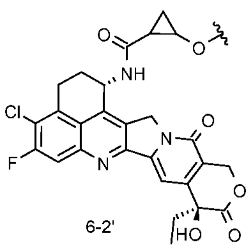
6-2'

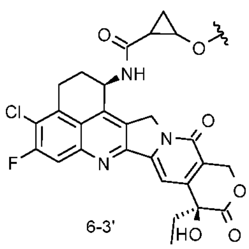
6-3'

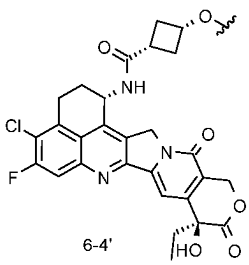
6-4'

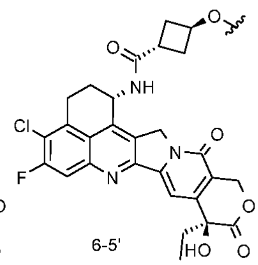
6-5'

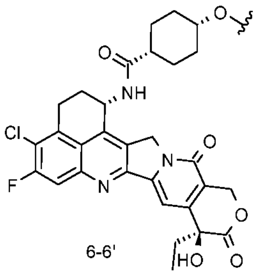
6-6'

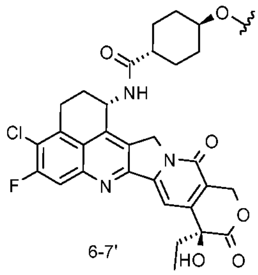
6-7'

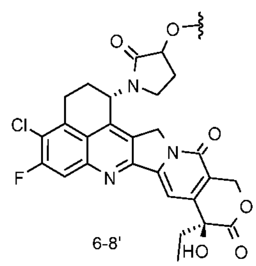
6-8'

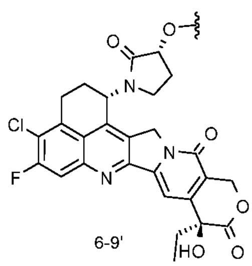
6-9'

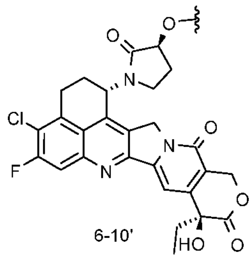
6-10'

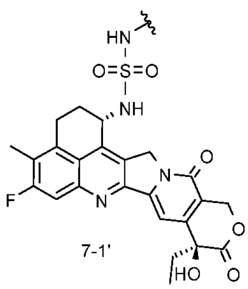
7-1'

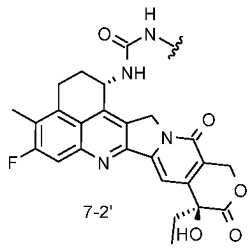
7-2'

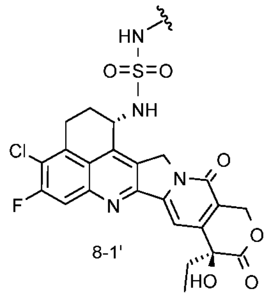
8-1'

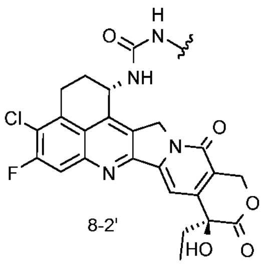
8-2'

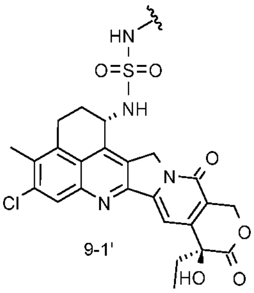
9-1'

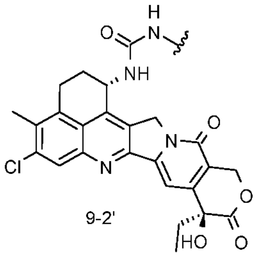
9-2'

.

In some embodiments, M-L-E-D is selected from the group consisting of the following compounds:

A-1:

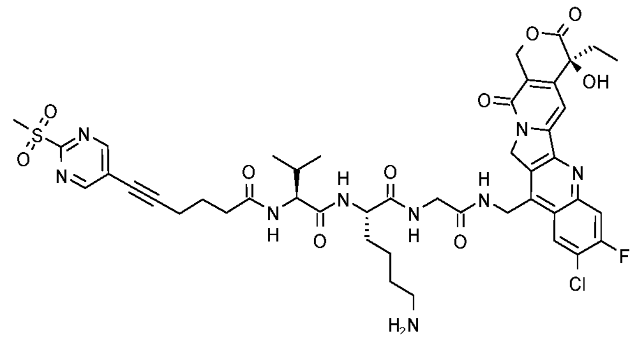

A-2:

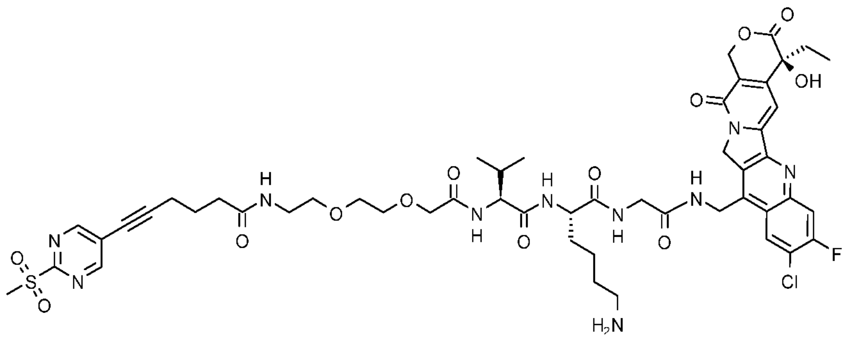

A-3:

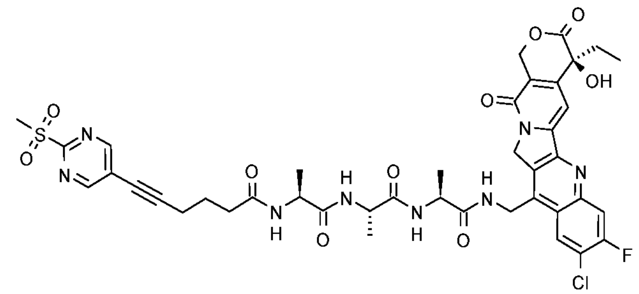

A-4:

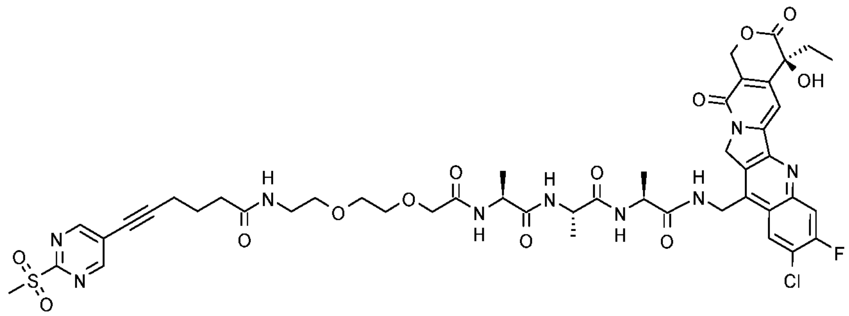

A-5:

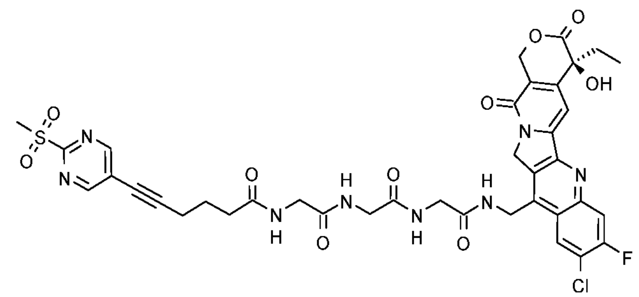

A-6:

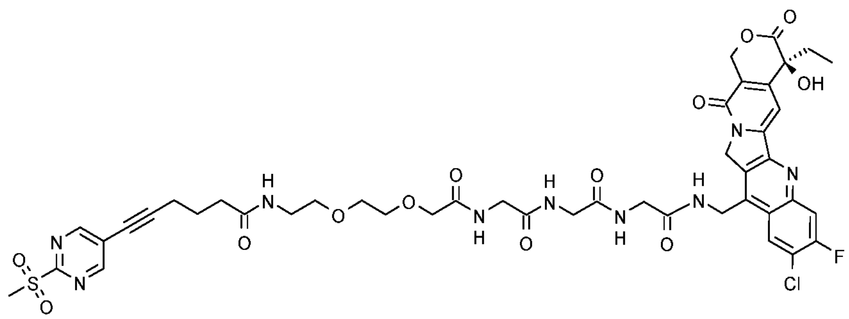

A-7:

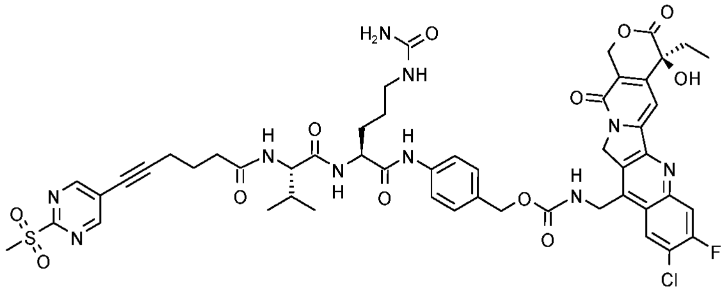

A-8:

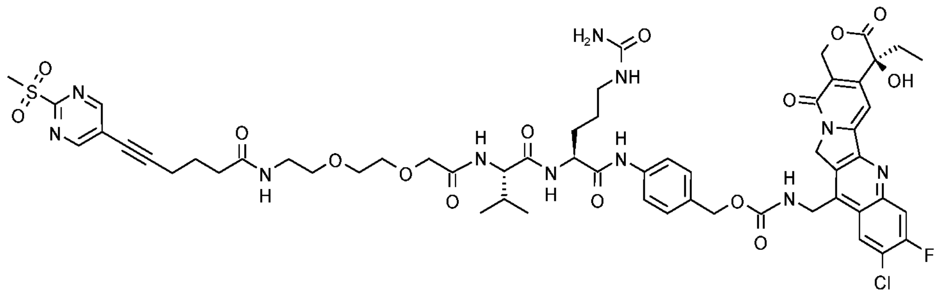

A-9:

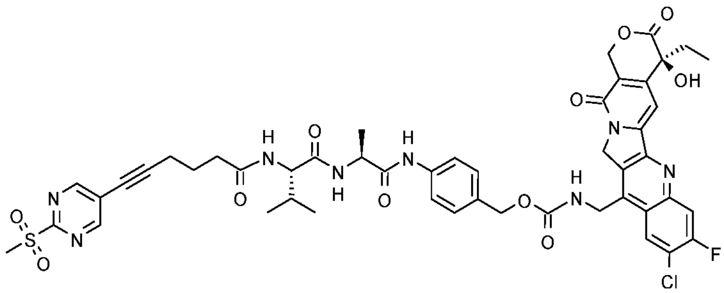

A-10:

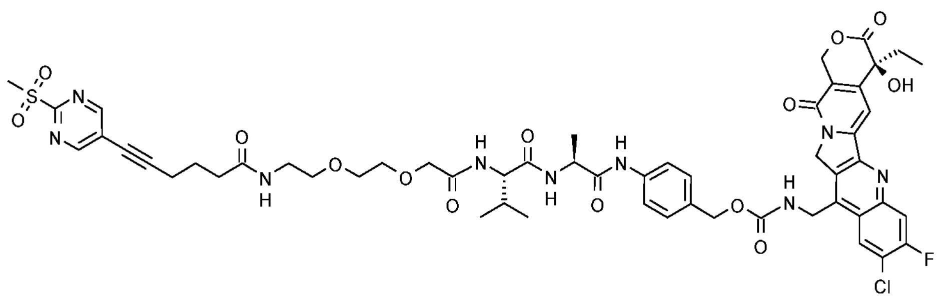

A-11:

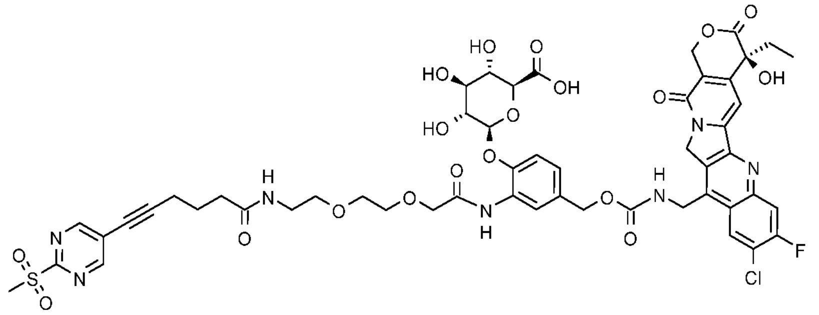

A-12:

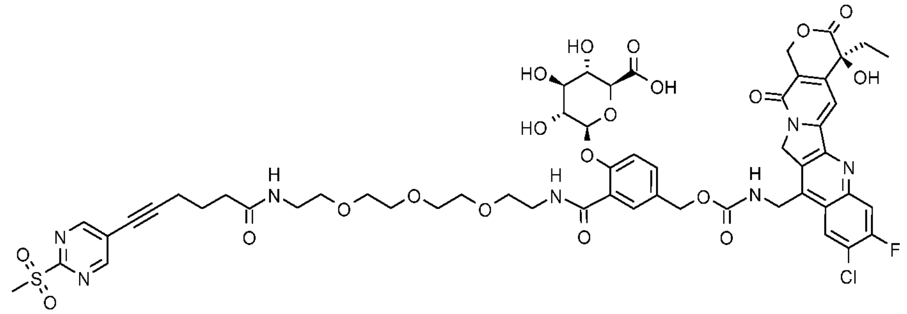

B-1:

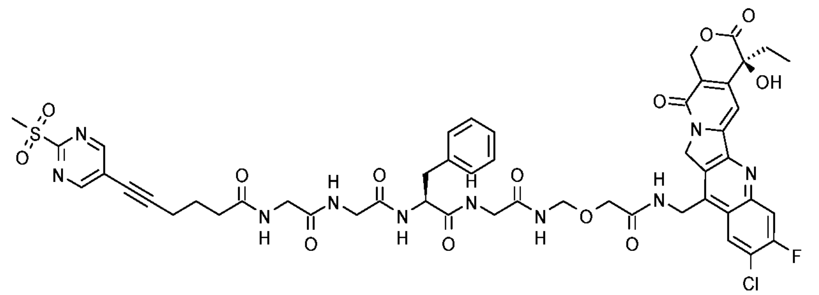

B-2:

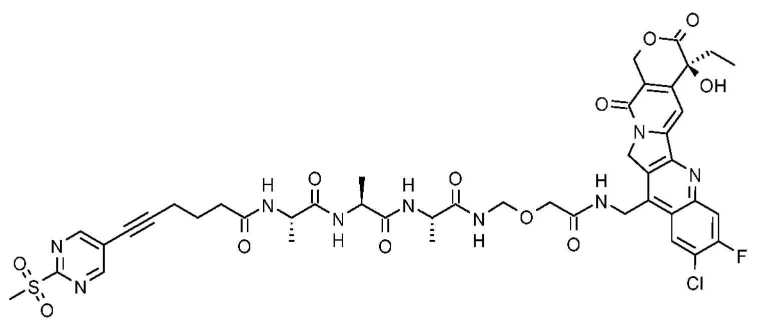

B-3:

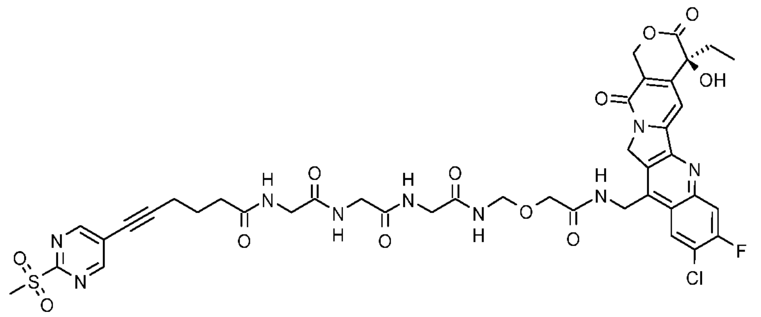

B-4:

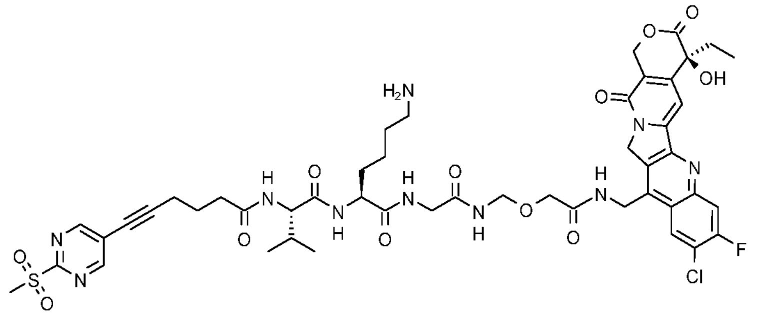

B-5:

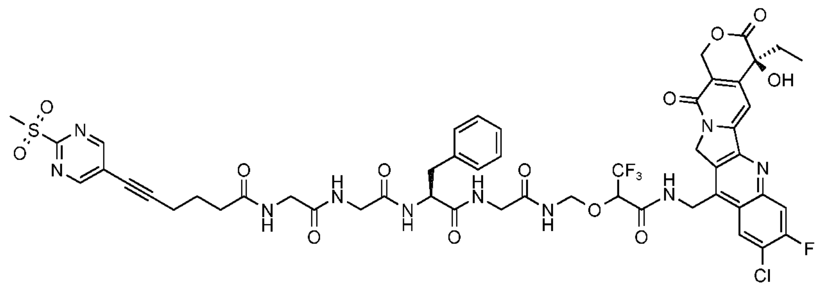

B-6:

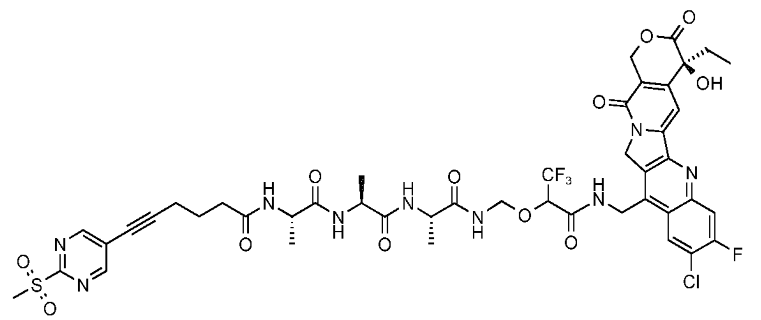

B-7:

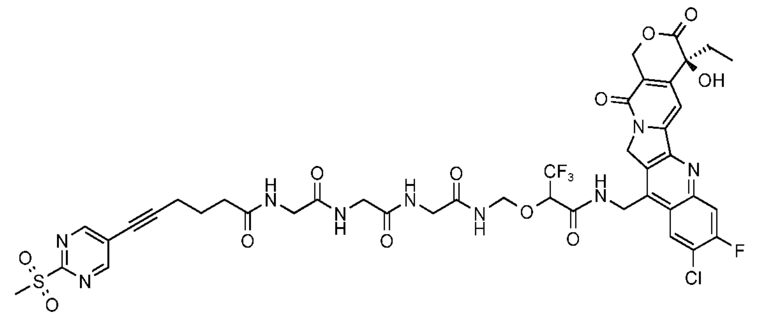

B-8:

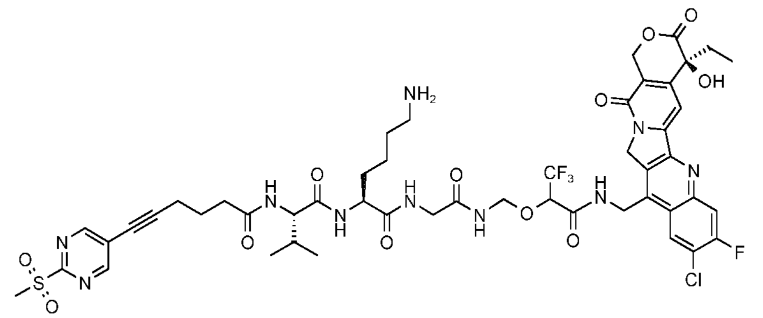

B-9:

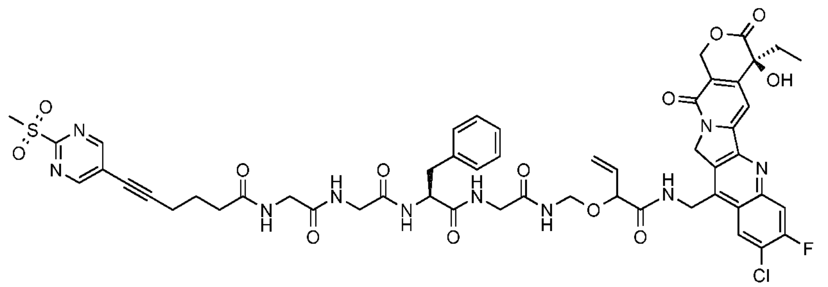

B-10:

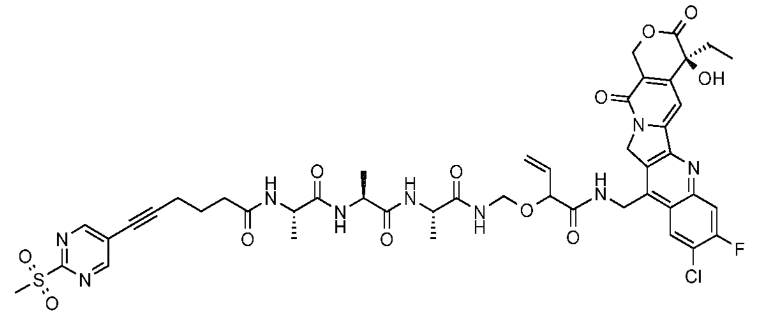

B-11:

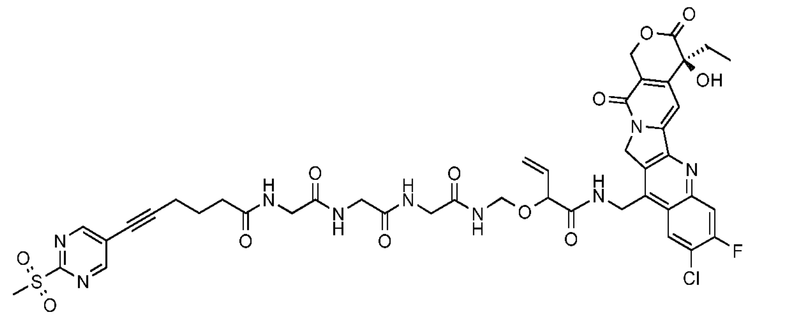

B-12:

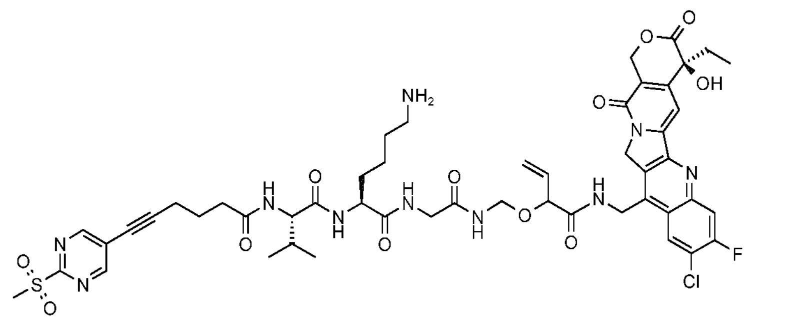

C-1:

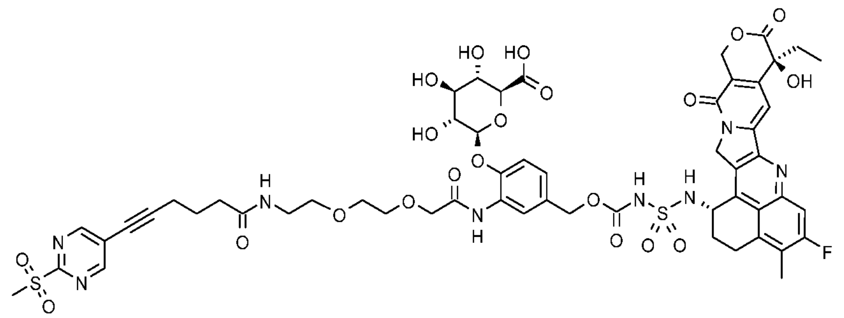

C-2:

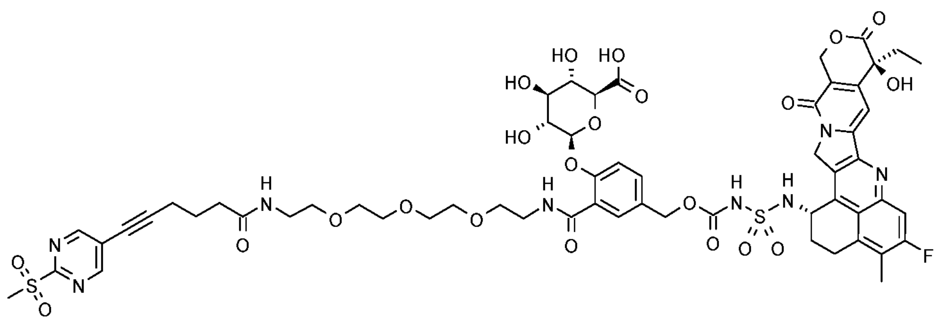

C-3:

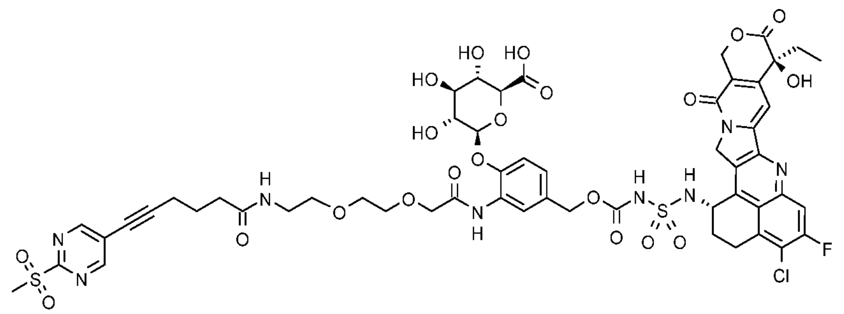

C-4:

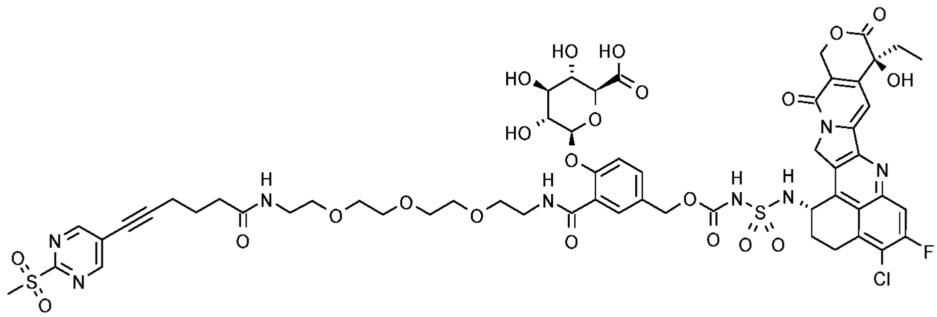

C-5:

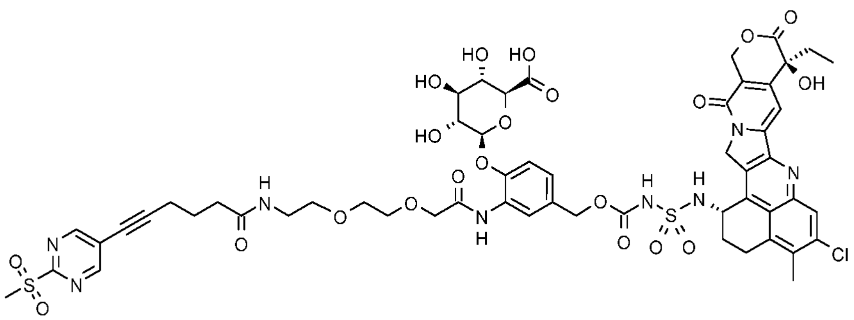

C-6:

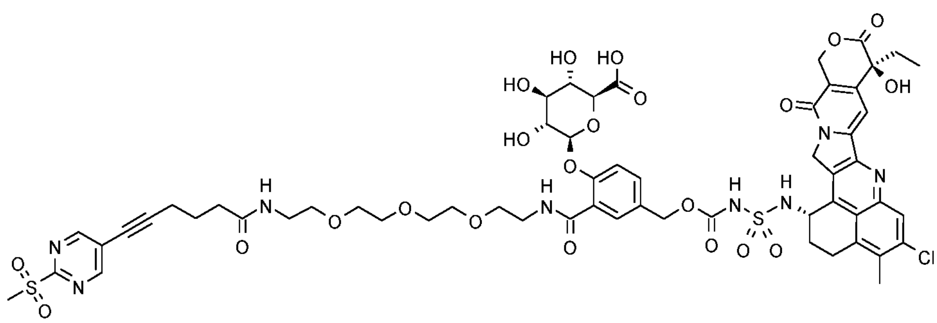

C-7:

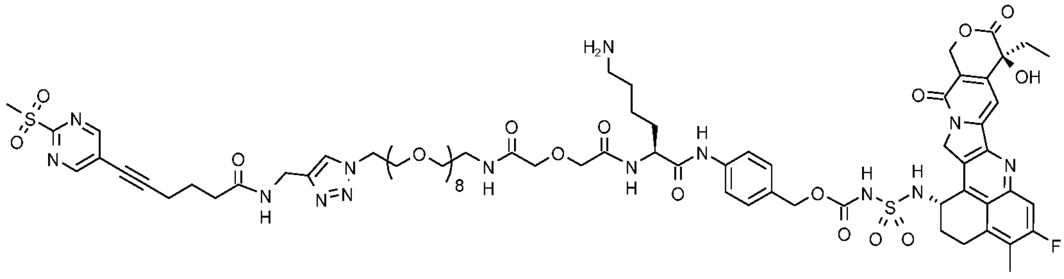

C-8:

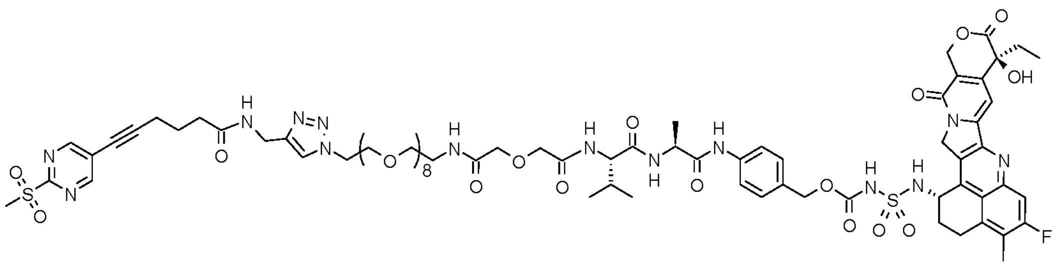

C-9:

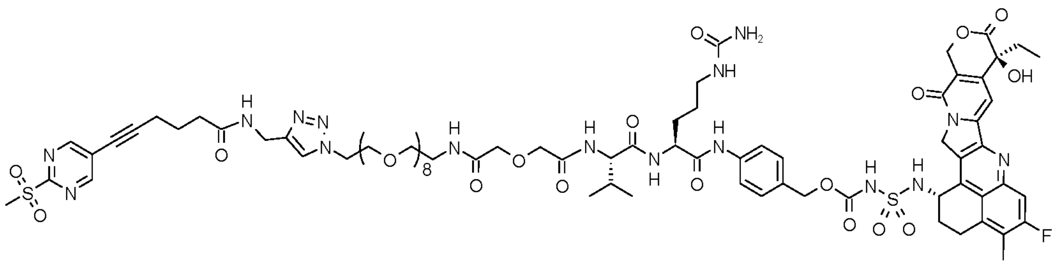

C-10:

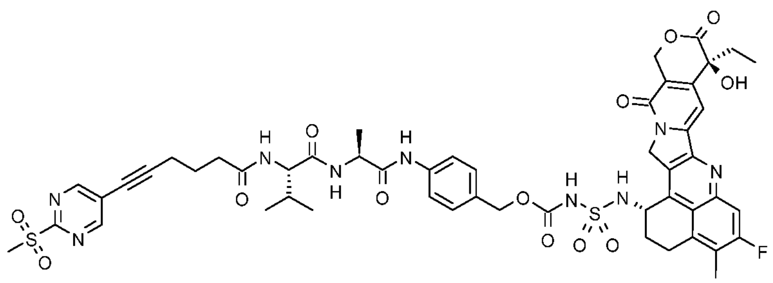

C-11:

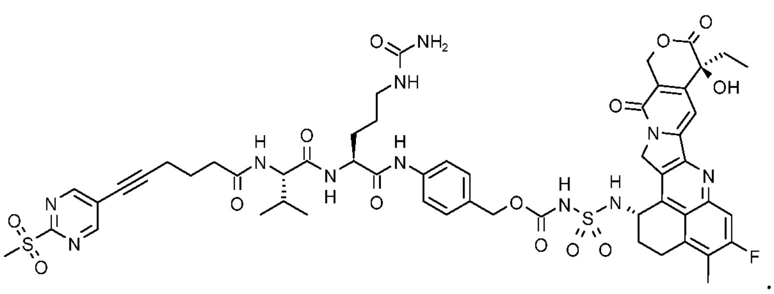

.

On the other hand, the present invention further provides an antibody-drug conjugate (ADC) represented by

Formula (VI):

Ab-(M-L-E-D)$_x$ Formula (VI)

wherein,

Ab represents an antibody or antigen-binding fragment thereof;

M represents a linking site connecting the antibody or antigen-binding fragment thereof;

L represents a linker connecting the linking site M and E;

E represents a structural fragment connecting L and D;

D represents a structural fragment of a cytotoxic drug;

x is 1 to 10.

In some embodiments, M, L, E, and D in Formula (VI) are as described above.

In some embodiments, the antibody-drug conjugate (ADC) is represented by Formula (VI-1):

Ab-M-L-E-D Formula (VI-1)

In some embodiments, Ab-(M-L-E-D)$_x$ is selected from the group consisting of the following structures:

ADC A-1:

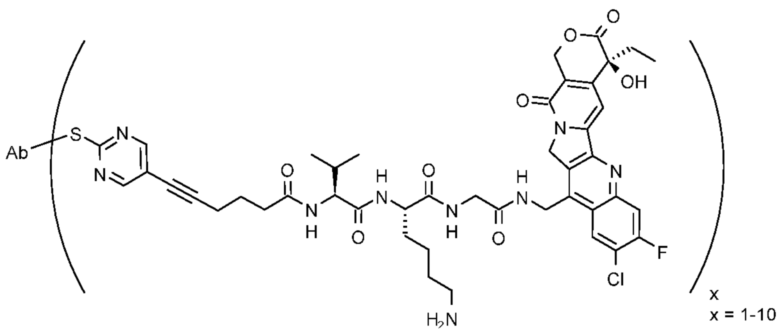

ADC A-2:

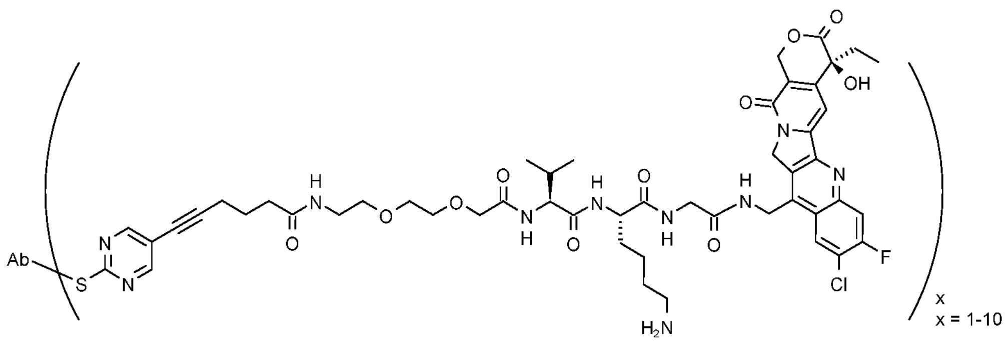

ADC A-3:

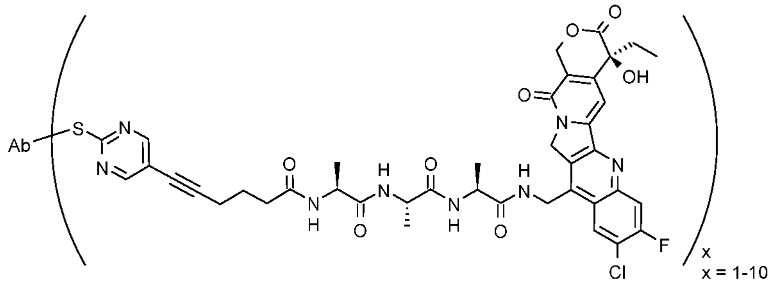

ADC A-4:

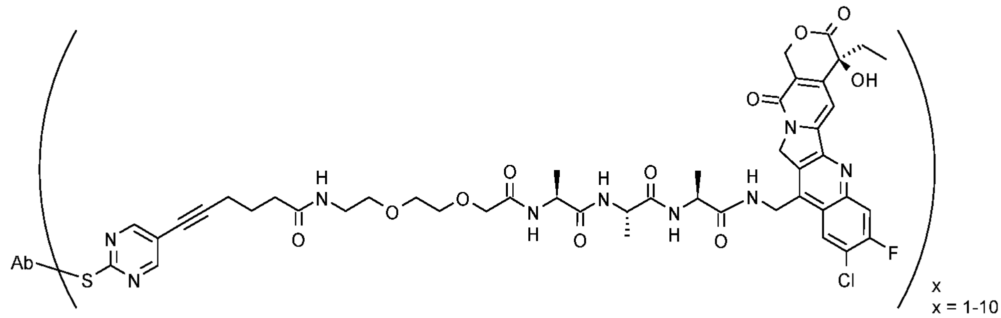

ADC A-5:

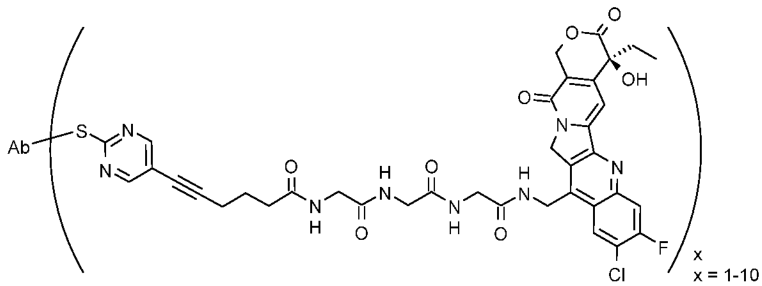

ADC A-6:

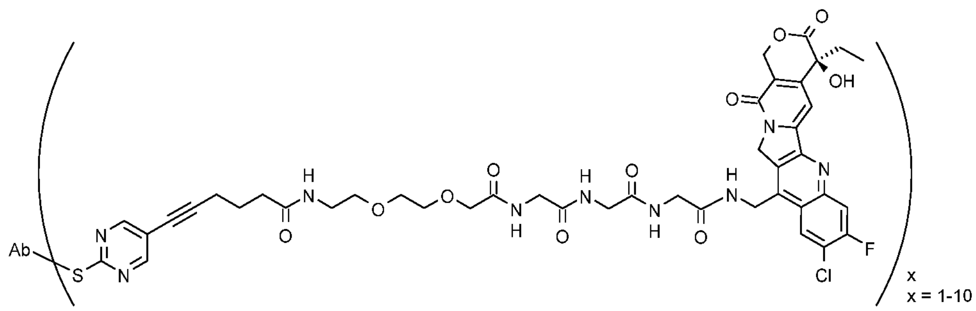

ADC A-7:

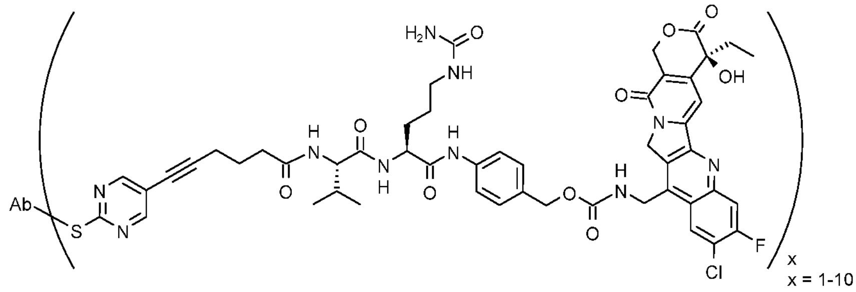

ADC A-8:

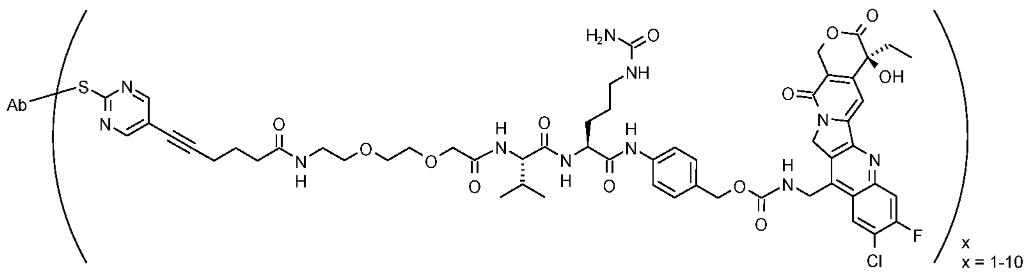

ADC A-9:

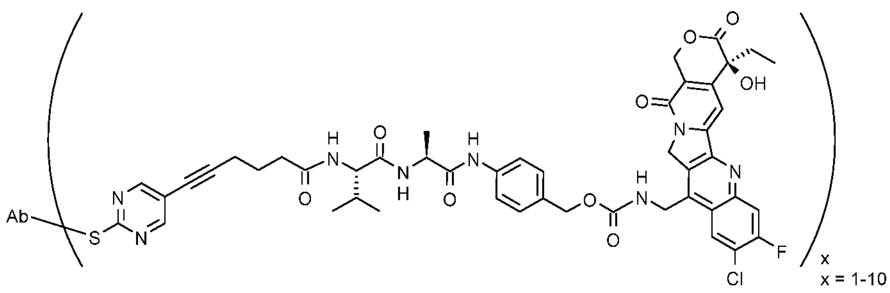

ADC A-10:

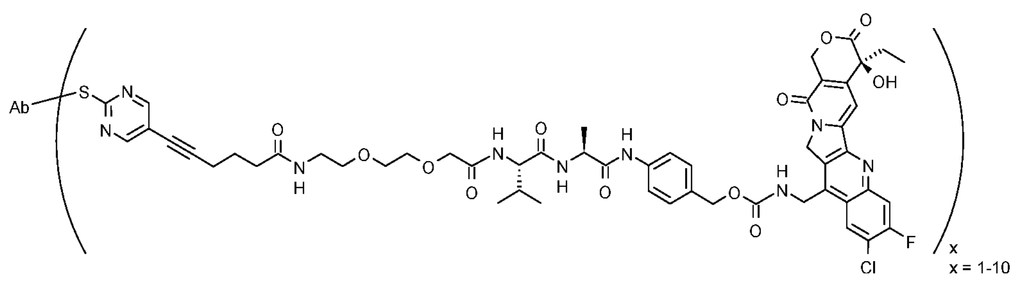

ADC A-11:

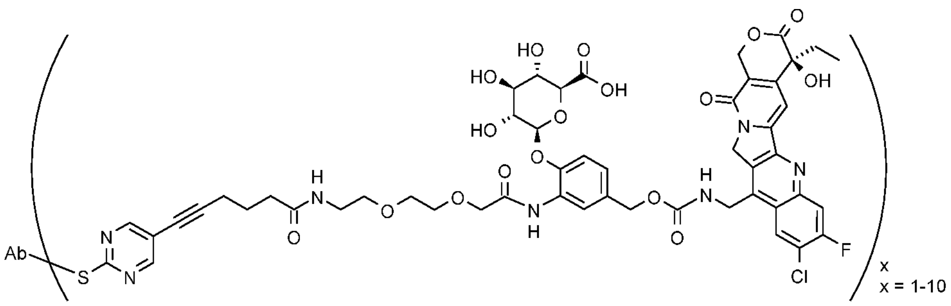

ADC A-12:

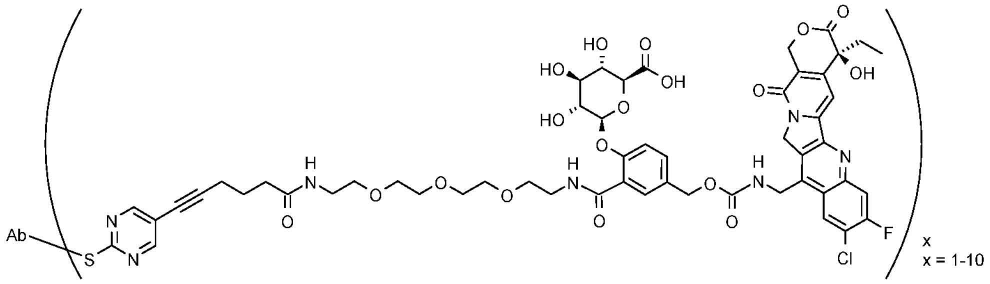

ADC B-1:

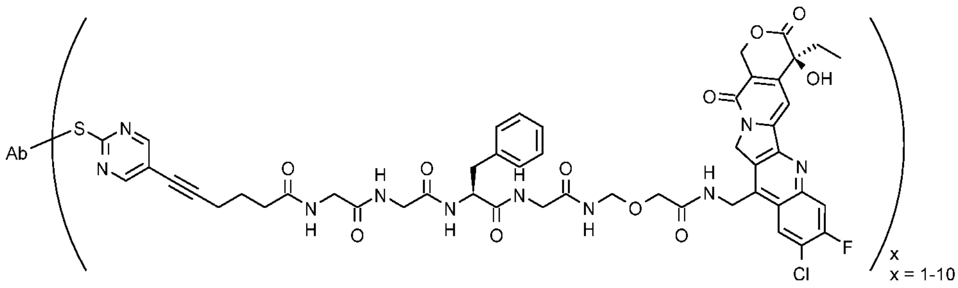

ADC B-2:

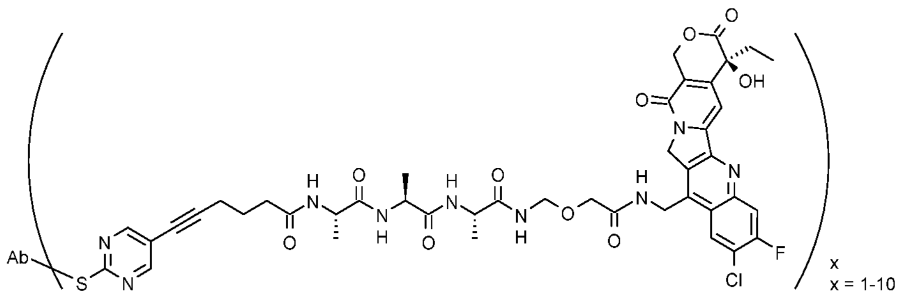

ADC B-3:

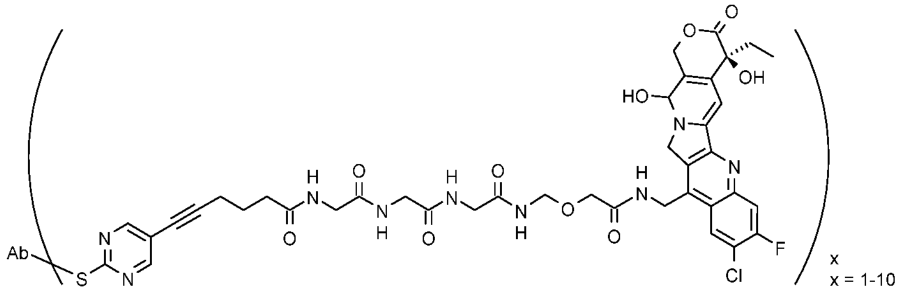

ADC B-4:

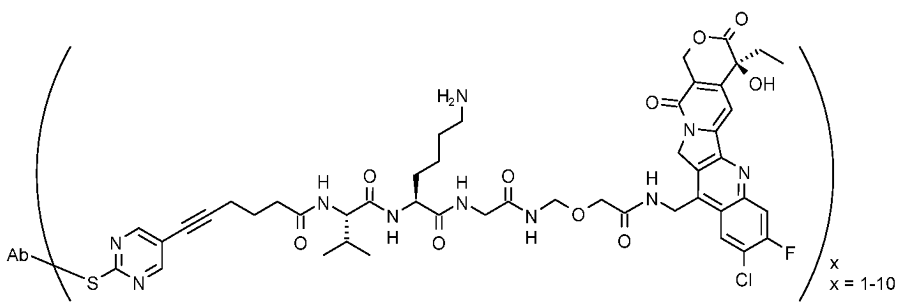

ADC B-5:

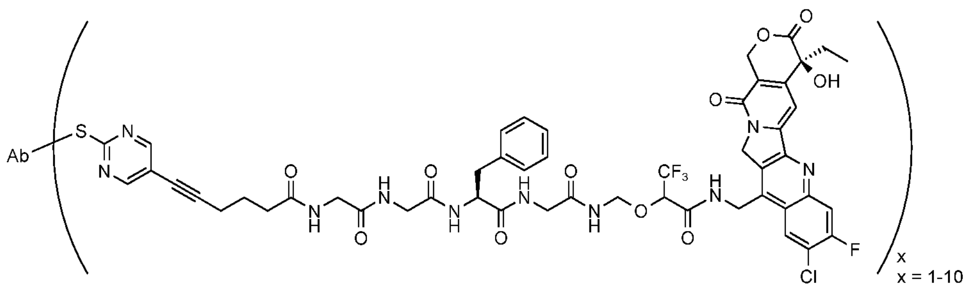

ADC B-6:

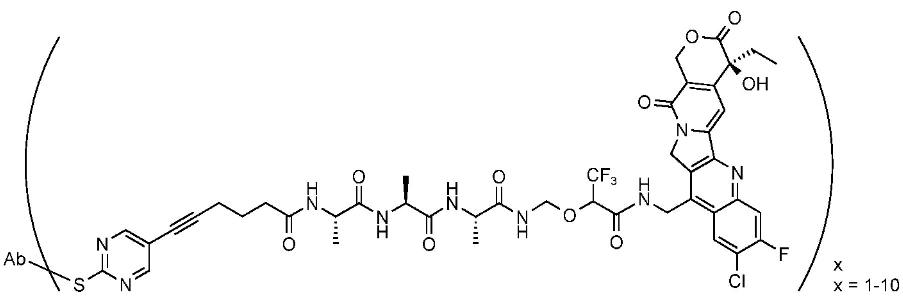

ADC B-7:

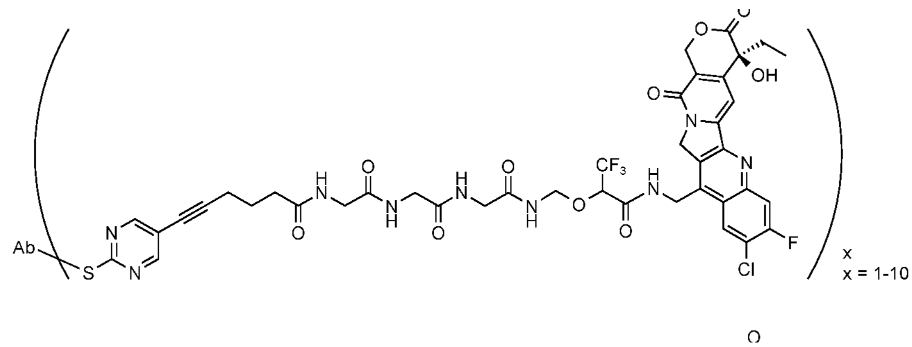

ADC B-8:

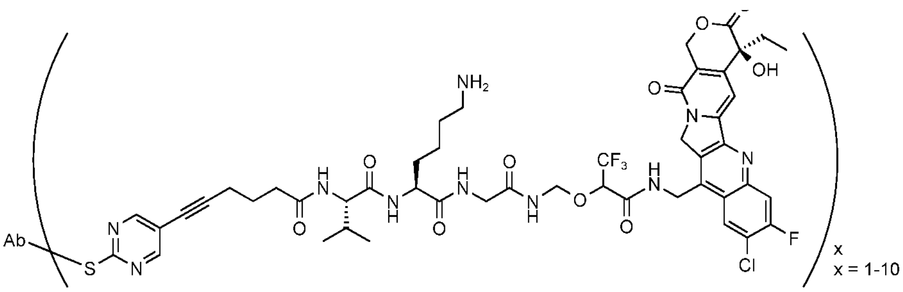

ADC B-9:

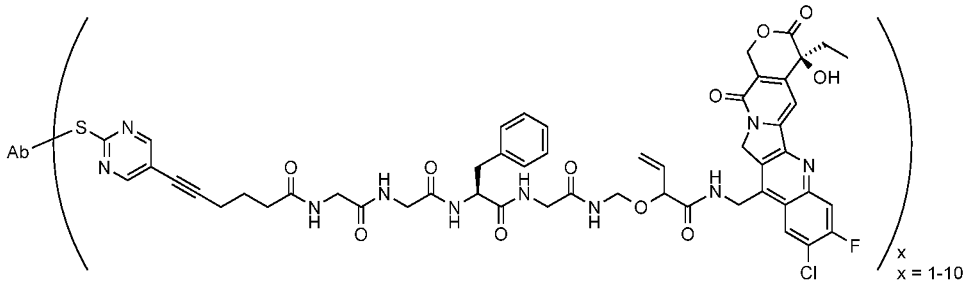

ADC B-10:

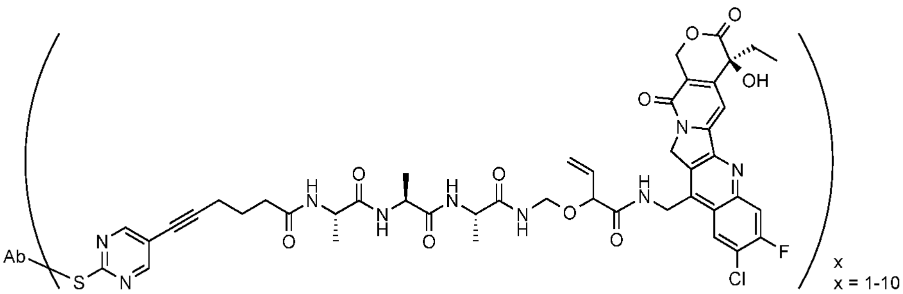

ADC B-11:

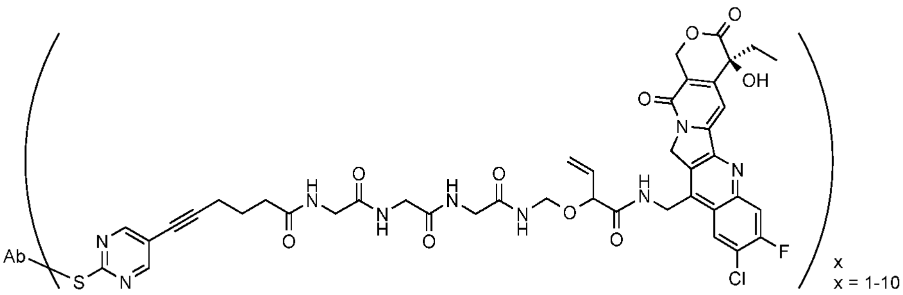

ADC B-12:

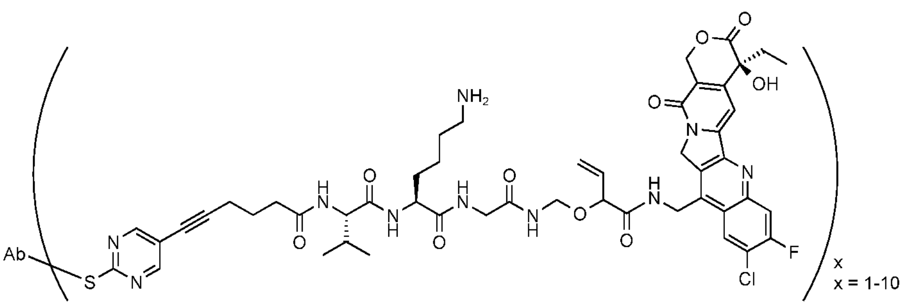

ADC C-1:

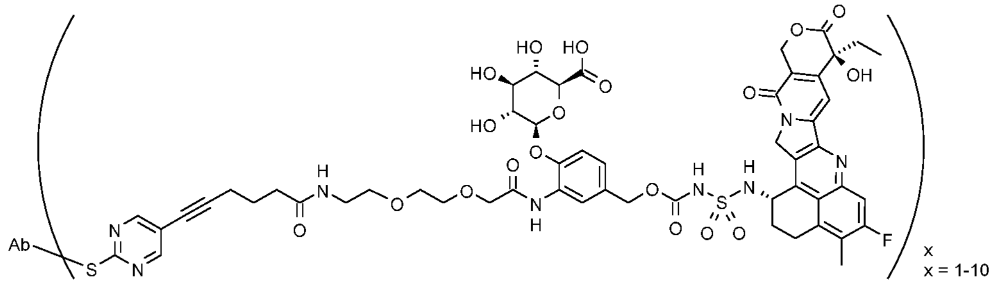

ADC C-2:

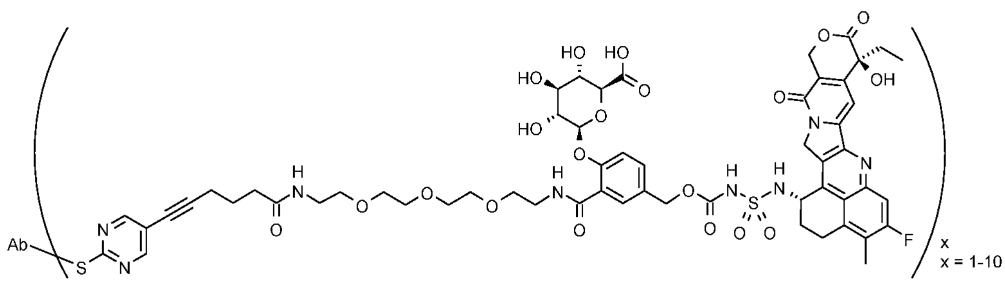

ADC C-3:

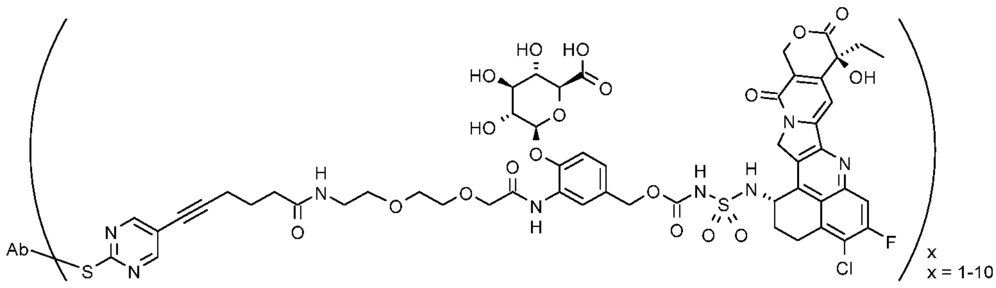

ADC C-4:

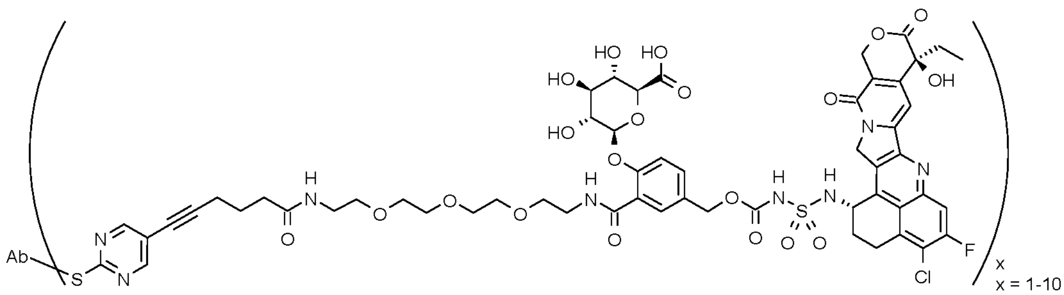

ADC C-5:

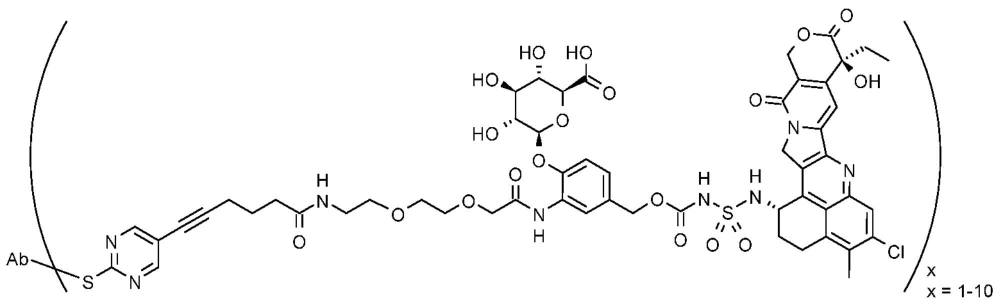

ADC C-6:

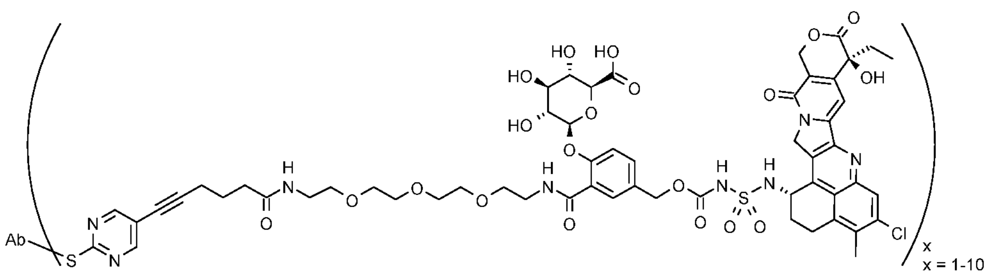

ADC C-7:

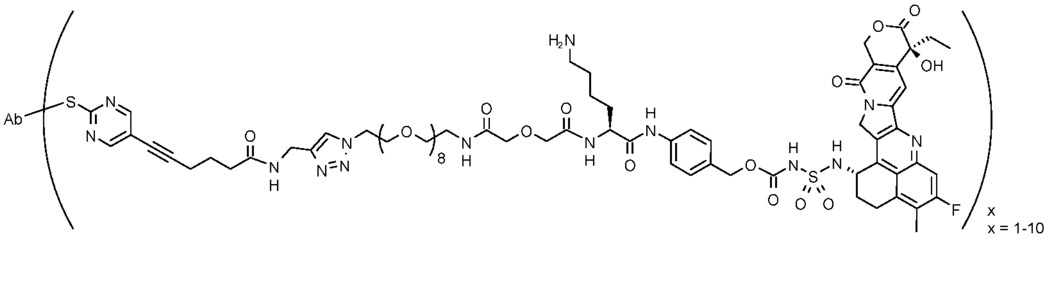

ADC C-8:

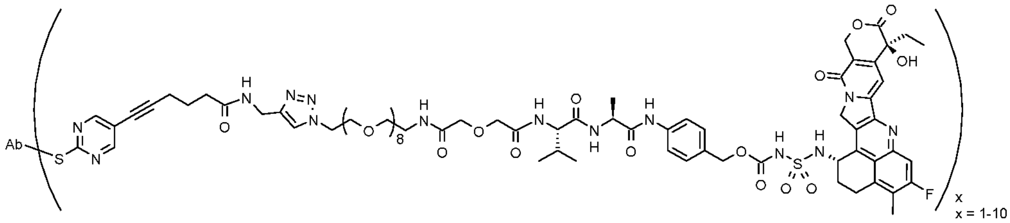

ADC C-9:

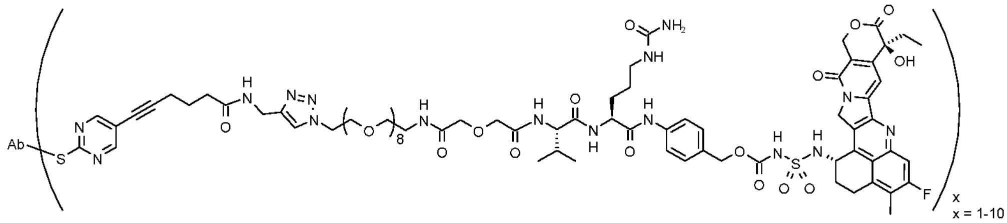

ADC C-10:

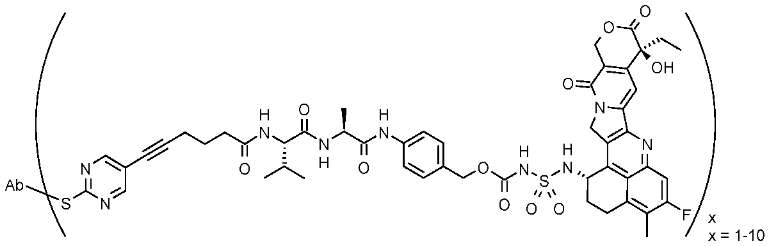

ADC C-11:

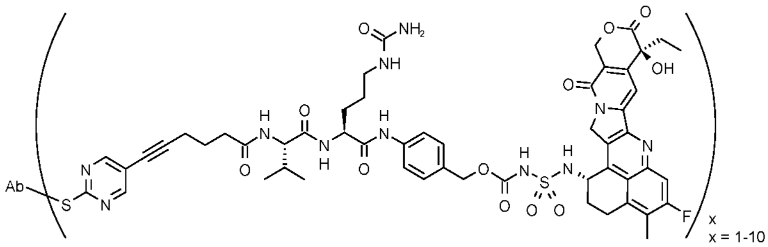

In some embodiments, x is 1 to 10, for example: 1 to 2, 1 to 3, 1 to 4, 1 to 5, 1 to 6, 1 to 7, 1 to 8, 1 to 9, 1 to 10, 2 to 3, 2 to 4, 2 to 5, 2 to 6, 2 to 7, 2 to 8, 2 to 9, 2 to 10, 3 to 4, 3 to 5, 3 to 6, 3 to 7, 3 to 8, 3 to 9, 3 to 10, 4 to 5, 4 to 6, 4 to 7, 4 to 8, 4 to 9, 4 to 10, 5 to 6, 5 to 7, 5 to 8, 5 to 9, 5 to 10, 6 to 7, 6 to 8, 6 to 9, 6 to 10, 7 to 8, 7 to 9, 7 to 10, 8 to 9, 8 to 10, or 9 to 10, preferably 3 to 9.

In some embodiments, x is 1, 2, 3, 4, 5, 6, 7, 8, 9 or 10.

In some embodiments, the present invention further provides the following antibody-drug conjugates (ADCs) or pharmaceutically acceptable salts, esters, stereoisomers, polymorphs, solvates, N-oxides, and isotopically labeled compounds, metabolites and prodrugs thereof:

Trastuzumab A-11:

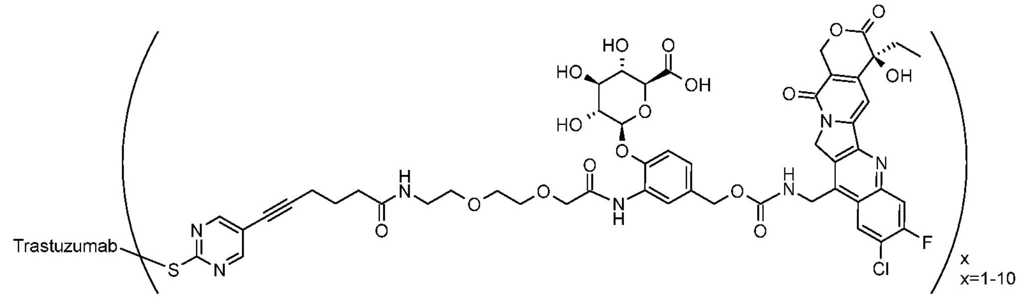

Trastuzumab B-1:

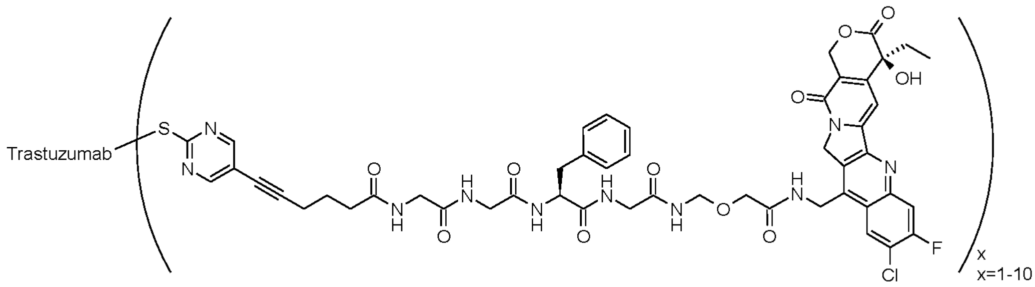

Trastuzumab B-2:

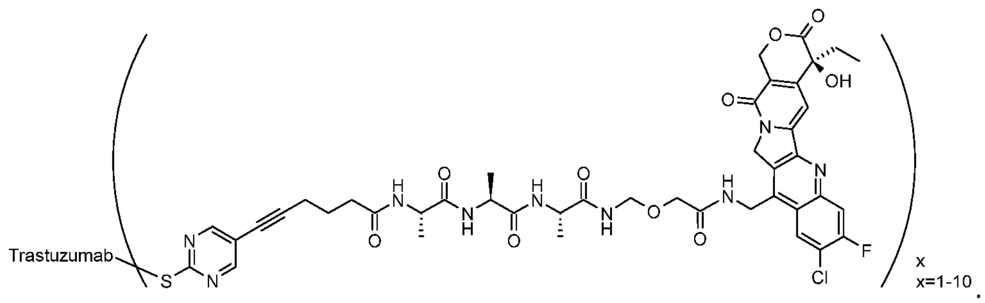

In another aspect, the present application provides a composition of the antibody-drug conjugate (ADC) as described herein. Such composition may comprise a plurality of ADCs described herein, wherein each ADC comprises a drug-linker described herein, wherein x is independently 1, 2, 3, 4, 5, 6, 7, 8, 9 or 10. In other words, each antibody molecule in the composition can be conjugated to 1, 2, 3, 4, 5, 6, 7, 8, 9 or 10 drug-linker. Accordingly, the composition is characterized by a "drug-to-antibody" ratio (DAR) in the range of about 1 to about 10. Methods for determining DAR are well known to the skilled artisan and include methods using reversed phase chromatography or HPLC-MS.

For example, in any embodiment, the composition of ADC described herein has a DAR of about 1 to about 10, or any subrange therebetween, such as: about 1 to 2, about 1 to 3, about 1 to 4, about 1 to 5, about 1 to 6, about 1 to 7, about 1 to 8, about 1 to 9, about 1 to 10, about 2 to 3, about 2 to 4, about 2 to 5, about 2 to 6, about 2 to 7, about 2 to 8, about 2 to 9, about 2 to 10, about 3 to 4, about 3 to 5, about 3 to 6, about 3 to 7, about 3 to 8, about 3 to 9, about 3 to 10, about 4 to 5, about 4 to 6, about 4 to 7, about 4 to 8, about 4 to 9, about 4 to 10, about 5 to 6, about 5 to 7, about 5 to 8, about 5 to 9, about 5 to 10, about 6 to 7, about 6 to 8, about 6 to 9, about 6 to 10, about 7 to 8, about 7 to 9, about 7 to 10, about 8 to 9, about 8 to 10, or about 9 to 10.

In certain embodiments, the ADC composition described herein has a DAR of about 3 to 9, such as about 3.0 to 3.5, about 3.0 to 4.0, about 3.0 to 4.5, about 3.0 to 5.0, about 3.0 to 6.0, about 3.5 to 4.0, about 3.5 to 4.5, about 3.5 to 5.0, about 3.5 to 5.5, about 3.5 to 6.0, about 3.5 to 6.5 to 6 about 4.0 to 4.5, about 4.0 to 5.0, about 4.0 to 5.5, about 4.0 to 6.0, about 4.0 to 6.5, about 4.0 to 7.0, about 4.0 to 8.0, about 4.5 to 5.0, about 4.5 to 5.5, about 4.5 to 6.0, about 4.5 to 6.5, about 4.5 to 7.0, about 4.5 to 7.5, about 5.0 to 8.0, about 5.5 to 6.0, about 5.5 to 6.5, about 5.5 to 7.0, about 5.5 to 7.5, about 5.5 to 8.0, about 6.0 to 6.5, about 6.0 to 7.0, about 6.0 to 7.5, about 6.0 to 8.5, about 6.5 to 7.0, about 6.5 to 7.5, about 6.5 to 7.5, about 6.5 to 8.5, about 7.0 to 7.5, about 7.5 to 8.0, about 7.5 to 8.5.

In some embodiments, the present invention further provides a composition comprising one or more antibody-drug conjugates as follows, and the composition has a DAR value (drug-to-antibody conjugation ratio) of 7.5 to 8.5, preferably 7.5 to 8.0, further preferably 8.0:

Trastuzumab A-11:

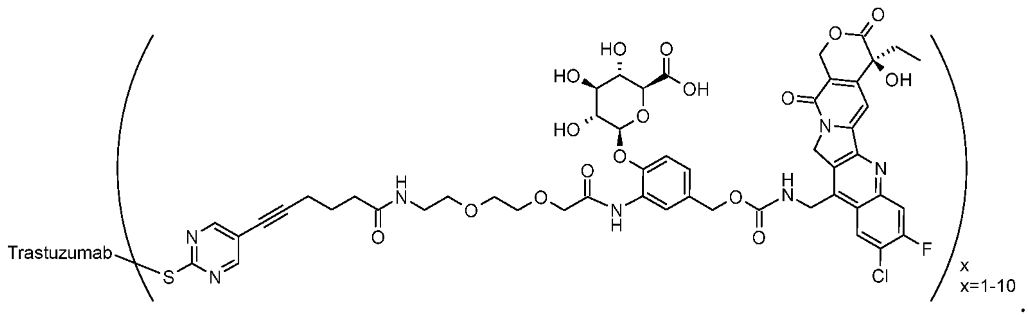

In some embodiments, the present invention further provides a composition comprising one or more antibody-drug conjugates as follows, and the composition has a DAR value (drug-to-antibody conjugation ratio) of 7.5 to 8.5, preferably 7.5 to 8.0, further preferably 7.96:
Trastuzumab B-1:

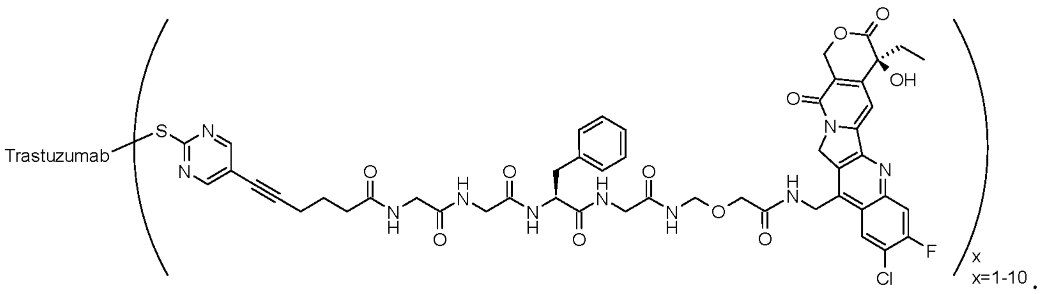

In some embodiments, the present invention also provides a composition comprising one or more of the following antibody-drug conjugates, wherein the composition has a DAR value (drug-to-antibody conjugation ratio) of 7.5 to 8.5, preferably 7.5 to 8.0, further preferably 7.95:
Trastuzumab B-2:

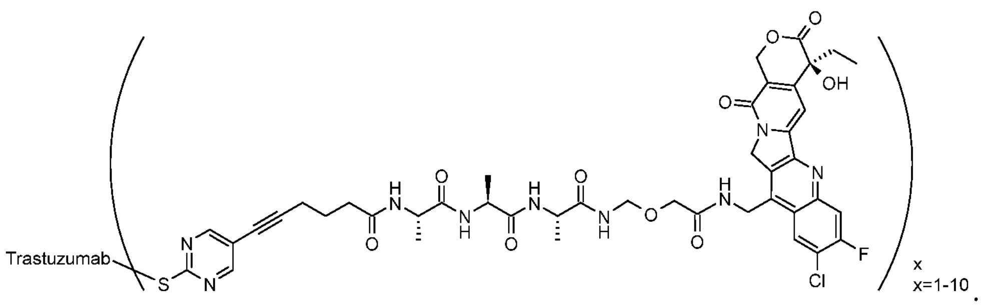

Definitions

Unless otherwise defined below, all technical and scientific terms used herein are intended to have the same meaning as commonly understood by one of ordinary skill in the art. References to technology as used herein are intended to mean technology as commonly understood in the art, including those obvious changes or equivalent substitutions of the technology that are apparent for those skilled in the art. Although the following terms are believed to be well understood by those skilled in the art, the following definitions are set forth to better explain the present invention.
The terms "comprise", "include", "have", "contain" or "involve" and their other variations herein are inclusive or open-ended and do not exclude other unrecited elements or method steps.
As used herein, the sign "*" marked in structure of a compound indicates that the marked carbon atom is a chiral carbon atom, and the present invention comprise a pair of enantiomers formed by the chiral carbon atom. For example, if a compound contains two different chiral carbon atoms, the present invention comprises four optical isomers formed by the chiral carbon atoms.

As used herein, " 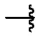 " refers to a site at which a structural fragment is connected to other part of the molecule.

The term "alkyl" is defined as a saturated straight- or branched-chain aliphatic hydrocarbonyl. In some embodiments, an alkyl has 1 to 12, such as 1 to 6 carbon atoms. For example, as used herein, the term "$C_{1-6}$ alkyl" refers to a linear or branched group of 1 to 6 carbon atoms (e.g., methyl, ethyl, n-propyl, isopropyl, n-butyl, isobutyl, sec-butyl, tert-butyl, n-pentyl and n-hexyl), which is optionally substituted with one or more (e.g., 1, 2 or 3) suitable substituents.

The term "alkenyl" refers to a straight- or branched-chain hydrocarbonyl containing at least one carbon-carbon double bond, including, for example, "$C_{2-6}$ alkenyl", "$C_{2-4}$ alkenyl", etc. Examples thereof include, but are not limited to: vinyl, 1-propenyl, 2-propenyl, 1-butenyl, 2-butenyl, 1,3-butadienyl, 1-pentenyl, 2-pentenyl, 3-pentenyl, 1,3-pentadienyl, 1,4-pentadienyl, 1-hexenyl, 2-hexenyl, 3-hexenyl, 1,4-hexadienyl, etc.

The term "alkynyl" refers to a straight- or branched-chain hydrocarbonyl containing at least one carbon-carbon triple bond, including, for example, "$C_{2-6}$ alkynyl", "C4-6 alkynyl" and the like. Examples thereof include, but are not limited to: ethynyl, 1-propynyl, 2-propynyl, 1-butynyl, 2-butynyl, 1,3-butadiynyl, 1-pentynyl, 2-pentynyl, 3-pentynyl, 1,3-pentadiynyl, 1,4-pentadiynyl, 1-hexynyl, 2-hexynyl, 3-hexynyl, 1,4-hexadiynyl, etc.

The term "cycloalkyl" refers to a saturated cyclic hydrocarbonyl, including but not limited to monocycloalkyl and bicycloalkyl (e.g., spirocycloalkyl, fused cycloalkyl and bridged cycloalkyl). The term "$C_{3-6}$ cycloalkyl" refers to a cycloalkyl having 3 to 6 ring-forming carbon atoms, such as cyclopropyl, cyclobutyl, cyclopentyl, cyclohexyl, etc., which may be optionally substituted with one or more (e.g., 1, 2 or 3) suitable substituents, such as methyl-substituted cyclopropyl.

The term "carbocycle" or "carbocyclyl" refers to a saturated or partially unsaturated non-aromatic monocyclic or polycyclic structure, a hydrocarbonyl connected through ring carbons. Examples thereof include, but are not limited to, cyclopropyl, cyclobutyl, cyclopentyl, cyclopentenyl, cyclohexyl, cyclohexenyl, cycloheptyl, and cyclooctyl.

The term "carbocycle" refers to a saturated or unsaturated non-aromatic monocyclic or polycyclic (e.g., bicyclic) hydrocarbon ring (e.g., a monocyclic ring such as cyclopropane ring, cyclobutane ring, cyclopentane ring, cyclohexane ring, cycloheptane ring, cyclooctane ring, cyclononane ring, or bicyclic ring, including spiro, fused or bridged systems (e.g., bicyclo[1.1.1]pentane ring, bicyclo[2.2.1]heptane ring, bicyclo[3.2.1]octane ring or bicyclo[5.2.0]nonane ring, decalin ring, etc.), which may be optionally substituted with one or more (e.g., 1, 2 or 3) suitable substituents. The term "3- to 6-membered carbocycle" refers to a carbocycle containing 3, 4, 5 or 6 ring-forming carbon atoms.

The term "heterocyclyl" or "heterocycle" refers to a saturated or partially saturated, monocyclic or polycyclic (e.g., bicyclic) non-aromatic cyclic structure in which the ring atoms are composed of carbon atoms and at least one (e.g., 1, 2 or 3) heteroatom selected from the group consisting of nitrogen, oxygen and sulfur. A heterocyclyl can be connected to the rest of molecule through any ring atom if the valence bond requirements are met. The heterocyclyl in the present invention is preferably a 3- to 6-membered heterocyclyl. The term "3- to 6-membered heterocyclyl" used in the present invention refers to a heterocyclyl with 3 to 6 ring atoms, including 3-membered heterocyclyl, 4-membered heterocyclyl, 5-membered heterocyclyl and 6-membered heterocyclyl, including nitrogen-containing heterocyclyl and oxygen-containing heterocyclyl, such as 4- to 6-membered heterocyclyl, for example, 4- to 6-membered nitrogen-containing heterocyclyl and 4- to 6-membered oxygen-containing heterocyclyl. Common heterocyclyl groups include (but are not limited to) azetidinyl, oxetanyl, tetrahydrofuryl, pyrrolidinyl, pyrrolidinonyl, imidazolidinyl, pyrazolidinyl, tetrahydropyranyl, piperidinyl, piperazinyl, morpholinyl. The heterocyclyl in the present invention may be optionally substituted with one or more substituents described in the present invention. The heterocyclyl in the present invention is optionally condensed with one or more aromatic or non-aromatic rings.

The term "oxygen-containing heterocycle" refers to the aforementioned heterocycle in which one or more (e.g., 1, 2 or 3) ring atoms are oxygen atoms, such as 5- to 6-membered oxygen-containing heterocycle. Specific examples include but are not limited to oxirane ring, tetrahydrofuran ring, furan ring, tetrahydropyran ring, pyran ring, etc. The "nitrogen-containing heterocycle" used in the present invention refers to the heterocycle as described above in which one or more (e.g., 1, 2 or 3) ring atoms are nitrogen atoms.

The term "haloalkyl" refers to an alkyl substituted with one or more (e.g., 1, 2 or 3) same or different halogen atoms, where the alkyl is as defined above. For example, the term "$C_{1-6}$ haloalkyl" as used in the present invention refers to a haloalkyl having 1 to 6 carbon atoms. Common haloalkyl groups include (but are not limited to) $-CH_2F$, $-CHF_2$, $-CF_3$, $-CH_2CF_3$, $-CF_2CF_3$, $-CH_2CH_2CF_3$, $-CH_2Cl$, etc. The haloalkyl groups in the present invention are optionally substituted with one or more substituents described in the present invention.

The term "alkoxy" refers to a group having the structure "alkyl-O-", wherein the alkyl is as defined above, for example, $C_{1-6}$ alkoxy, $C_{1-4}$ alkoxy, $C_{1-3}$ alkoxy or $C_{1-2}$ alkoxy, etc. Common alkoxy groups include (but are not limited to) methoxy, ethoxy, n-propoxy, isopropoxy, n-butoxy, isobutoxy, tert-butoxy, pentyloxy, hexyloxy, et al. The alkoxy groups in the present invention are optionally substituted with one or more substituents described in the present invention.

The term "alkoxyalkyl" refers to an alkyl substituted with one or more (e.g., 1, 2, 3 or 4) alkoxy groups, wherein the alkoxy and alkyl are as defined above. For example, the term "$C_{1-6}$ alkoxyalkyl" as used in the present invention refers to an alkyl having 1 to 6 carbon atoms and substituted with one or more (e.g., 1, 2, 3 or 4) alkoxy groups. Common alkoxyalkyl groups include (but are not limited to) $CH_3O-CH_2-$, $C_2H_5-O-CH_2-$, $C_2H_5-O-CH_2CH_2-$, etc.

The term "halo" or "halogen" group is defined to include F, Cl, Br or I.

The term "N-oxide" refers to an oxide (e.g., a mono- or di-oxide) of at least one nitrogen atom in the compound structure of the present application. N-mono-oxide may exist as a single positional isomer or as a mixture of positional isomers.

The term "substitution" refers to a selective replacement of one or more (e.g., one, two, three or four) hydrogen atoms on a designated atom by a specific group, provided that the designated atom still has normal valence at this situation and the substitution results in a stable compound. Combinations of substituents and/or variables are permissible only if such combinations result in stable compounds.

If a substituent is described as "optionally substituted," the substituent may be (1) unsubstituted, or (2) substituted. If a carbon of a substituent is described as optionally substituted with one or more substituents in the substituent list, one or more hydrogen atoms (to the extent of any hydrogen present) on the carbon may be individually and/or together substituted with independently selected optional substituents. If a nitrogen of a substituent is described as optionally substituted with one or more of the substituents listed, then one or more hydrogen atoms (to the extent of any hydrogen present) on the nitrogen each may be substituted with independently selected optional substituent.

If a substituent is described as being "independently selected from" a group, each substituent is selected independently of the other. Thus, each substituent may be the same as or different from another (other) substituent.

As used herein, the term "one or more" means 1 or more than 1, such as 2, 3, 4, 5 or 10 under reasonable conditions.

Unless otherwise specified, as used herein, the connecting site of a substituent may be from any suitable position on the substituent.

The term "stereoisomer" refers to an isomer formed due to at least one asymmetric center. In a compound with one or more (e.g., one, two, three or four) asymmetric centers, it may result in racemic mixtures, single enantiomers, diastereomeric mixtures and individual diastereomers. Certain individual molecules may also exist as geometric isomers (cis/trans). Similarly, the compound of the present invention may exist as a mixture of two or more structurally distinct forms (often referred to as tautomers) in rapid equilibrium. Representative examples of tautomers include keto-enol tautomers, phenol-ketone tautomers, nitroso-oxime tautomers, and imine-enamine tautomers, etc. It is to be understood that the scope of the present application encompasses all such isomers or mixtures thereof in any ratio (e.g., 60%, 65%, 70%, 75%, 80%, 85%, 90%, 95%, 96%, 97%, 98% or 99%).

Carbon-carbon bonds of the compound of the present invention may be depicted herein using solid lines (-), solid wedges (  ), or dashed wedges ( 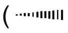 ). The use of a solid line to depict a bond to an asymmetric carbon atom is intended to indicate that all possible stereoisomers (e.g., specific enantiomers, racemic mixtures, etc.) at that carbon atom are included. The use of a solid or dashed wedge to depict a bond to an asymmetric carbon atom is intended to indicate that the stereoisomer as shown exists. When present in a racemic mixture, solid and dashed wedges are used to define relative stereochemistry rather than absolute stereochemistry. Unless otherwise specified, the compound of the present invention is intended to exist in the form of stereoisomers (which include cis and trans isomers, optical isomers (e.g., R and S enantiomers), diastereoisomers, geometric isomers, rotamers, conformational isomers, atropisomers and mixtures thereof). The compound of the present invention may exhibit two or more types of isomerism and consist of mixtures thereof (e.g., racemic mixtures and pairs of diastereoisomers).

The present invention encompasses all possible crystalline forms or polymorphs of the compound of the present invention, which may be a single polymorph or a mixture of two or more polymorphs in any proportion.

It will also be understood that certain compounds of the present invention may exist in free form for therapeutic use, or, where appropriate, as pharmaceutically acceptable derivatives thereof. In the present invention, pharmaceutically acceptable derivatives include, but are not limited to, pharmaceutically acceptable salts, esters, solvates, metabolites or prodrugs that, upon administration to a patient in need thereof, can directly or indirectly provide the compounds of the present invention or metabolites or residues thereof. Therefore, when reference is made herein to "the compound of the present invention", it is also intended to encompass the various derivative forms of the compound described above.

Pharmaceutically acceptable salts of the compounds of the present invention include acid addition salts and base addition salts thereof.

Suitable acid addition salts are formed from acids that form pharmaceutically acceptable salts, including aspartate, fumarate, glucoheptonate, gluconate, glucuronate, hexafluorophosphate, and the like.

Suitable base addition salts are formed from bases that form pharmaceutically acceptable salts, including aluminum salts, arginine salts, choline salts, diethylamine salts, and the like.

For a review of suitable salts, see Stahl and Wermuth, "Handbook of Pharmaceutical Salts: Properties, Selection, and Use" (Wiley-VCH, 2002). Methods for preparing pharmaceutically acceptable salts of the compounds of the present invention are known to those skilled in the art.

The term "ester" refers to an ester derived from the compound of each general formula herein, including physiologically hydrolyzable ester (which can be hydrolyzed under physiological conditions to release the compound of the present invention in the form of free acid or alcohol). The compound of the present invention may itself be an ester.

The compound of the present invention may exist in the form of solvate, preferably hydrate, wherein the

compound of the present invention comprises a polar solvent, in particular such as water, methanol or ethanol, as a structural element of the crystal lattice of the compound. The amount of the polar solvent, especially water, may be present in a stoichiometric or non-stoichiometric ratio.

Also included within the scope of the present invention is a metabolite of the compound of the present invention, that is a substance formed in the body upon administration of the compound of the present invention. Such product may result, for example, from oxidation, reduction, hydrolysis, amidation, deamidation, esterification, de-esterification, enzymatic hydrolysis, etc. of the administered compound. Accordingly, the present invention includes a metabolite of the compound of the present invention, including a compound prepared by contacting the compound of the present invention with a mammal for a time sufficient to produce metabolite thereof.

The present invention further includes within its scope a prodrug of the compound of the present invention. Typically, such prodrug is a functional derivative of the compound, which is readily converted in vivo to the desired therapeutically active compound. Therefore, in these instances, the term "administration" as used in the treatment method of the present invention shall include the treatment of various diseases or conditions with one or more prodrug forms of the compound sought to be protected, but the prodrug form is converted in vivo to the compound described above upon administration to an individual. For example, in "Design of Prodrug", ed. H. Bundgaard, Elsevier, 1985, general methods for selecting and preparing suitable prodrug derivatives are described.

The present invention further includes within its scope an isotopically labeled compound of the compound of the present invention, which is identical to the compound of the present invention except that one or more atoms are substituted with atoms having the same atomic number but different atomic mass or mass number from the atomic mass or mass number of atoms predominate in nature.

The present invention also encompasses the compound of the present invention that contains a protecting group. In any process for preparing the compound of the present invention, it may be necessary and/or desirable to protect a sensitive or reactive group on any relevant molecule, thereby forming a chemically protected form of the compound of the present invention. This can be achieved by using a conventional protecting group, for example, a protecting group as described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991, and these references are incorporated herein by reference. The protecting group can be removed at an appropriate subsequent stage by a method known in the art.

<u>Pharmaceutical composition</u>

In a third aspect, the present invention provides a pharmaceutical composition, which comprises the compound or pharmaceutically acceptable salt, ester, stereoisomer, polymorph, solvent, N-oxide, isotopically labeled compound, metabolite or prodrug thereof described in the first or second aspect of the present invention, and one or more pharmaceutically acceptable carriers.

The term "pharmaceutical composition" refers to a composition that can be used as a medicament and contains a pharmaceutically active ingredient (API) (or therapeutic agent) and optionally one or more pharmaceutically acceptable carriers. The term "pharmaceutically acceptable carrier" refers to an excipient that is administered with a therapeutic agent and that is suitable, within the scope of sound medical judgment, for contact with human and/or other animal tissue without undue toxicity, irritation, allergic reaction or other problems or complications commensurate with a reasonable benefit/risk ratio.

The above pharmaceutical composition can act systemically and/or locally, which can be achieved through suitable dosage forms. The dosage forms include, but are not limited to, tablets, capsules, lozenges, hard candies, powders, sprays, creams, ointments, suppositories, gels, pastes, lotions, ointments, aqueous suspensions, injectable solutions, elixirs, and syrups.

The above pharmaceutical composition may contain 0.01 mg to 1000 mg of at least one compound or pharmaceutically acceptable salt, ester, stereoisomer, polymorph, solvate, N-oxide, isotopically labeled compound, metabolite or prodrug thereof of the present invention.

The present invention also provides a method for preparing the above-mentioned pharmaceutical composition or corresponding dosage form thereof, which comprises combining at least one compound or pharmaceutically acceptable salt, ester, stereoisomer, polymorph, solvate, N-oxide, isotopically labeled compound, metabolite or prodrug thereof of the present invention with one or more pharmaceutically acceptable carriers.

Kit

In a fourth aspect, the present invention provides a kit, which comprises:

a) at least one compound or pharmaceutically acceptable salt, ester, stereoisomer, polymorph, solvate, N-oxide, isotopically labeled compound, metabolite or prodrug thereof according to the first or second aspect of the present

invention as a first therapeutic agent, or the pharmaceutical composition according to the third aspect as a first pharmaceutical composition;

b) optionally, at least one additional therapeutic agent as a second therapeutic agent, or a pharmaceutical composition containing an additional therapeutic agent as a second pharmaceutical composition; and

c) optionally, a packaging and/or instruction.

The above-mentioned kit may contain 0.01 mg to 1000 mg of at least one of the compound or pharmaceutically acceptable salt, ester, stereoisomer, polymorph, solvate, N-oxide, isotopically labeled compound, metabolite or prodrug thereof of the present invention.

The present invention also provides a method for preparing the above-mentioned kit, which comprises combining at least one of the compound or pharmaceutically acceptable salt, ester, stereoisomer, polymorph, solvate, N-oxide, isotopically labeled compound, metabolite or prodrug thereof of the present invention, or the pharmaceutical composition as described above with optionally at least one additional therapeutic agent or the pharmaceutical composition containing the additional therapeutic agent, packaging and/or instruction.

Medical use

The compound of the present invention can exhibit a strong effect in inhibiting abnormal cell proliferation.

Therefore, the present application provides the compound or pharmaceutically acceptable salt, ester, stereoisomer, polymorph, solvate, N-oxide, isotopically labeled compound, metabolite and prodrug thereof of the present invention or the pharmaceutical composition as described above, which is used for treating a disease associated with abnormal cell proliferation.

In addition, the present application also provides use of the compound or pharmaceutically acceptable salt, ester, stereoisomer, polymorph, solvate, N-oxide, isotopically labeled compound, metabolite and prodrug thereof of the present invention or the pharmaceutical composition as described above in the manufacture of a medicament for treating a disease associated with abnormal cell proliferation.

In some embodiments, the disease involving abnormal cellular proliferation includes, but is not limited to, tumor, such as advanced solid tumor.

The present application also provides use of the compound or pharmaceutically acceptable salt, ester, stereoisomer, polymorph, solvate, N-oxide, isotopically labeled compound, metabolite and prodrug thereof of the present invention or the pharmaceutical composition of the present invention in the manufacture of a preparation for inhibiting the proliferation of tumor cells. In certain embodiments, the preparation is used for in vivo or in vitro administration. For example, the preparation can be administered to a subject to inhibit the proliferation of tumor cells in the subject; alternatively, the preparation can be administered to cells in vitro (e.g., cell lines or cells from a subject), to inhibit the proliferation of tumor cells in vitro.

The tumors described in the present invention include (but are not limited to): brain tumor, lung cancer, squamous cell carcinoma, bladder cancer, gastric cancer, ovarian cancer, peritoneal cancer, pancreatic cancer, breast cancer, head and neck cancer, cervical cancer, endometrial cancer, colorectal cancer, liver cancer, kidney cancer, esophageal adenocarcinoma, squamous cell esophageal carcinoma, prostate cancer, female reproductive tract cancer, carcinoma in situ, lymphoma, neurofibroma, thyroid cancer, bone cancer, skin cancer, brain cancer, colon cancer, testicular cancer, gastrointestinal stromal tumor, prostate tumor, mast cell tumor, multiple myeloma, melanoma, glioma, or sarcoma.

Treatment method

In another aspect, the present invention provides a method for treating a disease associated with abnormal cell proliferation, which comprises the following steps: administering to an individual in need thereof with a therapeutically effective amount of the compound or pharmaceutically acceptable salt, ester, stereoisomer, polymorph, solvate, N-oxide, isotopically labeled compound, metabolite and prodrug thereof of the present invention or the pharmaceutical composition as described above.

The term "effective amount" refers to an amount capable of inducing a biological or medical response in a cell, tissue, organ or organism (e.g., an individual) and sufficient to achieve the desired preventive and/or therapeutic effect.

Dosage regimen can be adjusted to provide the best desired response. For example, it can be administered in a single dose, in divided doses over time, or in a proportionally reduced or increased dose according to the actual situation. It will be understood that, for any particular individual, specific dosage regimen should be adjusted according to the need and the professional judgment of the person administering or supervising the administration of the composition.

The amount of the compound of the present invention administered will depend on the individual circumstances,

severity of the disease or condition, rate of administration, disposition of the compound, and the judgment of the prescriber. Generally speaking, the effective amount is about 0.001 to 10000 mg/kg of subject's body weight/day. Where appropriate, the effective amount is about 0.01 to 1000 mg/kg subject's body weight/day. Administration can be carried out every day, every two days, or every three days at a dose of about 0.01 to 1000 mg/kg of subject's body weight, typically about 0.1 to 500 mg/kg of subject's body weight. Exemplary dosage regimen is once or more every day, or once or more every week, or once or more every month. When multiple doses are administered, the intervals between single doses may generally be every day, every week, every month, or every year. Alternatively, administration may be in the form of a sustained-release formulation, in which case low frequency of administration is required. The dosage and frequency of administration may vary based on the half-life of the drug in the subject and may vary based on whether the application is prophylactic or therapeutic. In prophylactic application, a relatively low dose is administrated at a relatively low frequency over a long period of time; in therapeutic applications, it is sometimes necessary to administer relatively high doses at shorter intervals until the progression of the disease is retarded or stopped, preferably until the individual demonstrates partial or complete improvement in disease symptoms, after which prophylactic application can be adopted.

The term "treatment" refers to the alleviation or elimination of a targeted disease or condition. If a subject receives a therapeutic amount of the compound or pharmaceutically acceptable form thereof of the present invention or the pharmaceutical composition of the present invention, at least one indicator and symptom of the subject shows observable and/or detectable remission and/or improvement, it indicates that the subject has been successfully "treated." It is understood that treatment includes not only complete treatment, but also less than complete treatment, but achieving some biologically or medically relevant results.

The term "administrate/administrating/administration" refers to a process in which a pharmaceutically active ingredient (e.g., the compound of the present invention) or a pharmaceutical composition containing a pharmaceutically active ingredient (e.g., the pharmaceutical composition of the present invention) is applied to an individual or cell, tissue, organ, biological fluid and other part thereof so as to bring the pharmaceutical active ingredient or the pharmaceutical composition into contact with the individual or cell, tissue, organ, biological fluid and other part thereof. Common administration methods include (but are not limited to) oral administration, subcutaneous administration, intramuscular administration, subperitoneal administration, ocular administration, nasal administration, sublingual administration, rectal administration, vaginal administration, etc.

The term "in need thereof" refers to a physician's or other caregiver's judgment that an individual needs or would benefit from a preventive and/or therapeutic procedure, and this judgment is based on a variety of factors that the physician or other caregiver has in his or her area of expertise.

The term "individual" (or subject) refers to a human or non-human animal. The individual of the present invention includes an individual (patient) suffering from a disease and/or disorder and a normal individual. The non-human animal of the present invention includes all vertebrates, such as non-mammals, such as birds, amphibians, reptiles, etc., and mammals, such as non-human primates, domestic animals and/or domesticated animals (e.g., sheep, dogs, cats, cows, pigs, etc.).

<u>Preparation method</u>

The fourth aspect of the present invention provides a method for synthesizing the compound.

When $R_3$ is hydrogen, the compound of Formula (I) in the present invention can be synthesized through the following synthetic route.

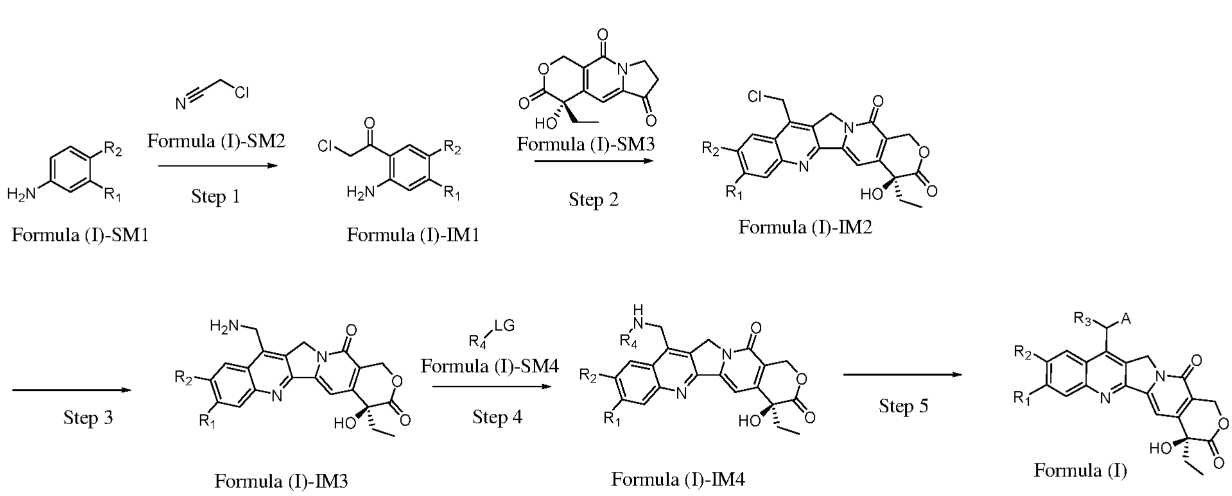

wherein, the meanings of $R_1$, $R_2$, $R_3$, $R_4$ and A are as mentioned above, LG is a leaving group and selected from the group consisting of methanesulfonyl, trifluoromethanesulfonyloxy and halogen, preferably trifluoromethanesulfonyloxy or iodine;

Step 1

Compound Formula (I)-SM1 and Compound Formula (I)-SM2 undergo Friedel-Crafts acylation reaction to obtain Compound Formula (I)-IM1;

In some embodiments, this step is carried out at a suitable temperature, which is 5°C, 20°C, 25°C, 40°C, 50°C, 60°C, 100°C, preferably 25°C.

Step 2

Compound Formula (IV)-IM1 and Compound Formula (I)-SM3 undergo ring-closing reaction under acidic conditions to obtain Compound Formula (I)-IM2;

In some embodiments, this step is carried out at a suitable temperature, which is 20°C, 25°C, 40°C, 50°C, 60°C, 100°C, 120°C, preferably 120°C;

In some embodiments, this step is carried out in a suitable organic solvent, which may be selected from the group consisting of toluene, xylene, N,N-dimethylformamide, N-methylpyrrolidone, dimethyl sulfoxide, preferably toluene and xylene;

In some embodiments, this step is carried out under acidic condition;

and the acidic conditions are provided by an agent selected from the group consisting of p-toluenesulfonic acid, hydrochloric acid, trifluoroacetic acid, formic acid, sulfuric acid, methanesulfonic acid, preferably p-toluenesulfonic acid.

Step 3:

Compound Formula (I)-IM2 undergoes Delépine reaction to obtain Compound Formula (I)-IM3
In some embodiments, this step is carried out at a suitable temperature, which is 5°C, 20°C, 25°C, 40°C, 50°C, 60°C, 85°C, 100°C, preferably 85°C.

Step 4:

Compound Formula (I)-IM3 and Compound Formula (I)-SM4 undergo substitution reaction to obtain Compound Formula (I)-IM4.

In some embodiments, this step is carried out at a suitable temperature, which is 20°C, 25°C, 40°C, 50°C, 60°C, 100°C, 140°C, preferably 50°C;

In some embodiments, this step is carried out in a suitable organic solvent, which may be selected from the group consisting of halogenated hydrocarbon (e.g., dichloromethane (DCM), chloroform (TCM), 1,2-dichloroethane (1,2-DCE), etc.), nitrile (e.g., acetonitrile (AN), etc.), *N*-methylpyrrolidone (NMP), N,N-dimethylformamide (DMF), N,N-dimethylacetamide (DMA), tetrahydrofuran (THF), 1,4-dioxane (Dioxane), dimethyl sulfoxide (DMSO) and any combination thereof, preferably acetonitrile.

In some embodiments, this step is carried out in the presence of a suitable base, which includes an organic base or an inorganic base. The organic base can be selected from the group consisting of N,N-diisopropylethylamine (DIPEA), triethylamine (TEA), potassium tert-butoxide (t-BuOK) and pyridine (Py), and the inorganic base can be selected from the group consisting of potassium phosphate ($K_3PO_4$), sodium hydride (NaH), potassium carbonate ($K_2CO_3$), sodium carbonate ($Na_2CO_3$), sodium bicarbonate ($NaHCO_3$), cesium carbonate ($Cs_2CO_3$) and NaOH, preferably $Na_2CO_3$ or $NaHCO_3$;

Step 5

When A is

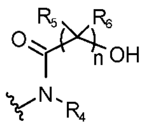

or

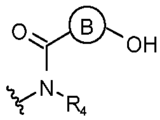

,

Compound Formula (I)-IM4 undergoes condensation reaction with Compound Formula (I)-SM5-1 or Compound Formula (I)-SM5-2 to obtain a Compound Formula (I), in whihch $R_5$, $R_6$, n and Ring B have the same meanings as mentioned above.

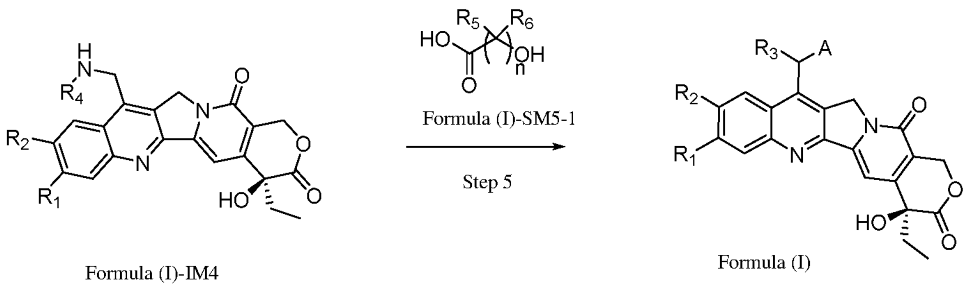


Formula (I)-IM4

Formula (I)

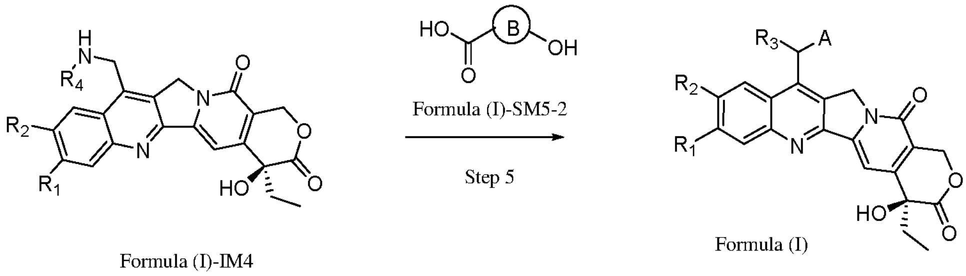


Formula (I)-IM4

Formula (I)

In some embodiments, this step is carried out in the presence of a suitable condensation reagent, which can be selected from the group consisting of HATU, HBTU, EDCI, DCC and HOBT, preferably HBTU and HATU;

In some embodiments, this step is carried out at a suitable temperature, which is 20°C, 25°C, 40°C, 50°C, 60°C, 100°C, preferably 25°C;

In some embodiments, this step is carried out in a suitable organic solvent, which may be selected from the group consisting of methanol, tetrahydrofuran, dichloromethane, *N,N*-dimethylformamide, N-methylpyrrolidone, dimethyl sulfoxide, n-heptane, n-hexane, ethyl acetate, preferably N,N-dimethylformamide.

In some embodiments, this step is carried out in the presence of a suitable base, which includes an organic base or an inorganic base, the organic base can be selected from the group consisting of DIPEA, TEA, t-BuOK and Py, the inorganic base can be selected from the group consisting of $K_3PO_4$, NaH, $K_2CO_3$, $Na_2CO_3$, $Cs_2CO_3$ and NaOH, and DIPEA is preferred.

When A is

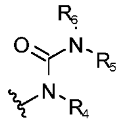

,

$R_4$ and $R_5$ are hydrogen, Compound Formula (I)-IM4 reacts with trimethylsilyl cyanide to obtain Compound Formula (I).

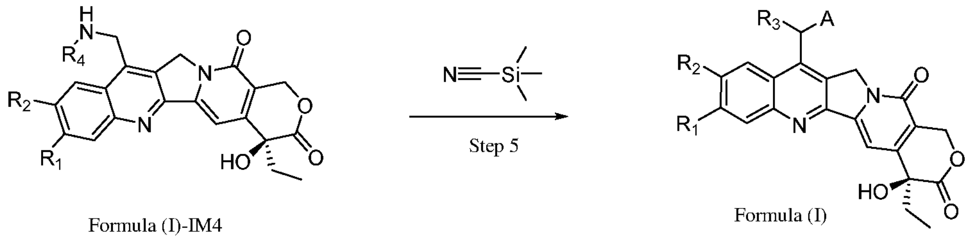


Formula (I)-IM4 Formula (I)

In some embodiments, this step is carried out at a suitable temperature, which is 5°C, 20°C, 25°C, 40°C, 50°C, 60°C, 90°C, 100°C, preferably 90°C;

In some embodiments, this step is carried out in a suitable organic solvent, which may be selected from the group consisting of tetrahydrofuran, dichloromethane, N,N-dimethylformamide, n-methylpyrrolidone, dimethyl sulfoxide and 1,4-dioxane, preferably 1,4-dioxane.

In some embodiments, this step is carried out in the presence of a suitable base, which includes an organic base or an inorganic base, the organic base can be selected from the group consisting of DIPEA, TEA, t-BuOK and Py, the inorganic base can be selected from the group consisting of $K_3PO_4$, NaH, $K_2CO_3$, $Na_2CO_3$, $Cs_2CO_3$ and NaOH, and TEA is preferred.

When A is

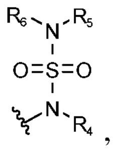

,

$R_5$ and $R_6$ are hydrogen, Compound Formula (I)-IM4 reacts with chlorosulfonyl isocyanate, and then the amino protecting group is removed to obtain Compound Formula (I).

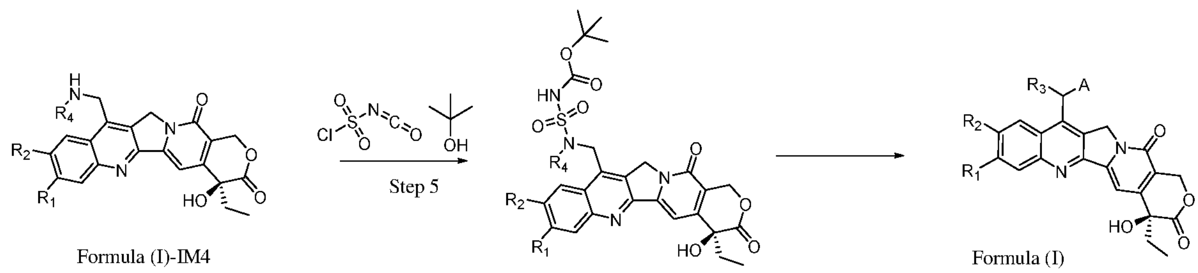


Formula (I)-IM4 Formula (I)

In some embodiments, this step is carried out at a suitable temperature, the temperature is 20°C, 25°C, 40°C, 50°C, 60°C, 100°C, preferably 0-25°C;

In some embodiments, this step is carried out in a suitable organic solvent, which may be selected from the group consisting of methanol, tetrahydrofuran, dichloromethane, *N,N*-dimethylformamide, N-methylpyrrolidone, dimethyl sulfoxide, n-heptane, n-hexane, ethyl acetate, preferably N,N-dimethylformamide.

In some embodiments, this step is carried out in the presence of a suitable base, which includes an organic base or an inorganic base, the organic base can be selected from the group consisting of DIPEA, TEA, t-BuOK and Py, the inorganic base can be selected from the group consisting of $K_3PO_4$, NaH, $K_2CO_3$, $Na_2CO_3$, $Cs_2CO_3$ and NaOH, and TEA is preferred.

When $R_3$ is connected to an adjacent benzene ring carbon atom to form a 6-membered carbocycle, Compound Formula (I) in the present invention can be synthesized through the following synthesis route.

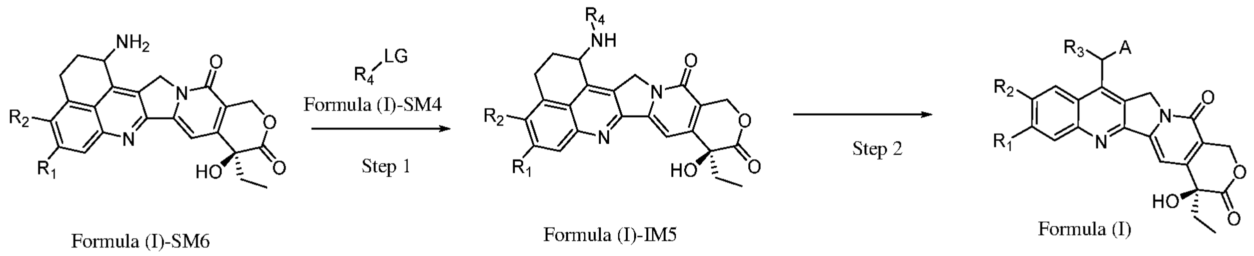


wherein, the meanings of $R_1$, $R_2$, $R_3$, $R_4$ and A are as mentioned above, LG is a leaving group and selected from the group consisting of methanesulfonyl, trifluoromethanesulfonyloxy and halogen, preferably trifluoromethanesulfonyloxy or iodine;

Step 1:

Compound Formula (I)-SM6 and Compound Formula (I)-SM4 undergo substitution reaction to obtain Compound Formula (I)-IM5.

In some embodiments, this step is carried out at a suitable temperature, which is 20°C, 25°C, 40°C, 50°C, 60°C, 100°C, 140°C, preferably 50°C;

In some embodiments, this step is carried out in a suitable organic solvent, which may be selected from the group consisting of halogenated hydrocarbon (e.g., dichloromethane (DCM), chloroform (TCM), 1,2-dichloroethane (1,2-DCE), etc.), nitrile (e.g., acetonitrile (AN), etc.), *N*-methylpyrrolidone (NMP), N,N-dimethylformamide (DMF), N,N-dimethylacetamide (DMA), tetrahydrofuran (THF), 1,4-dioxane (Dioxane), dimethyl sulfoxide (DMSO) and any combination thereof, preferably acetonitrile.

In some embodiments, this step is carried out in the presence of a suitable base, which includes an organic base or an inorganic base. The organic base can be selected from the group consisting of N,N-diisopropylethylamine (DIPEA), triethylamine (TEA), potassium tert-butoxide (t-BuOK) and pyridine (Py), the inorganic base can be selected from the group consisting of potassium phosphate ($K_3PO_4$), sodium hydride (NaH), potassium carbonate ($K_2CO_3$), sodium carbonate ($Na_2CO_3$), sodium bicarbonate ($NaHCO_3$), cesium carbonate ($Cs_2CO_3$) and NaOH, preferably $Na_2CO_3$ or $NaHCO_3$;

Step 2

When A is

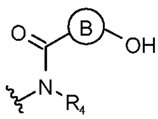

,

Compound Formula (I)-IM5 and Compound Formula (I)-SM5-2 undergo condensation reaction to obtain Compound formula (I). The meanings of $R_5$, $R_6$ and Ring B are as mentioned above.

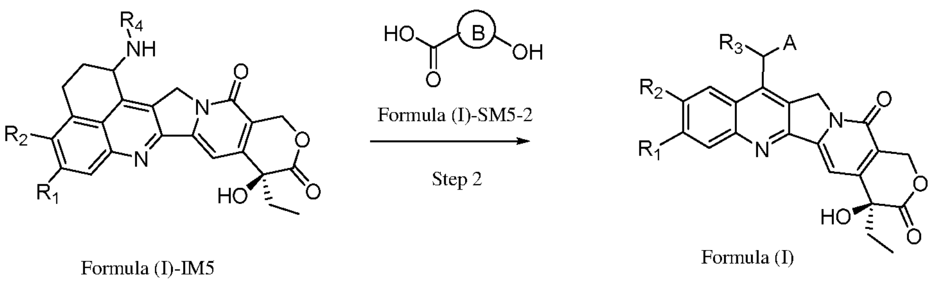


In some embodiments, this step is carried out under a suitable condensation reagent, which can be selected from the group consisting of HATU, HBTU, EDCI, DCC and HOBT, preferably HBTU and HATU;

In some embodiments, this step is carried out at a suitable temperature, which is 20°C, 25°C, 40°C, 50°C, 60°C,

100°C, preferably 25°C;

In some embodiments, this step is carried out in a suitable organic solvent, which may be selected from the group consisting of methanol, tetrahydrofuran, dichloromethane, *N,N*-dimethylformamide, N-methylpyrrolidone, dimethyl sulfoxide, n-heptane, n-hexane, ethyl acetate, preferably N,N-dimethylformamide.

In some embodiments, this step is carried out in the presence of a suitable base, which includes an organic base or an inorganic base, the organic base can be selected from the group consisting of DIPEA, TEA, t-BuOK and Py, the inorganic base can be selected from the group consisting of $K_3PO_4$, NaH, $K_2CO_3$, $Na_2CO_3$, $Cs_2CO_3$ and NaOH, and DIPEA is preferred.

When A is

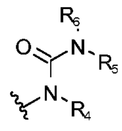

,

$R_4$ and $R_5$ are hydrogen, Compound Formula (I)-IM5 reacts with trimethylsilyl cyanide to obtain Compound Formula (I).

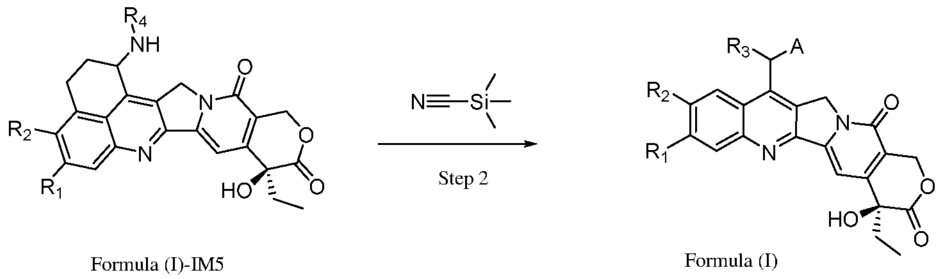


Formula (I)-IM5

Formula (I)

In some embodiments, this step is carried out at a suitable temperature, which is 5°C, 20°C, 25°C, 40°C, 50°C, 60°C, 90°C, 100°C, preferably 90°C;

In some embodiments, this step is carried out in a suitable organic solvent, which may be selected from the group consisting of tetrahydrofuran, dichloromethane, N,N-dimethylformamide, *N*-methylpyrrolidone, dimethyl sulfoxide and 1,4-dioxane, preferably 1,4-dioxane.

In some embodiments, this step is carried out in the presence of a suitable base, which includes an organic base or an inorganic base, the organic base can be selected from the group consisting of DIPEA, TEA, t-BuOK and Py, the inorganic base can be selected from the group consisting of $K_3PO_4$, NaH, $K_2CO_3$, $Na_2CO_3$, $Cs_2CO_3$ and NaOH, and TEA is preferred.

When A is

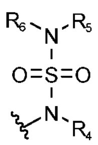

,

$R_5$ and $R_6$ are hydrogen, Compound Formula (I)-IM5 reacts with chlorosulfonyl isocyanate, and then the amino protecting group is removed to obtain Compound Formula (I).

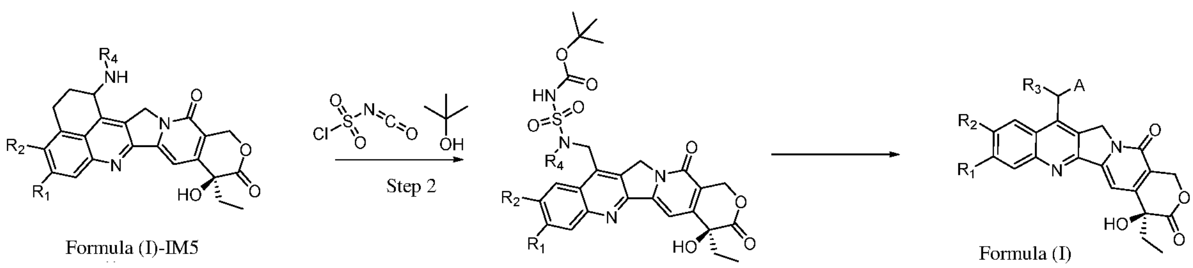


Formula (I)-IM5

Formula (I)

In some embodiments, this step is carried out at a suitable temperature, and the temperature is 20°C, 25°C, 40°C, 50°C, 60°C, 100°C, preferably 0-25°C;
In some embodiments, this step is carried out in a suitable organic solvent, which may be selected from the group consisting of methanol, tetrahydrofuran, dichloromethane, *N,N*-dimethylformamide, N-methylpyrrolidone, dimethyl Sulfoxide, n-heptane, n-hexane, ethyl acetate, preferably N,N-dimethylformamide.
In some embodiments, this step is carried out in the presence of a suitable base, which includes an organic base or an inorganic base, the organic base can be selected from the group consisting of DIPEA, TEA, t-BuOK and Py, the inorganic base can be selected from the group consisting of $K_3PO_4$, NaH, $K_2CO_3$, $Na_2CO_3$, $Cs_2CO_3$ and NaOH, and TEA is preferred.

Beneficial effects of the present invention

The present invention provides camptothecin compounds represented by Formula (I) to Formula (IV), pharmaceutical compositions, preparation methods and uses thereof. These compounds have good anti-tumor activity, have the potential to overcome drug resistance, and can be used to treat abnormal cell proliferation disorders, which include but are not limited to advanced solid tumors.

**Specific Models for Carrying Out the present invention**

The present application will be further described below through the description of specific embodiments, but this is not intended to limit the present application. Those skilled in the art can make various modifications or improvements based on the teachings of the present application without departing from the basic idea and scope of the present application.
The abbreviations in the present invention have the following meanings:

| Abbreviation of reagents | Full name of reagents | Abbreviation of reagents | Full name of reagents |
|---|---|---|---|
| NBS | *N*-Bromosuccinimide | HATU | *N*,*N*,*N'*,*N'*-Tetramethyl-O- (7-aza-benzotriazol-1-yl)urea hexafluoro-phosphate |
| DMF-DMA | *N,N*-dimethylformamide dimethyl acetal | DIPEA | Diisopropylethylamine |
| THF | Tetrahydrofuran | DMSO | Dimethyl sulfoxide |
| DMF | *N,N*-Dimethylformamide | Pd/C | Palladium on carbon |
| LCMS | Liquid chromatography-mass spectrometry | Py | Pyridine |
| FBS | Fetal calf serum | CCK8 | 2-(2-Methoxy-4-niphenyl)-3-(4-niphenyl)-5-(2,4-disulfobenzene)-2*H*-tetrazole monosodium salt |
| DMTMM | 4-(4,6-Dimethoxytriazin-2-yl)-4-methylmorpholine hy-drochloride | EDTA | Ethylenediaminetetraacetic acid so-dium salt |

The structures of the compounds described in the following examples were determined by nuclear magnetic resonance ($^1H$ NMR) or mass spectrometry (MS).
The measurement instrument of nuclear magnetic resonance ($^1H$ NMR) used was a Bruker 400 MHz nuclear magnetic resonance instrument; hexadeuterated dimethyl sulfoxide (DMSO-$d_6$); the internal standard substance was tetramethylsilane (TMS).
The abbreviations in the nuclear magnetic resonance (NMR) spectra used in the examples were shown below.
s: singlet, d: doublet, t: triplet, q: quartet, m: multiplet, br: broad, J: coupling constant, Hz: Hertz, DMSO-$d_6$: deuterated dimethyl sulfoxide. The $\delta$ values were expressed as ppm values.
The measurement instrument of mass spectrometry (MS) used was an Agilent (ESI) mass spectrometer, and its model was Agilent 6120B.

Example 1: Preparation of (*S*)-11-(aminomethyl)-9-chloro-4-ethyl-8-fluoro-4-hydroxy- 1,12-dihydro-14*H*-pyrano [3',4':6,7]indolizino[1,2-b]quinolin-3,14(4*H*)-dione (Compound **1-1)**

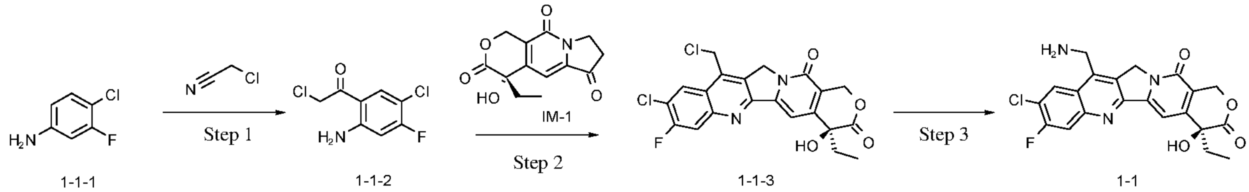


1-1-1 1-1-2 1-1-3 1-1

Step 1: Preparation of 1-(2-amino-5-chloro-4-fluorophenyl)-2-chloroethan-1-one

To an ice-water-cooled solution of boron trichloride (1 M, 36 mL) in 1,2-dichloroethane (40 mL), 4-chloro-3-fluoroaniline (4.36 g, 29.95 mmol) in 1,2-dichloroethane (20 mL) solution was added dropwise, then 2-chloroacetonitrile (2.71 g, 35.94 mmol) and titanium tetrachloride (6.82 g, 35.94 mmol) were added successively at room temperature, and the temperature was raised to reflux to perform reaction for 16 hours. After the reaction solution was cooled to room temperature, 2.5 N hydrochloric acid (55.0 mL) was added and heated to 85 °C again to perform reaction for 30 minutes. The reaction solution was cooled to room temperature and extracted with dichloromethane (3 × 25 mL). The organic phase was washed with saturated brine, and dried over anhydrous sodium sulfate, and the filtrate was filtered and concentrated to obtain the title compound (2 g, 7.21 mmol), which was used directly in the next reaction.

ESI-MS (m/z): 222$[M+H]^+$.

Step 2: Preparation of (*S*)-9-chloro-11-(chloromethyl)-4-ethyl-8-fluoro-4-hydroxy-1,12-dihydro-14*H*-pyrano[3',4':6,7]indolizino[1,2-b]quinolin-3,14(4*H*)-dione

1-(2-Amino-5-chloro-4-fluorophenyl)-2-chloroethan-1-one (**1-1-2**, 0.5 g, 1.80 mmol) was added to toluene (10 mL), then added successively with (S)-4-ethyl-4-hydroxy-7,8-dihydro- 1*H*-pyrano[3,4-f]indolizin-3,6,10(4*H*)-trione (**IM-1**, 500.00 mg, 1.90 mmol) and p-toluenesulfonic acid monohydrate (68.53 mg, 360.29 μmol), the reaction solution was heated to 120°C and reacted for 4 hours. After concentration, the reaction solution was slurried with ethyl acetate, and the solid was collected by filtration to obtain the title compound (0.6 g, 1.34 mmol), which was directly used in the next reaction.

ESI-MS (m/z): 449$[M+H]^+$.

Step 3: Preparation of (S)-11-(aminomethyl)-9-chloro-4-ethyl-8-fluoro-4-hydroxy-1,12-dihydro-14*H*-pyrano[3',4':6,7]indolizino[1,2-b]quinolin-3,14(4*H*)-dione

(*S*)-9-Chloro-11-(chloromethyl)-4-ethyl-8-fluoro-4-hydroxy-1,12-dihydro-14*H*-pyrano[3',4':6,7]indolizino[1,2-b] quinolin-3,14(4*H*)-dione (**1-1-3**, 0.1 g, 222.59 μmol) was dissolved in ethanol (5 mL), and added with urotropine (94 mg, 670.54 μmol). The reaction solution was heated to 85 °C to perform reaction for 5 hours. After the reaction solution was cooled to room temperature, 0.2 mL of concentrated hydrochloric acid was added. After stirring for 10 minutes, it was directly concentrated. The crude product was slurried with ethyl acetate. The solid was collected by filtration and dried to obtain a hydrochloride of the title compound (180 mg, 386.03 μmol). The hydrochloride of **1-1** (20 mg, 46.62 μmol) was added to a mixed solvent of DMF (1 mL) and water (1 mL), adjusted with sodium bicarbonate to reach pH=8, and purified by preparative high performance liquid chromatography (the purification conditions were as follows) to obtain (S)-9-chloro-11-(chloromethyl)-4-ethyl-8-fluoro-4-hydroxy-1,12-dihydro-14*H*-pyrano [3',4':6,7]indolizino[1,2-b]quinolin-3,14(4*H*)-dione (**1-1,** 3 mg).
Chromatography column: SunFire Prep C18 OBD 19 mm×150 mm×5.0μm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 10 | 90 | 30 |
| 2.00 | 10 | 90 | 30 |
| 18.00 | 80 | 20 | 30 |

The structural characterization data were as follows:

$^1$H NMR (400 MHz, DMSO-$d_6$) δ 8.62 (d, *J* = 8.1 Hz, 1H), 8.16 (d, *J* = 10.3 Hz, 1H), 7.33 (s, 1H), 6.56 (s, 1H), 5.45 (d, *J* = 8.6 Hz, 4H), 4.35 (s, 2H), 1.97 - 1.74 (m, 2H), 0.88 (t, *J* = 7.3 Hz, 3H).

ESI-MS (m/z): 430[M+H]$^+$.

Example 2: Preparation of (*S*)-*N*-((9-chloro-4-ethyl-8-fluoro-4-hydroxy-3,14-dioxy-3,4,12,14-tetrahydro-1*H*-pyrano[3',4':6,7]indolizino[1,2-b]quinolin-11-yl)methyl)-2-hydroxyacetamide (Compound **2-1**)

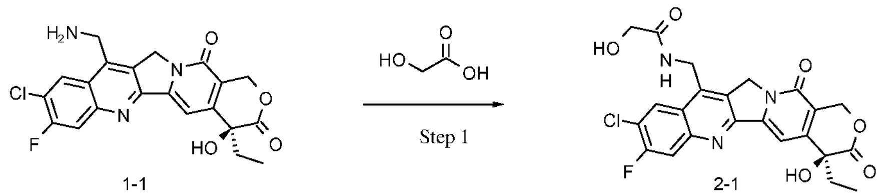


1-1 2-1

(*S*)-11-(Aminomethyl)-9-chloro-4-ethyl-8-fluoro-4-hydroxy-1,12-dihydro-14*H*-pyrano[3',4':6,7]indolizino[1,2-b]quinolin-3,14(4*H*)-dione (**1-1**, 60 mg, 128.68 μmol, hydrochloride) was dissolved in DMF (1 mL), and added successively with glycolic acid (20 mg, 262.98 μmol), triethylamine (26.04 mg, 257.35 μmol) and DMTMM (71.21 mg, 257.35 μmol), and the reaction system was reacted at 20°C for 1 hour. The reaction solution was purified by preparative high performance liquid chromatography to obtain title compound (4 mg, 8.04 μmol).
Chromatography column: SunFire Prep C18 OBD 19 mm×150 mm×5.0μm
Mobile phase A: acetonitrile; mobile phase B: water (0.05% trifluoroacetic acid)

| Time [min] | Mobile phase A [%] | Mobile phase B [%] | Flow rate [mL/min] |
|---|---|---|---|
| 0.00 | 15 | 85 | 28 |
| 3.00 | 15 | 85 | 28 |
| 18.00 | 90 | 10 | 28 |

The structural characterization data were as follows:

$^1$H NMR (400 MHz, DMSO-$d_6$) δ 8.87 (d, *J* = 4.1 Hz, 1H), 8.85 (s, 1H), 8.19 (d, *J* = 10.3 Hz, 1H), 7.34 (s, 1H), 6.55 (s, 1H), 5.55 (s, 2H), 5.45 (s, 2H), 4.82 (d, *J* = 6.0 Hz, 2H), 3.82 (s, 2H), 1.87 (dd, *J* = 9.7, 7.5 Hz, 2H), 0.87 (t, *J* = 7.3 Hz, 3H).

ESI-MS (m/z): 488[M+H]$^+$.

Example 3: Preparation of (1*S*,9*S*)-1-sulfamoylamido-9-ethyl-5-fluoro-9-hydroxy-4-methyl-1,2,3,9,12,15-hexahydro-10*H*,13*H*-benzo[de]pyrano[3',4':6,7]indolizino[1,2-b]quinolin-10,13-dione (Compound **7-1**)

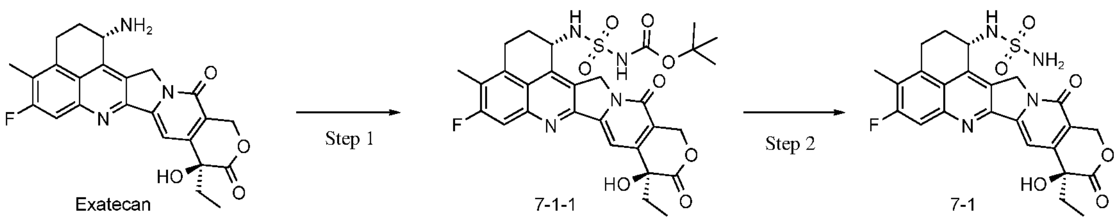


Exatecan 7-1-1 7-1

Step 1: Preparation of tert-butyl (*N*-((1S,9S)-9-ethyl-5-fluoro-9-hydroxy-4-methyl- 10,13-dioxy-2,3,9,10,13,15-hexahydro-1*H*,12*H*-benzo[de]pyrano[3',4':6,7]indolizino[1,2-b]quinolin-1-yl)sulfamoyl)carbamate

Tert-butanol (20.92 mg, 0.282mmol) was dissolved in DCM (0.5 mL), cooled and stirred to 0°C, added dropwise with chlorosulfonyl isocyanate (39.94 mg, 0.282mol, 24.56 μL), kept at the temperature and reacted for 0.5 hours for later use. Exatecan mesylate (100 mg, 0.188mol) was dissolved in DMF (2 mL), added dropwise to TEA (57.11 mg, 0.564mmol, 78.45 μL), cooled and stirred to 0°C, then the above DCM reaction solution was added dropwise to the reaction system, warmed naturally to room temperature and reacted for 1 hour. The reaction was monitored by LCMS and the product was obvious. The reaction solution was directly used in the next reaction.
ESI-MS (m/z): 615.1[M+1]$^+$.

Step 2: Preparation of (1*S*,9*S*)-1-sulfamoylamido-9-ethyl-5-fluoro-9-hydroxy-4-methyl - 1,2,3,9,12,15-hexahydro-10*H*,13*H*-benzo[de]pyrano[3',4':6,7]indolizino[1,2-b]quinoline-10,13-dione

Tert-butyl (*N*-((1*S*,9*S*)-9-ethyl-5-fluoro-9-hydroxy-4-methyl-10,13-dioxy-2,3,9,10,13,15-hexahydro-1*H*,12*H*-benzo[de]pyrano[3',4':6,7]indolizino[1,2-b]quinolin-1-yl)sulfamoyl)carbamate reaction solution was diluted by adding dichloromethane (1 mL), then added dropwise with trifluoroacetic acid (0.5 mL), stirred and reacted for 1 hour. The product was monitored by LCMS. After concentration under reduced pressure, 52.26 mg of the title compound was obtained.
ESI-MS (m/z): 515.2[M+1]$^+$.
$^1$H NMR (400 MHz, DMSO): δ 7.78 (d, *J* = 10.9 Hz, 1H), 7.30 (d, *J* = 9.0 Hz, 2H), 6.98 (s, 2H), 6.53 (s, 1H), 5.55 - 5.36 (m, 4H), 4.92 (d, *J* = 4.2 Hz, 1H), 3.27 (dd, *J* = 19.1, 6.9 Hz, 1H), 3.14 - 3.04 (m, 1H), 2.38 (s, 3H), 2.36 - 2.22 (m, 2H), 1.93 - 1.79 (m, 2H), 0.87 (t, *J* = 7.3 Hz, 3H).
Chromatography column: SunFire Prep C18 OBD 19 mm×150 mm×5.0μm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 10 | 90 | 28 |
| 2.00 | 10 | 90 | 28 |
| 18.00 | 90 | 10 | 28 |

Example 4: Preparation of 1-((1*S*,9*S*)-9-ethyl-5-fluoro-9-hydroxy-4-methyl-10,13-dioxy-2,3,9,10,13,15-hexahydro-1*H*,12*H*-benzo[de]pyrano[3',4':6,7]indolizino[1,2-b]quinolin-1-yl)urea (Compound **7-2**)

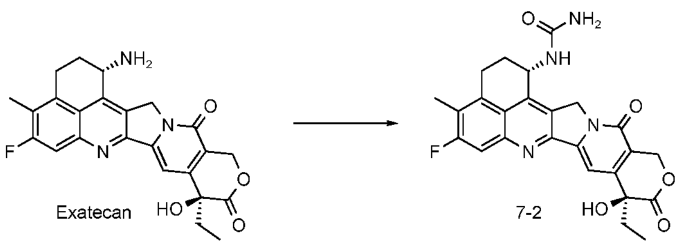
Exatecan
7-2

Exatecan mesylate (50 mg, 0.082mmol) was dissolved in 1,4-dioxane (1 mL), added dropwise with triethylamine (16.65 mg, 0.165mmol, 22.87 μL), and added dropwise with trimethylsilyl cyanide (6.53 mg, 0.165mmol, 8.24 μL) under stirring, heated to 90°C and reacted for 2 hours. The reaction was monitored by LCMS until the starting material disappeared. The reaction solution was cooled to room temperature, added dropwise with MeOH (1 mL), stirred for 10 minutes, concentrated under reduced pressure and purified to obtain 12.92 mg of the title compound.
ESI-MS (m/z): 479.1[M+1]$^+$.
$^1$H NMR (400 MHz, DMSO): δ 7.78 (d, *J* = 10.9 Hz, 1H), 7.30 (s, 1H), 6.74 (d, *J* = 9.1 Hz, 1H), 6.53 (s, 1H), 5.69 (s, 2H), 5.42 (s, 2H), 5.29 (q, *J* = 19.2 Hz, 3H), 3.15 (s, 2H), 2.39 (s, 3H), 2.20 - 2.07 (m, 2H), 1.86 (dd, *J* = 10.4, 7.4 Hz, 2H), 0.87 (t, *J* = 7.3 Hz, 3H).

Chromatography column: SunFire Prep C18 OBD 19 mm×150 mm×5.0μm

Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 10 | 90 | 28 |
| 2.00 | 10 | 90 | 28 |
| 18.00 | 90 | 10 | 28 |

Example 5: Preparation of *N*-((*S*)-9-chloro-4-ethyl-8-fluoro-4-hydroxy-3,14-dioxo-3,4,12,14-tetrahydro-1*H*-pyrano [3',4':6,7]indolizino[1,2-b]quinolin-11-yl)methyl)-2-cyclopropyl-2-hydroxyacetamide (Compound **2-4**)

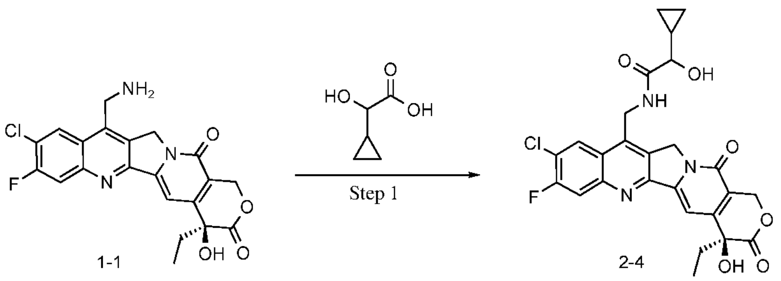


(*S*)-11-(Aminomethyl)-9-chloro-4-ethyl-8-fluoro-4-hydroxy-1,12-dihydro-14*H*-pyrano[3',4':6,7]indolizino[1,2-b] quinolin-3,14(4*H*)-dione (**1-1**, 30 mg, 69.8 μmol) was dissolved in DMF (1 mL), and added successively with 2-cyclopropyl-2-hydroxyacetic acid (16.21 mg, 139.6 μmol), DIPEA (22.55 mg, 174.5 μmol) and HATU (31.83 mg, 83.75 μmol). The reaction system was reacted at 20°C for 1 hour. The reaction solution was directly purified by high performance chromatography to obtain 11 mg of the title compound.

Chromatography column: SunFire Prep C18 OBD 19 mm×150 mm×5.0μm

Mobile phase A: acetonitrile; mobile phase B: water (0.05% trifluoroacetic acid)

| Time [min] | Mobile phase A [%] | Mobile phase B [%] | Flow rate [mL/min] |
|---|---|---|---|
| 0.00 | 15 | 85 | 28 |
| 3.00 | 15 | 85 | 28 |
| 18.00 | 90 | 10 | 28 |

The structural characterization data were as follows:

$^1$H NMR (400 MHz, DMSO-$d_6$) δ 8.81 (d, *J* = 8 Hz, 1H), 8.75 (m, 1H), 8.19 (d, *J* = 8 Hz, 1H), 7.34 (s, 1H), 6.55 (s, 2H), 5.53 (s, 2H), 5.45 (s, 2H), 4.82 (m, 2H), 3.57 (m, 1H), 1.86 (m, 2H), 0.97 (m, 1H), 0.87 (t, *J* = 8 Hz, 3H), 0.32 (m, 2H), 0.23 (m, 2H).

ESI-MS (m/z): 529 [M+H]$^+$.

Example 6: Preparation of *N*-(((S)-9-chloro-4-ethyl-8-fluoro-4-hydroxy-3,14-dioxo-3,4, 12,14-tetrahydro-1*H*-pyrano [3',4':6,7]indolizino[1,2-b]quinolin-11-yl)methyl)-2-hydroxy-3-enamine (Compound **2-22**)

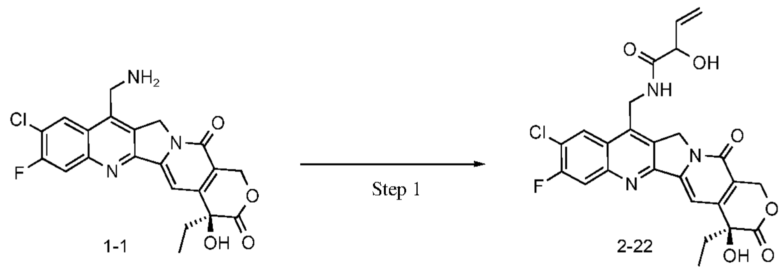


1-1 2-22

(*S*)-11-(Aminomethyl)-9-chloro-4-ethyl-8-fluoro-4-hydroxy-1,12-dihydro-14*H*-pyrano[3',4':6,7]indolizino[1,2-b]quinolin-3,14(4*H*)-dione (**1-1**, 30 mg, 73.28 μmol) was dissolved in DMF (1 mL), and added successively with 2-hydroxy-3-enoic acid (7.48 mg, 73.28 μmol), DIPEA (23.68 mg, 183.19 μmol) and HATU (31.42 mg, 87.93 μmol), the reaction system was reacted at 20°C for 1 hour, and the reaction solution was directly purified by preparative high performance liquid chromatography to obtain 5.36 mg of the title compound.

Chromatography column: SunFire Prep C18 OBD 19 mm×150 mm×5.0μm

Mobile phase A: acetonitrile; mobile phase B: water (0.05% trifluoroacetic acid)

| Time [min] | Mobile phase A [%] | Mobile phase B [%] | Flow rate [mL/min] |
|---|---|---|---|
| 0.00 | 15 | 85 | 28 |
| 3.00 | 15 | 85 | 28 |
| 18.00 | 90 | 10 | 28 |

The structural characterization data were as follows:

$^1$H NMR (400 MHz, DMSO-$d_6$) δ 8.58 (m, 1H), 7.97 (m, 1H), 7.63 (m, 1H), 5.59 (m, 1H), 5.51 (s, 1H), 5.40 (m, 2H), 5.18 (d, *J* = 12 Hz, 1H), 4.95 (s, 2H), 4.61 (s, 2H), 4.58 (m, 1H), 1.95 (m, 2H), 0.98 (t, *J* = 8 Hz, 3H).

ESI-MS (m/z): 514 $[M+H]^+$.

Example 7: Preparation of N-(((S)-9-chloro-4-ethyl-8-fluoro-4-hydroxy-3,14-dioxo-3,4, 12,14-tetrahydro-1H-pyrano [3',4':6,7]indolizino[1,2-b]quinolin-11-yl)methyl)-3,3,3-trifluoro-2-hydroxypropionamide (Compound **2-19**)

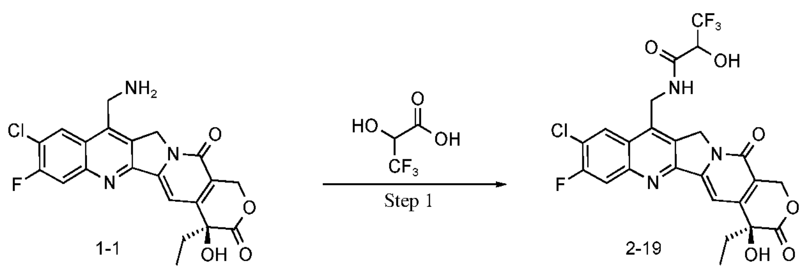


1-1 2-19

(*S*)-11-(Aminomethyl)-9-chloro-4-ethyl-8-fluoro-4-hydroxy-1,12-dihydro-14*H*-pyrano[3',4':6,7]indolizino[1,2-b]quinolin-3,14(4*H*)-dione (**1-1**, 22 mg, 51.18 μmol) was dissolved in DMF (1 mL), and added successively with 3,3,3-trifluoro-2-hydroxypropionic acid (8.85 mg, 61.42 μmol), DIPEA (19.84 mg, 153.55 μmol) and HATU (23.34 mg, 61.42 μmol), the reaction system was reacted at 20°C for 1 hour, and the reaction solution was directly purified by preparative high performance liquid chromatography to obtain 2 mg of the title compound.

Chromatography column: SunFire Prep C18 OBD 19 mm×150 mm×5.0μm

Mobile phase A: acetonitrile; mobile phase B: water (0.05% trifluoroacetic acid)

| Time [min] | Mobile phase A [%] | Mobile phase B [%] | Flow rate [mL/min] |
|---|---|---|---|
| 0.00 | 15 | 85 | 28 |
| 3.00 | 15 | 85 | 28 |
| 18.00 | 90 | 10 | 28 |

The structural characterization data were as follows:

$^{1}$H NMR (400 MHz, DMSO-$d_6$) δ 8.78 (d, *J* = 8 Hz, 1H), 8.19 (d, *J* = 8 Hz 1H), 7.34 (s, 1H), 6.55 (s, 1H), 5.91 (m, 1H), 5.85 (m, 1H), 5.54 (s, 2H), 5.45 (s, 2H), 4.86 (m, 2H), 4.58 (m, 1H), 1.86 (m, 2H), 0.87 (t, *J* = 8 Hz, 3H).

ESI-MS (m/z): 556 $[M+H]^+$.

Example 8: Preparation of (2*R*,3*R*,4R,5*S*,6*R*)-6-(4-(((((S)-9-chloro-4-ethyl-8-fluoro-4-hydroxy-3,4,12,14-tetrahydro-1*H*-pyrano[3',4':6,7]indolo[1,2-b]quinolin-11-yl)methyl)carbamoyl)oxy)methyl)-2-(2-(2-(*N*-(6-(2-(methanesulfonyl) pyrimidin-5-yl)hex-5-ynamido)ethylamino)ethoxy)acetamido)phenoxy)-3,4,5-trihydroxy-tetrahydro-2*H*-pyran-2-carboxylic acid (**A-11**)

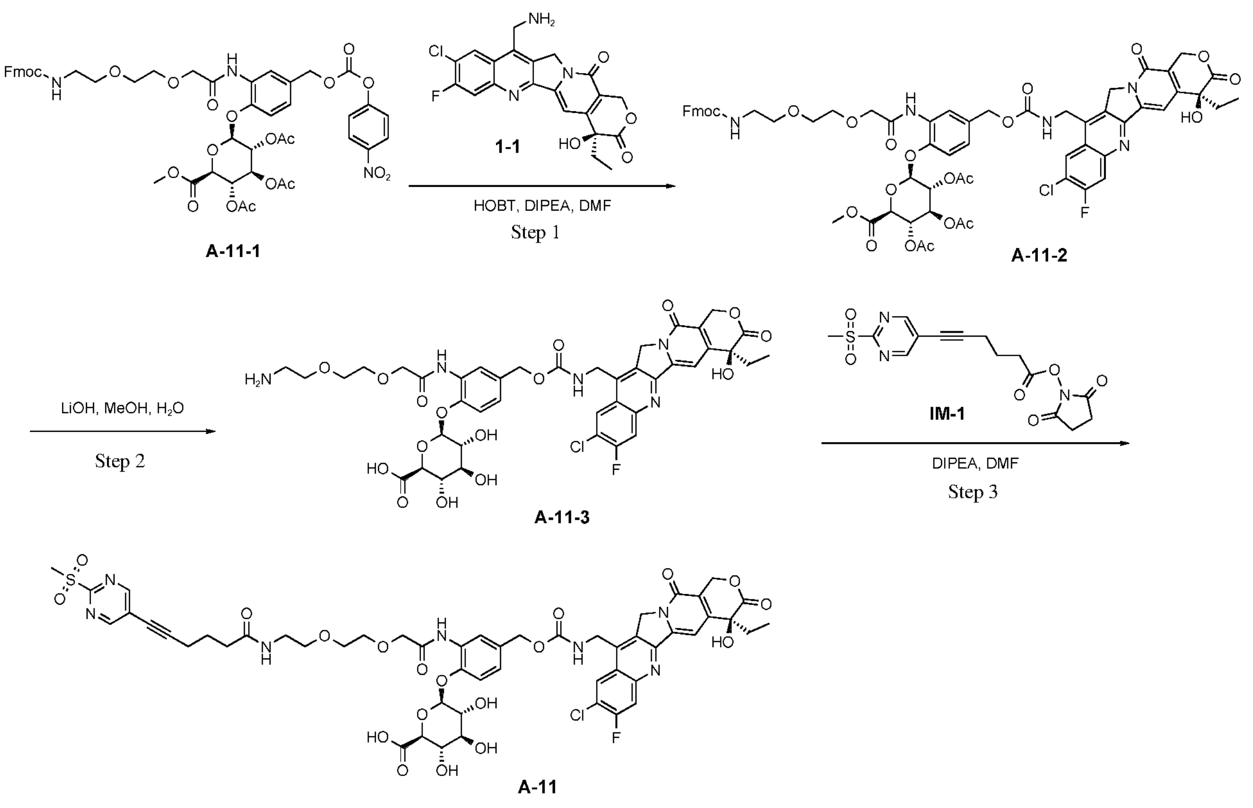


Step 1: Preparation of (2*R*,3*S*,4*R*,5*R*,6*R*)-2-(2-(8-(9-fluorenylmethoxycarbonylamino)-3,6-dioxa-octanoamido)-4-((((((*S*)-9-chloro-4-ethyl-8-fluoro-4-hydroxy-3,14-dioxo-3,4,12,14-tetrahydro-1*H*-pyrano[3',4':6,7]indolizino[1,2b] quinolin-11-yl)methyl)carbamoyl)oxy)methyl)phenoxy)-6-(methoxycarbonyl)-tetrahydro-2*H*-pyran-3,4,5-triacetate (**A-11-2**)

Compound **1-1** (30 mg, 69.80 μmol) and Compound **A-11-1** (75.85 mg, 76.77 μmol, prepared with reference to page 81 of WO2022253035) were dissolved in *N,N*-dimethylformamide (1 mL), then added with HOBT (11.32 mg, 83.75 μmol) and *N,N*-diisopropylethylamine (22.55 mg, 174.49 μmol), and stirred at room temperature for 1 hour. After the reaction was completed, the reaction solution was spin-dried to obtain crude product **A-11-2,** which was directly used in the next reaction.

Step 2: Preparation of (2*R*,3*R*,4*R*,5*S*,6*R*)-6-(2-(2-(2-(2-aminoethyl)ethoxy)acetamido)- 4-((((((*S*)-9-chloro-4-ethyl-8-fluoro-4-hydroxy-3,14-dioxo-3,4,12,14-tetrahydro-1*H*-pyrano[3',4':6,7]indolizino[1,2-b]quinolin-11yl)methyl)carbamoyl) oxy)methyl)phenoxy)-3,4,5-trihydroxytetrahydro-2*H*-pyran-2-carboxylic acid **(A-11-3)**

**A-11-2** was dissolved in MeOH (1mL) and water (0.5mL), added with LiOH·$H_2O$ (26.28 mg, 625.67 μmol), and reacted at room temperature for 4 hours. After the reaction was completed, the reaction solution was directly purified by preparative high-performance liquid chromatography and freeze-dried to obtain the title compound **A-11-3** (20 mg).

Its purification method was as follows:

Chromatography column: Waters SunFire Prep C18 OBD (5 μm*19 mm*150 mm)

Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 20 | 80 | 28 |
| 2.00 | 20 | 80 | 28 |
| 18.00 | 80 | 20 | 28 |

Step 3: Preparation of (2*R*,3*R*,4*R*,5*S*,6*R*)-6-(4-(((((*S*)-9-chloro-4-ethyl-8-fluoro-4-hydroxy-3,4,12,14-tetrahydro-1*H*-pyrano[3',4':6,7]indolo[1,2-b]quinolin-11-yl)methyl)carbamoyl)oxy)methyl)-2-(2-(2-(N-(6-(2-(methanesulfonyl)pyrimidin-5-yl)hex-5-ynamido)ethylamino)ethoxy)acetamido)phenoxy)-3,4,5-trihydroxy-tetrahydro-2*H*-pyran-2-carboxylic acid **(A-11)**

**A-11-3** (20 mg, 21.83 μmol), **IM-1** (7.98 mg, 21.83 μmol) and *N,N*-diisopropylethylamine (8.46 mg, 65.49 μmol) were dissolved in DMF (1mL), and the reaction solution was stirred at room temperature for 1 hour. After the reaction was completed, the reaction solution was directly purified by preparative high-performance liquid chromatography and freeze-dried to obtain 3.42 mg of the title compound.

Its structural characterization data were as follows:

MS m/z (ESI): 1168.2 $[M+H]^+$.

Its purification method was as follows:

Chromatography column: Waters SunFire Prep C18 OBD (5 μm*19 mm*150 mm)

Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 30 | 70 | 28 |
| 2.00 | 30 | 70 | 28 |
| 18.00 | 90 | 10 | 28 |

Example 9: Preparation of *N*-((*S*)-12-benzyl-1-((*S*)-9-chloro-4-ethyl-8-fluoro-4-hydroxy-3,14-dioxo-3,4,12,14-tetrahydro-1*H*-pyrano[3',4':6,7]indolizino[1,2-b]quinolin-11-yl)-3,8,11,14, 17-pentaoxo-5-oxa-2,7,10,13-tetraazapentadecan-15-yl)-6-(2-(methanesulfonyl)pyrimidin-5-yl)hex-5-ynamide **(B-1)**

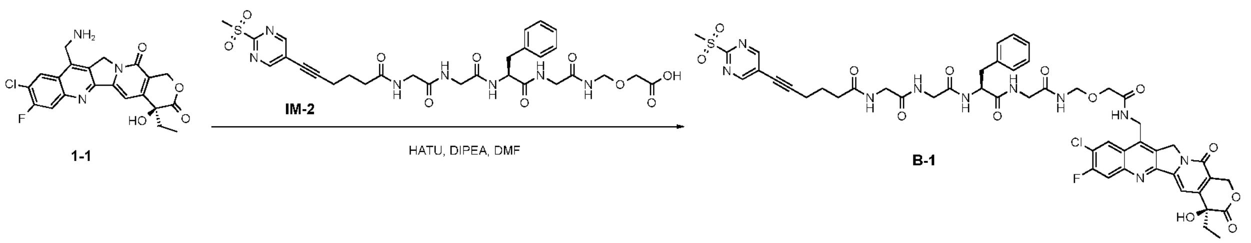


1-1

B-1

Compound **1-1** (30 mg, 69.80 μmol) and Compound **IM-2** (51.72 mg, 76.77 μmol, prepared with reference to page 92 of WO2022253035) were dissolved in *N,N*-dimethylformamide (1 mL), and then added with HATU (29.17 mg, 76.77 μmol) and *N,N*-diisopropylethylamine (22.55 mg, 174.49 μmol), and stirred at room temperature for 1 hour. After the

reaction was completed, the reaction solution was directly purified by preparative high performance liquid chromatography and freeze-dried to obtain 10 mg of the title compound.

Its structural characterization data were as follows:

MS m/z (ESI): 1087.3 $[M+H]^+$.

Its purification method was as follows:

Chromatography column: Waters SunFire Prep C18 OBD (5 μm*19 mm*150 mm)

Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 20 | 80 | 28 |
| 2.00 | 20 | 80 | 28 |
| 18.00 | 80 | 20 | 28 |

Example 10: Preparation of *N*-((9S,12S,15S)-1-((*S*)-9-chloro-4-ethyl-8-fluoro-4-hydroxy-3,14-dioxo-3,4,12,14-tetrahydro-1-pyrano[3',4':6,7]indolizino[1,2-b]quinolin-11-yl)-9,12-dimethyl-3,8,11,14-tetraoxo-5-oxa-2,7,10,13-tetraazahexadecan-15-yl)-6-(2-(methanesulfonyl)pyrimidin-5-yl)hex-5-ynamide **(B-2)**

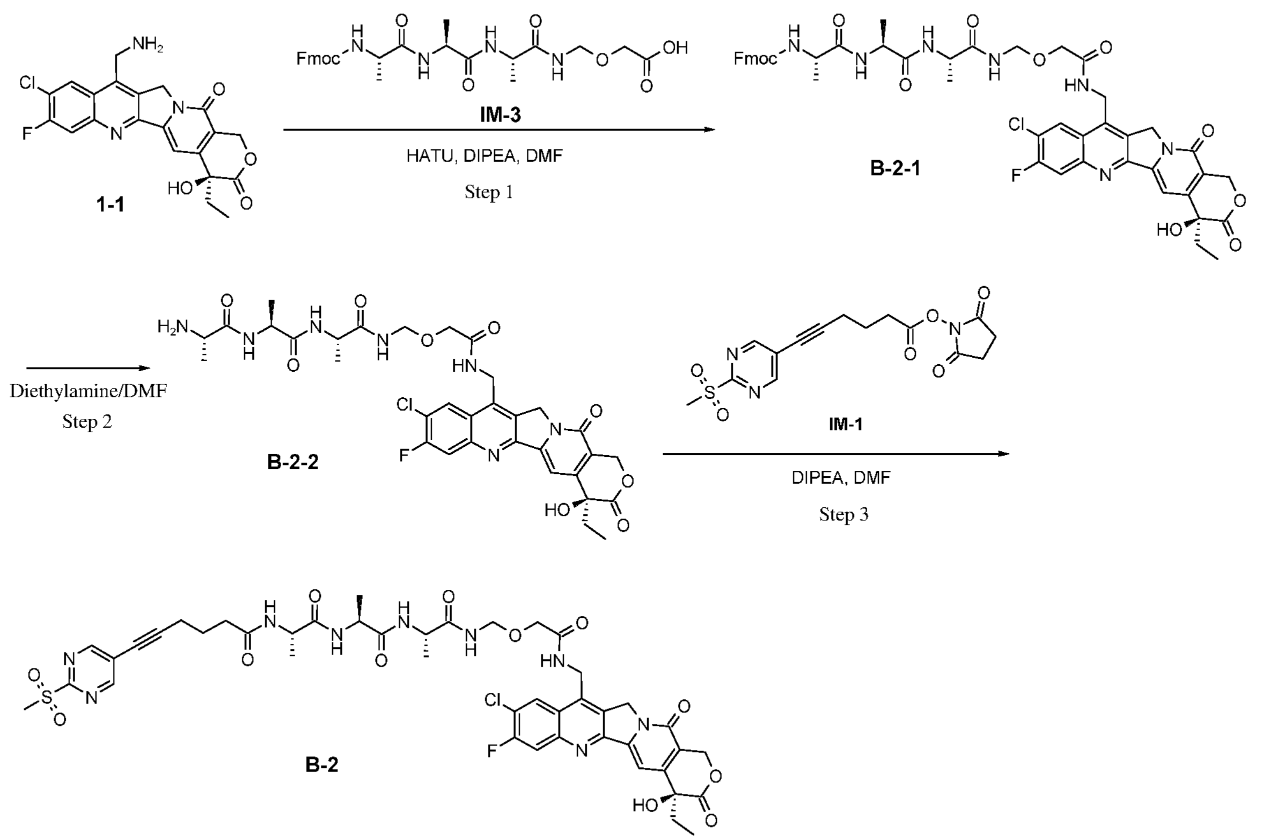


Step 1: Preparation of (9*H*-fluoren-9-yl)methyl((9*S*,12*S*,15*S*)-1-((*S*)-9-chloro-4-ethyl-8-fluoro-4-hydroxy-3,14-dioxo-3,4,12,14-tetrahydro-1*H*-pyrano[3',4':6,7]indolizino[1,2-b]quinolin-11-yl)-9,12-dimethyl-3,8,11,14-tetraoxo-5-oxa-2,7,10,13-tetraazahexadecan-15-yl)carbamate **(B-2-1)**

Compound **1-1** (50 mg, 116.33 μmol) and Compound **IM-3** (66.03 mg, 122.14 μmol) were dissolved in *N,N*-dimethylformamide (1 mL), then added with HATU (48.62 mg, 127.96 μmol) and *N,N*-diisopropylethylamine (37.58 mg, 290.81 μmol), and stirred at room temperature for 1 hour. After the reaction was completed, the reaction solution was

concentrated and the crude product was purified by high-performance liquid chromatography and freeze-dried to obtain 100 mg of the title compound.

Its purification method was as follows:

Chromatography column: Waters SunFire Prep C18 OBD (5 μm*19 mm*150 mm)

Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 20 | 80 | 28 |
| 2.00 | 20 | 80 | 28 |
| 18.00 | 80 | 20 | 28 |

Step 2: Preparation of (*S*)-2-amino-*N*-((9*S*,12*S*)-1-((S)-9-chloro-4-ethyl-8-fluoro-4-hydroxy-3,14-dioxy-3,4,12,14-tetrahydro-1*H*-pyrano[3',4':6,7]indolizino[1,2-b]quinolin-11-yl)-9-methyl-3,8,11-trioxo-5-oxa-2,7,10-triazatridecan-12-yl)propionamide **(B-2-2)**

Compound **B-2-1** (100 mg, 105.00 μmol) was dissolved in *N,N*-dimethylformamide (1 mL), added with diethylamine (0.5 mL), and reacted at room temperature for 1 hour. After the reaction was completed, the reaction solution was concentrated under reduced pressure to obtain a crude product, which was directly used in the reaction of next step.

Step 3: Preparation of *N*-((9*S*,12*S*,15*S*)-1-((*S*)-9-chloro-4-ethyl-8-fluoro-4-hydroxy-3,14-dioxo-3,4,12,14-tetrahydro-1-pyrano[3',4':6,7]indolizino[1,2-b]quinolin-11-yl)-9,12-dimethyl-3,8,11,14-tetraoxo-5-oxa-2,7,10,13-tetraazahexadecan-15-yl)-6-(2-(methanesulfonyl)pyrimidin-5 -yl)hex-5-ynamide **(B-2)**

Compound **IM-1** (17.66 mg, 48.39 μmol) and *N,N*-diisopropylethylamine (12.48 mg, 0.226 mmol) were dissolved in *N,N*-dimethylformamide (1 mL) that contained the crude product of **B-2-2,** the reaction system was stirred at room temperature for 1 hour. After the reaction was completed, the reaction solution was concentrated under reduced pressure to obtain a crude product, which was purified by high-performance liquid chromatography and then freeze-dried to obtain 5 mg of the title compound.

Its structural characterization data were as follows:

MS m/z (ESI): 981.7 $[M+H]^+$.

Its purification method was as follows:

Chromatography column: Waters SunFire Prep C18 OBD (5 μm*19 mm*150 mm)

Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 20 | 80 | 28 |
| 2.00 | 20 | 80 | 28 |
| 18.00 | 80 | 20 | 28 |

Example 11: Preparation of (*S*)-*N*-((1*S*,9*S*)-4-chloro-9-ethyl-5-fluoro-9-hydroxy-10,13-dioxo-2,3,9,10,13,15-hexahydro-1*H*,12*H*-benzo[d]pyrano[3',4':6,7]indolizino[1,2-b]quinolin-1-yl)-3-hydroxybutyamide **(2-25)**

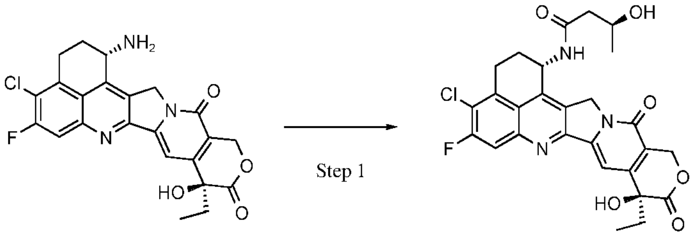


2-25-1 2-25

(1*S*,9*S*)-1-Amino-4-chloro-9-ethyl-5-fluoro-9-hydroxy-1,2,3,9,12,15-hexahydro-10*H*,13*H*-benzo[d]pyrano [3',4':6,7]indolizino[1,2-b]quinolin-10,13-dione (prepared with reference to WO2022166762A1) (30 mg, 0.053 mmol), (*S*)-3-hydroxybutyric acid (16.5 mg, 0.158 mmol), HATU (40.0 mg, 0.105 mmol) and DIPEA (20.4 mg, 0.158 mmol) were added into a DMF (0.5 mL) reaction system, stirred and reacted at 25°C for 2 hours, and the reaction solution was directly prepared by high-performance liquid chromatography and then freeze-dried to obtain the title compound (13.5 mg, 0.024 mmol).

Its separation and purification method were as follows:
Chromatography column: Waters XBridge Prep C18OBD (5 μm*19mm*150 mm)
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 30 | 70 | 28 |
| 2.00 | 30 | 70 | 28 |
| 18.00 | 90 | 10 | 28 |

Its structural characterization data were as follows:

ESI-MS (m/z): 542.1 $[M+H]^+$

$^1H$ NMR (400 MHz, DMSO-$d_6$) δ 8.44 (d, *J* = 8.8 Hz, 1H), 8.09 (d, *J* = 10.4 Hz, 1H), 7.34 (s, 1H), 6.56 (m, 1H), 5.64 - 5.60 (m, 1H), 5.44 (s, 2H), 5.32 - 5.25 (m,2H), 4.63 (d, *J* = 4.8 Hz, 1H), 4.06 - 4.01 (m, 1H), 3.31 - 3.26 (m, 2H), 2.28 - 2.23 (m, 1H), 2.20 - 2.13 (m, 3H), 1.90 - 1.82 (m, 2H), 1.08 (d, *J* = 6.4 Hz, 3H), 0.87 (t, *J* = 7.2 Hz, 3H).

Example 12: Preparation of (*R*)-*N*-((1*S*,9*S*)-4-chloro-9-ethyl-5-fluoro-9-hydroxy-10,13-dioxo-2,3,9,10,13,15-hexahydro-1*H*,12*H*-benzo[d]pyrano[3',4':6,7]indolizino[1,2-b]quinolin-1-yl)-3-hydroxybutanamide **(2-26)**

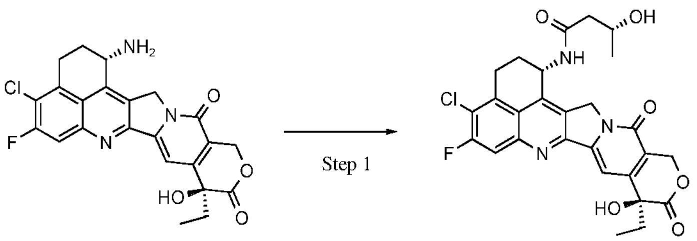


2-25-1 2-26

(1*S*,9*S*)-1-Amino-4-chloro-9-ethyl-5-fluoro-9-hydroxy-1,2,3,9,12,15-hexahydro-10*H*,13*H*-benzo[d]pyrano [3',4':6,7]indolizino[1,2-b]quinolin-10,13-dione (prepared with reference to WO2022166762A1) (30 mg, 52.64 μmol), (*R*)-3-hydroxybutyric acid (10.96 mg, 105.28 μmol), HATU (30.02 mg, 78.96 μmol) and DIPEA (20.41 mg, 157.93 μmol) were added to a DMF (2 mL) reaction system, and the reaction was stirred at 25°C for 2 hours, the reaction solution was directly purified by high-performance liquid chromatography and then freeze-dried to obtain the title compound (17 mg, 31.42 μmol).

Its separation and purification method were as follows:
Column: Waters XBridge Prep C18OBD (5 μm*19mm*150 mm)

Mobile phase A: acetonitrile; mobile phase B: water (0.05% formic acid)

| Time [min] | Mobile phase A [%] | Mobile phase B [%] | Flow rate [mL/min] |
|---|---|---|---|
| 0.00 | 20 | 80 | 30 |
| 2.00 | 20 | 80 | 30 |
| 18.00 | 85 | 15 | 30 |

Its structural characterization data were as follows:

ESI-MS (m/z): 542.1 $[M+H]^+$

$^1$H NMR (400 MHz, DMSO) δ 8.43 (d, *J* = 8.8 Hz, 1H), 8.06 (d, *J* = 10.4 Hz, 1H), 7.33 (s, 1H), 6.54 (s, 1H), 5.63-5.55 (m, 1H), 5.43 (s, 2H), 5.27 (d, *J* = 18.8 Hz, 1H), 5.21 (d, *J* = 18.8 Hz, 1H), 4.66 (d, *J* =4.8 Hz, 1H), 4.09 -3.98 (m, 1H), 3.32-3.26 (m, 2H), 2.32-2.25 (m,1H), 2.24-2.12 (m, 3H), 1.93-1.78 (m, 2H), 1.08 (d, *J* = 6.0 Hz, 3H), 0.87 (t, *J* = 7.2 Hz, 3H).

Preparation examples of antibody-drug conjugates

Preparation of **Trastuzumab-A-11** (DAR 8)

1.781mL of Trastuzumab antibody (16mg/mL) was taken, diluted with 89μL of 20mM phosphate buffer + 0.1M EDTA (pH 7.60), then adjusted to pH 7.60 with 1M $Na_2HPO_4$ solution, added with 10mM TCEP (tris(2-carboxyethyl) phosphine, 108 μL, pH 7.60) solution and mixed well, and allowed to stand at room temperature for 1.5 h. Then **A-11** in 10 times amount of substance (218 μL, 10 mM) dissolved in dimethyl sulfoxide solution was slowly added, mixed well, and allowed to stand at room temperature for 2 hours. After that, a NAP-5 gel column (Cytiva) was used to replace the buffer solution with 20 mM pH 6.0 histidine buffer solution, thereby obtaining the antibody-drug conjugate **Trastuzumab-A-11.** Its DAR value was 8.0 as measured by mass spectrometry.

Preparation of **Trastuzumab-B-1** (DAR 8)

1.973mL of Trastuzumab antibody (15.2mg/mL) was taken, diluted with 98.7μL of 20mM phosphate buffer + 0.1M EDTA (pH 7.60), then adjusted to pH 7.60 with 1M $Na_2HPO_4$ solution, added with 10mM TCEP (tris(2-carboxyethyl) phosphine, 56.92 μL, pH 7.60) solution and mixed well, and allowed to stand at room temperature for 1.5 h. Then **B-1** in 12 times amount of substance (258.16 μL, 10 mM) dissolved in dimethyl sulfoxide solution was slowly added, mixed well, and allowed to stand at room temperature for 2 hours. After that, a NAP-5 gel column (Cytiva) was used to replace the buffer solution with 20 mM pH 6.0 histidine buffer solution, thereby obtaining the antibody-drug conjugate **Trastuzumab-B-1.** Its DAR value was 7.96 as determined by mass spectrometry.

Preparation of **Trastuzumab-B-2** (DAR 8)

2.16mL of Trastuzumab antibody (16.2mg/mL) was taken, diluted with 108μL of 20mM phosphate buffer + 0.1M EDTA (pH 7.60), then adjusted to pH 7.60 with 1M $Na_2HPO_4$ solution, added with 10mM TCEP (tris(2-carboxyethyl) phosphine, 88.42 μL, pH 7.60) solution and mixed well, and allowed to stand at room temperature for 1.5 h. Then **B-2** in 20 times amount of substance (502.38 μL, 10 mM) dissolved in dimethyl sulfoxide solution was slowly added, mixed well, and allowed to stand at room temperature for 2 hours. After that, a NAP-5 gel column (Cytiva) was used to replace the buffer solution with 20 mM pH 6.0 histidine buffer solution, thereby obtaining the antibody-drug conjugate **Trastuzumab-B-2.** Its DAR value was 7.95 as determined by mass spectrometry.

Biological evaluation

I. Tumor cell proliferation inhibition test

1. Inhibitory effect of compounds on HT29 cell proliferation

(1) Cell plating: First, tumor cells HT29 were cultured with corresponding culture medium, the cells were digested with trypsin and centrifuged, then the cells were resuspended and counted, and the cells were adjusted to an appropriate concentration for plating. The sources of tumor cells were shown in Table 1.

Table 1. Source of tumor cells

| Cell name | Tumor type | Source |
|---|---|---|
| HT29 | Human colon cancer cells | Cell Bank of Chinese Academy of Sciences |

Co-incubation of the compounds of the present invention and tumor cells: 90 μL of cell suspension was added to 96-well plates respectively. After cell adherence, 10 μL of bioactive molecules (compounds of the present invention) diluted with culture medium were added into the wells of the above plates, and incubated for 72 h.

In vitro cell viability detection: After the incubation, 50 μL of Cell Counting-Lite™ 2.0 reagent (Vazyme) was added to each well, shaken and mixed well in the dark, and reacted for 10 minutes before testing. Microplate reader (manufacturer: BMG, Model: PHERA Star-FS) was used for reading. The culture wells without cells were used as the background RLU, and the culture wells with cells but no compound were used as the cell control RLU. Cell inhibition rate = 1 - (sample RLU - background RLU) / (cell control RLU - background RLU) × 100%. The halfinhibition concentration ($IC_{50}$) of compound was calculated by fitting the curve according to the four-parameter model. The test results were shown in Table 2.

(2) Data results

Table 2. Inhibitory activity on HT29 cell proliferation

| Name | $IC_{50}$ (nM) |
|---|---|
| Example 1 (Compound **1-1**) | 1.07 |
| Example 2 (Compound **2-1**) | 14.58 |
| Example 3 (Compound **7-1**) | 10.12 |
| Example 4 (Compound **7-2**) | 22.80 |
| Example 5 (Compound **2-4**) | 5.99 |
| Example 6 (Compound **2-22**) | 10.16 |
| Example 7 (Compound **2-19**) | 2.93 |

The test results showed that the compounds of the present invention in Table 2 had strong inhibitory effects on the proliferation of HT29 colon cancer cells.

2. Inhibitory effect of compounds on HCC1806 cell proliferation

(1) Cell plating: First, tumor cells HCC1806 were cultured with corresponding culture medium, the cells were digested with trypsin and centrifuged, then the cells were resuspended and counted, and the cells were adjusted to an appropriate concentration for plating. The sources of tumor cells were shown in Table 3.

Table 3. Source of tumor cells

| Cell name | Tumor type | Source |
|---|---|---|
| HCC1806 | Human breast squamous cell carcinoma | ATCC |

Co-incubation of the compounds of the present invention and tumor cells: 90 μL of cell suspension was added to 96-well plates respectively. After cell adherence, 10 μL of bioactive molecules (compounds of the present invention) diluted with culture medium were added into the wells of the above plates, and incubated for 72 h.

In vitro cell viability detection: After the incubation, 50 μL of Cell Counting-Lite™ 2.0 reagent (Vazyme) was added to each well, shaken and mixed well in the dark, and reacted for 10 minutes before testing. Microplate reader (manufacturer: BMG, Model: PHERA Star-FS) was used for reading. The culture wells without cells were used as the background RLU, and the culture wells with cells but no compound were used as the cell control RLU. Cell inhibition rate = 1 - (sample RLU - background RLU) / (cell control RLU - background RLU) × 100%. The halfinhibition concentration ($IC_{50}$) of compound was calculated by fitting the curve according to the four-parameter model. The test results were shown in Table 4.

(2) Data results

Table 4. Inhibitory activity on HCC1806 cell proliferation

| Name | $IC_{50}$ (nM) |
|---|---|
| Example 1 (Compound **1-1**) | 1.23 |
| Example 2 (Compound **2-1**) | 8.08 |
| Example 3 (Compound **7-1**) | 3.67 |
| Example 4 (Compound **7-2**) | 18.58 |
| Example 5 (Compound **2-4**) | 1.08 |
| Example 6 (Compound **2-22**) | 6.09 |
| Example 7 (Compound **2-19**) | 0.78 |

The test results showed that the compounds of the present invention in Table 4 had significant inhibitory effect on the proliferation of HCC1806 human breast squamous cancer cells.

3. Inhibitory effect of compounds on NCI-H358 cell proliferation

(1) Cell plating: First, tumor cells NCI-H358 were cultured with corresponding culture medium, the cells were digested with trypsin and centrifuged, then the cells were resuspended and counted, and the cells were adjusted to an appropriate concentration for plating. The sources of tumor cells were shown in Table 5.

Table 5. Source of tumor cells

| Cell name | Tumor type | Source |
|---|---|---|
| NCI-H358 | Human non-small cell lung cancer cell | Nanjing Cobioer Biosciences Co., Ltd |

Co-incubation of the compounds of the present invention and tumor cells: 90 μL of cell suspension was added to 96-well plates respectively. After cell adherence, 10 μL of bioactive molecules (compounds of the present invention) diluted with culture medium were added into the wells of the above plates, and incubated for 72 h.

In vitro cell viability detection: After the incubation, 50 μL of Cell Counting-Lite™ 2.0 reagent (Vazyme) was added to each well, shaken and mixed well in the dark, and reacted for 10 minutes before testing. Microplate reader (manufacturer: BMG, Model: PHERA Star-FS) was used for reading. The culture wells without cells were used as the background RLU, and the culture wells with cells but no compound were used as the cell control RLU. Cell inhibition rate = 1 - (sample RLU - background RLU) / (cell control RLU - background RLU) × 100%. The halfinhibition concentration ($IC_{50}$) of compound was calculated by fitting the curve according to the four-parameter model. The test results were shown in Table 6.

(2) Data results

Table 6. Inhibitory activity on NCI-H358 cell proliferation

| Name | $IC_{50}$ (nM) |
|---|---|
| Example 1 (Compound **1-1**) | 25.67 |
| Example 2 (Compound **2-1**) | 120.90 |
| Example 3 (Compound **7-1**) | 267.80 |
| Example 4 (Compound **7-2**) | 287.00 |
| Example 5 (Compound **2-4**) | 53.40 |
| Example 7 (Compound **2-19**) | 12.70 |

The test results showed that the compounds of the present invention in Table 6 had significant inhibitory effects on the proliferation of NCI-H358 human non-small cell lung cancer cells.

4. Inhibitory effect of compounds on NCI-N87 cell proliferation

(1) Cell plating: First, tumor cells NCI-N87 were cultured with corresponding culture medium, the cells were digested with trypsin and centrifuged, then the cells were resuspended and counted, and the cells were adjusted to an appropriate concentration for plating. The sources of tumor cells were shown in Table 7.

Table 7. Source of tumor cells

| Cell name | Tumor type | Source |
|---|---|---|
| NCI-N87 | Human gastric cancer cell | ATCC |

Co-incubation of the compounds of the present invention and tumor cells: 90 μL of cell suspension was added to 96-well plates respectively. After cell adherence, 10 μL of bioactive molecules (compounds of the present invention) diluted with culture medium were added into the wells of the above plates, and incubated for 72 h.

In vitro cell viability detection: After the incubation, 50 μL of Cell Counting-Lite™ 2.0 reagent (Vazyme) was added to each well, shaken and mixed well in the dark, and reacted for 10 minutes before testing. Microplate reader (manufacturer: BMG, Model: PHERA Star-FS) was used for reading. The culture wells without cells were used as the background RLU, and the culture wells with cells but no compound were used as the cell control RLU. Cell inhibition rate = 1 - (sample RLU - background RLU) / (cell control RLU - background RLU) × 100%. The halfinhibition concentration ($IC_{50}$) of compound was calculated by fitting the curve according to the four-parameter model. The test results were shown in Table 8.

(2) Data results

Table 8. Inhibitory activity on NCI-N87 cell proliferation

| Name | $IC_{50}$ (nM) |
|---|---|
| Example 1 (Compound **1-1**) | 8.59 |
| Example 2 (Compound **2-1**) | 81.61 |
| Example 3 (Compound **7-1**) | 31.34 |
| Example 4 (Compound **7-2**) | 80.78 |

(continued)

| Name | $IC_{50}$ (nM) |
|---|---|
| Example 5 (Compound **2-4**) | 5.72 |
| Example 6 (Compound **2-22**) | 7.27 |
| Example 7 (Compound **2-19**) | 5.69 |

The test results showed that the compounds of the present invention in Table 8 had significant inhibitory effect on the proliferation of NCI-N87 human gastric cancer cells.

5. Inhibitory effect of compounds on MDA-MB-231 cell proliferation

(1) Cell plating: First, tumor cells MDA-MB-231 were cultured with corresponding culture medium, the cells were digested with trypsin and centrifuged, then the cells were resuspended and counted, and the cells were adjusted to an appropriate concentration for plating. The sources of tumor cells were shown in Table 9.

Table 9. Source of tumor cells

| Cell name | Tumor type | Source |
|---|---|---|
| MDA-MB-231 | Human breast cancer cell | Nanjing Cobioer Biosciences Co., Ltd |

Co-incubation of the compounds of the present invention and tumor cells: 90 μL of cell suspension was added to 96-well plates respectively. After cell adherence, 10 μL of bioactive molecules (compounds of the present invention) diluted with culture medium were added into the wells of the above plates, and incubated for 72 h.
In vitro cell viability detection: After the incubation, 50 μL of Cell Counting-Lite™ 2.0 reagent (Vazyme) was added to each well, shaken and mixed well in the dark, and reacted for 10 minutes before testing. Microplate reader (manufacturer: BMG, Model: PHERA Star-FS) was used for reading. The culture wells without cells were used as the background RLU, and the culture wells with cells but no compound were used as the cell control RLU. Cell inhibition rate = 1 - (sample RLU - background RLU) / (cell control RLU - background RLU) × 100%. The halfinhibition concentration ($IC_{50}$) of compound was calculated by fitting the curve according to the four-parameter model. The test results were shown in Table 10.

(2) Data results

Table 10. Inhibitory activity on MDA-MB-231 cell proliferation

| Name | $IC_{50}$ (nM) |
|---|---|
| Example 1 (Compound **1-1**) | 3.55 |
| Example 2 (Compound **2-1**) | 38.46 |
| Example 3 (Compound **7-1**) | 30.98 |
| Example 4 (Compound **7-2**) | 30.61 |

The test results showed that the compounds of the present invention in Table 10 had significant inhibitory effects on the proliferation of MDA-MB-231 human breast cancer cells.

6. Inhibitory effect of compounds on Jeko-1 cell proliferation

(1) Cell plating: First, tumor cells Jeko-1 were cultured with corresponding culture medium, the cells were resuspended and counted, and finally the cells were adjusted to an appropriate concentration for plating. The sources of tumor cells were shown in Table 11.

Table 9. Source of tumor cells

| Cell name | Tumor type | Source |
|---|---|---|
| Jeko-1 | Human mantle cell lymphoma cell | ATCC |

Co-incubation of the compounds of the present invention and tumor cells: 90 μL of cell suspension was added to 96-well plates respectively. After cell adherence, 10 μL of bioactive molecules (compounds of the present invention) diluted with culture medium were added into the wells of the above plates, and incubated for 72 h.

In vitro cell viability detection: After the incubation, 50 μL of Cell Counting-Lite™ 2.0 reagent (Vazyme) was added to each well, shaken and mixed well in the dark, and reacted for 10 minutes before testing. Microplate reader (manufacturer: BMG, Model: PHERA Star-FS) was used for reading. The culture wells without cells were used as the background RLU, and the culture wells with cells but no compound were used as the cell control RLU. Cell inhibition rate = 1 - (sample RLU - background RLU) / (cell control RLU - background RLU) × 100%. The halfinhibition concentration ($IC_{50}$) of compound was calculated by fitting the curve according to the four-parameter model. The test results were shown in Table 12.

(2) Data results

Table 12. Inhibitory activity on Jeko-1 cell proliferation

| Name | $IC_{50}$ (nM) |
|---|---|
| Example 2 (Compound **2-1**) | 1.62 |
| Example 3 (Compound **7-1**) | 4.07 |
| Example 4 (Compound **7-2**) | 2.17 |
| Example 5 (Compound **2-4**) | 0.34 |
| Example 6 (Compound **2-22**) | 1.11 |
| Example 7 (Compound **2-19**) | 1.09 |

The test results showed that the compounds of the present invention in Table 12 had significant inhibitory effect on the proliferation of Jeko-1 human mantle cell lymphoma cells.

7. Inhibitory effect of compounds on MDA-MB-453 cell proliferation

(1) Cell plating: First, tumor cells MDA-MB-453 were cultured with corresponding culture medium, the cells were digested with trypsin and centrifuged, then the cells were resuspended and counted, and the cells were adjusted to an appropriate concentration for plating. The sources of tumor cells were shown in Table 13.

Table 13. Source of tumor cells

| Cell name | Tumor type | Source |
|---|---|---|
| MDA-MB-453 | Human breast cancer cell | Jiangyin Concortis Biotechnology Co., Ltd |

Co-incubation of the compounds of the present invention and tumor cells: 90 μL of cell suspension was added to 96-well plates respectively. After cell adherence, 10 μL of bioactive molecules (compounds of the present invention)

diluted with culture medium were added into the wells of the above plates, and incubated for 72 h.

In vitro cell viability detection: After the incubation, 50 μL of Cell Counting-Lite™ 2.0 reagent (Vazyme) was added to each well, shaken and mixed well in the dark, and reacted for 10 minutes before testing. Microplate reader (manufacturer: BMG, Model: PHERA Star-FS) was used for reading. The culture wells without cells were used as the background RLU, and the culture wells with cells but no compound were used as the cell control RLU. Cell inhibition rate = 1 - (sample RLU - background RLU) / (cell control RLU - background RLU) × 100%. The halfinhibition concentration ($IC_{50}$) of compound was calculated by fitting the curve according to the four-parameter model. The test results were shown in Table 14.

(2) Data results

Table 14. Inhibitory activity on MDA-MB-453 cell proliferation

| Name | $IC_{50}$ (nM) |
|---|---|
| Example 1 (Compound **1-1**) | 290.6 |
| Example 2 (Compound **2-1**) | 156.2 |
| Example 3 (Compound **7-1**) | 50.19 |
| Example 4 (Compound **7-2**) | 202.6 |
| Example 5 (Compound **2-4**) | 95.56 |

The test results showed that the compounds of the present invention in Table 14 had significant inhibitory effect on the proliferation of MDA-MB-453 human breast cancer cells.

8. Inhibitory effect of compounds on HCC827 cell proliferation

(1) Cell plating: First, tumor cells HCC827 were cultured with corresponding culture medium, the cells were digested with trypsin and centrifuged, then the cells were resuspended and counted, and the cells were adjusted to an appropriate concentration for plating. The sources of tumor cells were shown in Table 15.

Table 13. Source of tumor cells

| Cell name | Tumor type | Source |
|---|---|---|
| HCC827 | Non-small cell lung cancer | ATCC |

Co-incubation of the compounds of the present invention and tumor cells: After cell adherence, the culture medium in the cells was removed, and the diluted bioactive molecules (compounds of the present invention) were added into the wells of the above plates, and incubated for 72 h.

In vitro cell viability detection: After the incubation, 50 μL of Cell Counting-Lite™ 2.0 reagent (Vazyme) was added to each well, shaken and mixed well in the dark, and reacted for 10 minutes before testing. Microplate reader (manufacturer: BMG, Model: PHERA Star-FS) was used for reading. The Cell Counting-Lite™ without cells was used to obtain the background RLU, and the Cell Counting-Lite™ with cells was used obtain the solvent RLU. Cell inhibition rate = 1 - (sample RLU - background RLU) / (solvent RLU - background RLU) × 100%. The halfinhibition concentration ($IC_{50}$) of compound was calculated by fitting the curve according to the four-parameter model. RLU: relative light unit. The test results were shown in Table 16.

(2) Data results

Table 16. Inhibitory activity of compounds on HCC827 cell proliferation

| Name | $IC_{50}$ (nM) |
|---|---|
| Comparative Compound **4** | 221.90 |
| **2-25** | 109.80 |

The test results showed that the compound of the present invention in Table 16 had inhibitory effect on the proliferation of HCC827 human non-small cell lung cancer cells.

9. Inhibitory effect of compounds on HCC1954 cell proliferation

(1) Cell plating: First, tumor cells HCC1954 were cultured with corresponding culture medium, the cells were digested with trypsin and centrifuged, then the cells were resuspended and counted, and the cells were adjusted to an appropriate concentration for plating. The sources of tumor cells were shown in Table 17.

Table 13. Source of tumor cells

| Cell name | Tumor type | Source |
|---|---|---|
| HCC1954 | Human breast cancer | Jiangyin Concortis Biotechnology Co., Ltd |

Co-incubation of the compounds of the present invention and tumor cells: After cell adherence, the culture medium in the cells was removed, and the diluted bioactive molecules (compounds of the present invention) were added into the wells of the above plates, and incubated for 72 h.

In vitro cell viability detection: After the incubation, 50 μL of Cell Counting-Lite™ 2.0 reagent (Vazyme) was added to each well, shaken and mixed well in the dark, and reacted for 10 minutes before testing. Microplate reader (manufacturer: BMG, Model: PHERA Star-FS) was used for reading. The Cell Counting-Lite™ without cells was used to obtain the background RLU, and the Cell Counting-Lite™ with cells was used obtain the solvent RLU. Cell inhibition rate = 1 - (sample RLU - background RLU) / (solvent RLU - background RLU) × 100%. The halfinhibition concentration ($IC_{50}$) of compound was calculated by fitting the curve according to the four-parameter model. RLU: relative light unit. The test results were shown in Table 18.

(2) Data results

Table 18. Inhibitory activity of compounds on HCC1954 cell proliferation

| Name | $IC_{50}$ (nM) |
|---|---|
| Comparative Compound **4** | 195.20 |
| **2-25** | 170.70 |

The test results showed that the compound of the present invention in Table 18 had inhibitory effect on the proliferation of HCC1954 human breast cancer cells.

10. Inhibitory effect of compounds on NCI-H1975 cell proliferation

(1) Cell plating: First, tumor cells NCI-H1975 were cultured with corresponding culture medium, the cells were digested with trypsin and centrifuged, then the cells were resuspended and counted, and the cells were adjusted to an appropriate concentration for plating. The sources of tumor cells were shown in Table 19.

Table 19. Source of tumor cells

| Cell name | Tumor type | Source |
|---|---|---|
| NCI-H1975 | Human non-small cell lung cancer | Nanjing Cobioer Biosciences Co., Ltd. |

Co-incubation of the compounds of the present invention and tumor cells: After cell adherence, the culture medium in the cells was removed, and the diluted bioactive molecules (compounds of the present invention) were added into the wells of the above plates, and incubated for 72 h.

In vitro cell viability detection: After the incubation, 50 μL of Cell Counting-Lite™ 2.0 reagent (Vazyme) was added to each well, shaken and mixed well in the dark, and reacted for 10 minutes before testing. Microplate reader (manufacturer: BMG, Model: PHERA Star-FS) was used for reading. The Cell Counting-Lite™ without cells was used to obtain the background RLU, and the Cell Counting-Lite™ with cells was used obtain the solvent RLU. Cell inhibition rate = 1 - (sample RLU - background RLU) / (solvent RLU - background RLU) × 100%. The halfinhibition concentration ($IC_{50}$) of compound was calculated by fitting the curve according to the four-parameter model. RLU: relative light unit. The test results were shown in Table 20.

(2) Data results

Table 20. Inhibitory activity of compounds on NCI-H1975 cell proliferation

| Name | $IC_{50}$ (nM) |
|---|---|
| Comparative Compound **4** | 68.87 |
| **2-25** | 29.38 |

The test results showed that the compound of the present invention in Table 20 had inhibitory effect on the proliferation of NCI-H1975 human non-small cell lung cancer cells.

II. Permeability test

The MDCK cell model was used to evaluate the permeability of the compounds, the LC-MS/MS method was used to determine the concentration of the substance to be tested, and the apparent permeability coefficient ($P_{app}$) and efflux ratio were calculated [Efflux ratio = $P_{app}(B \rightarrow A)/P_{app}(A \rightarrow B)$].

1. Preparation of reagents

Complete culture medium: 10% fetal bovine serum and 1% penicillin/streptomycin were added to MEM culture medium.

Digestive solution: 0.05% trypsin-EDTA.

HBSS buffer: HBSS containing $Ca^{2+}$, $Mg^{2+}$.

Permeating solution: HBSS containing 10mM Hepes, 4% BSA, pH 7.4.

Compound stock solution: a certain amount of the compound to be tested was weighed and formulated with DMSO to prepare a 10 mM stock solution.

2. Cell culture

MDCK cells (26$^{th}$ generation) in the logarithmic growth phase were digested and dispersed evenly with 0.05% trypsin-EDTA, and then inoculated into the Transwell chamber at $3*10^5$ cells/$cm^2$, with 200 μL on the A side and 1000 μL on the B side. After inoculation, the medium was changed once every day and the culturing was carried out for 3 to 4 days.

3. Permeability test

The cell culture medium was discarded by pipetting, then washing was carried out three times with the permeating solution. 200 μL of permeating solution was added to A side and 1000 μL of permeating solution was added to B side. A voltage resistance meter was used to measure the cell transmembrane resistance (TEER). After the TEER value of the cells was measured, the permeating solution on both sides was discarded by pipetting, and the solution was

added. After 120 minutes, 150 μL of samples were taken from A side and B side, respectively, and frozen at - 80°C before testing. Then, 10 μg/ml Fluorescent Yellow was added to A side. After incubation for 30 minutes, 100 μL of soluton at B side was pipetted and added into a 96-well white plate. The fluorescence value was measured at the wavelengths of Ex=485 nm and Em=530 nm using a microplate reader, and the permeance was calculated (which was less than 1%).

4. Sample processing

80 μL of the compound to be tested was taken, added to 320 μL of acetonitrile solution containing internal standard (30 ng/mL Tolbutamide), vortexed, and centrifuged for 10 min (4000 rpm, 4°C), and the supernatant was taken for LC-MS/MS analysis.

5. Data processing

Excel 2013 was used to calculate $P_{app}$ values and $P_{app}$ ratio of B side → A side to A side → B side, and the recovery rates of each Control Compound and the compound to be tested were calculated.

(1) Calculation of apparent permeability

Apparent permeability coefficient ($P_{app}$) of compound was calculated according to the following formula:

$$P_{app} = (dC_r/dt) \times V_r/(A \times C_0)$$

$C_0$ was the initial concentration (μM) of the drug to be tested at the donor, $(dC_r/dt) \times V_r$ was the rate (μM/s) at which the drug to be tested appeared at the receiver, $V_r$ was the solution volume (mL) at the receiver, and A was the surface area ($cm^2$) of polycarbonate membrane.

Evaluation criteria:

Low permeability: $P_{app} \leq 0.5$ ($\times 10^{-6}$ cm/s)
Medium permeability: $0.5 < P_{app} < 2.5$ ($\times 10^{-6}$ cm/s)
High permeability: $P_{app} \geq 2.5$ ($\times 10^{-6}$ cm/s)

(2) Transporter

Efflux ratio (ER) was calculated according to $P_{app}$:

$$ER = P_{app}(B \rightarrow A)/P_{app}(A \rightarrow B)$$

wherein, $P_{app}(B \rightarrow A)$ was the apparent permeability coefficient of the drug to be tested from the BL (baso-lateral) end to the AP (apical) end, and $P_{app}(A \rightarrow B)$ was the apparent permeability coefficient of the drug to be tested from the AP end to the BL end.

Evaluation criteria:

Probably being an efflux transporter substrate (Probably): ERa ≥ 2 and ERa/ERi > 2
Likely being an efflux transporter substrate (Likely): ERa ≥ 2
Unlikely or impossibly being an efflux transporter substrate (Poor or non): ERa < 2

wherein, ERi and ERa were the efflux ratios of the compound to be tested in the presence and absence of P-gp inhibitor, respectively.

The recovery rate was calculated by the formula as follows:

$$\text{Recovery rate } (\%) = 100 \times [(V_r \times C_r) + (V_d \times C_d)] / (V_d \times C_0)$$

wherein, $V_d$ and $V_r$ were the solution volumes on the donor side and receiver side respectively, and $C_d$ and $C_r$ were the compound concentrations on the donor side and receiver side respectively.

6. Results

The specific data was as follows:

| Name | Concentration (μM) | Evaluation $P_{app}$ ($10^{-6}$cm/s) | | Efflux ratio (ERa) |
|---|---|---|---|---|
| | | A to B | B to A | |
| Control Compound 1 | 2.00 | 1.31 | 2.71 | 2.06 |
| Control Compound 2 | 2.00 | 0.01 | 0.40 | 28.0 |
| Control Compound 3 | 2.00 | 0.31 | 4.65 | 15.2 |
| Control Compound 4 | 2.00 | 0.24 | 5.25 | 21.7 |
| Compound **1-1** | 2.00 | 2.59 | 3.65 | 1.41 |
| Compound **2-1** | 2.00 | 0.52 | 0.42 | 0.81 |
| Compound **2-19** | 2.00 | 0.50 | 0.73 | 1.48 |
| Compound **2-22** | 2.00 | 0.71 | 1.59 | 2.24 |
| Compound **2-25** | 2.00 | 0.78 | 3.56 | 4.57 |

According to the $P_{app}$(A→B) results, Control Compound 2, Control Compound 3, Control Compound 4 and Compound **2-19** were all low-permeability compounds, especially the permeability of Control Compound 2 was relatively low; Control Compound 1, Compound **2-1,** Compound **2-22** and Compound **2-25** were medium-permeability compounds; Compound **1-1** was a high-permeability compound; the efflux ratio data indicated that Control Compound 1, Control Compound 2, Control Compound 3, Control Compound 4, Compound **2-22** and Compound **2-25** could be efflux transporter substrates, especially the efflux ratios of Control Compound 2, Control Compound 3 and Control Compound 4 were relatively high; Compound **1-1,** Compound **2-1** and Compound **2-19** were not efflux transporter substrates or were weak efflux transporter substrates.

III. Liver microsome stability test

Liver microsomes were used as an in vitro model to evaluate the metabolic stability of compounds in human and monkey liver microsomes.

1. Materials and methods

1.1 Main test materials

Control Compounds and the compounds of the present invention:

Testosterone: Dr. Ehrenstorfer, Germany

Mixed human liver microsomes: Xenotech, USA

Mixed male cynomolgus liver microsomes: RILD, China

1.2 Liver microsome incubation system

The positive compound testosterone or the tested substance (liver microsome solution, 50 μL) was mixed with PBS (25 μL). After pre-incubation (37°C) for 5 min, NADPH (25 μL) was added to make the final concentration of the positive compound or the tested substance 1 μM, the final concentration of human liver microsomal protein was 0.5 mg/ml, the final concentration of monkey liver microsomal protein was 1 mg/ml, the tested substance group and the positive compound group were incubated for 0 min, 15 min. After being reacted for corresponding time, 300 μL of ice-cold acetonitrile containing internal standard was added to terminate the reaction, vortexed, and stored at -80°C for later analysis. All incubated samples were double samples.

1.3 Sample pretreatment

The tested compound was vortexed for 1 min, and centrifuged for 10 min (4°C, 4000 rpm), and 300 μL of the supernatant was taken for LC-MS/MS analysis.

2. Data processing

Excel software was used to calculate the remaining rate (%) of the drug in primary form in the incubation system:

$$\text{Primary form remaining rate (\%)} = 100\times(A_{\text{incubated sample}}/A_{0h})$$

Note: $A_{\text{incubated sample}}$ represented the peak area ratio of the compound to the internal standard after incubation for the corresponding time; $A_{0h}$ represented the peak area ratio of the compound to the internal standard when unreacted.

3. Results and conclusions

The remaining rates of the compounds in primary form after incubation in human and monkey liver microsomes for 15 minutes were shown in Table 22.

Table 22: Primary form remaining rates of compounds after incubation in human and monkey liver microsomes for 15 min

| Compound | Remaining rate (%) | |
|---|---|---|
| | Human | Monkey |
| Control Compound 4 | 64.6 | 65.1 |
| Compound **2-25** | 80.0 | 79.5 |

The data in Table 22 showed that compared to the Control Compound 4, Compound **2-25** had obvious advantages of stability in liver microsomes.

Structures of control compounds:

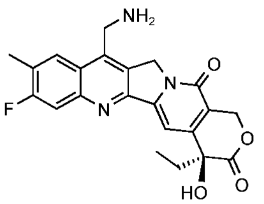

Control Compound 1

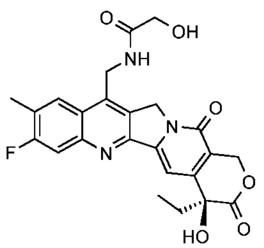

Control Compound 2

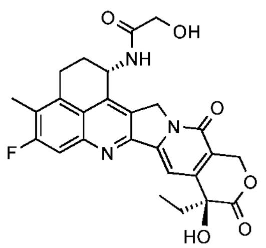

Control Compound 3 (Dxd)

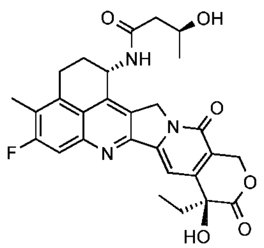

Control Compound 4

Although the specific embodiments of the present invention have been described in detail, those skilled in the art will understand that various modifications and substitutions can be made to those details based on all teachings that have been disclosed, and these changes are within the scope of the present invention. The full scope of the present invention is given by the appended claims and any equivalents thereof.

## Claims

1. A compound or a pharmaceutically acceptable salt, ester, stereoisomer, polymorph, solvate, N-oxide, isotopically labeled compound, metabolite and prodrug thereof, wherein the compound has the following structure:

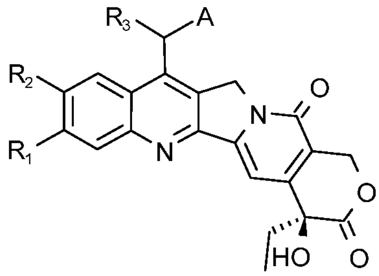

Formula (I)

wherein,

$R_1$ is selected from the group consisting of hydrogen, fluorine, and chlorine;
$R_2$ is selected from the group consisting of hydrogen, methyl, fluorine, chlorine, and hydroxyl; or $R_1$ and $R_2$ together with the carbon atom to which they are attached are connected to form a 5-to 6-membered oxygen-containing heterocycle;
$R_3$ is selected from the group consisting of hydrogen, $C_{1-6}$ alkyl, deuterated $C_{1-6}$ alkyl, $C_{1-6}$ haloalkyl, $C_{1-6}$ alkoxyalkyl, $C_{3-6}$ cycloalkyl, and 3- to 6-membered heterocyclyl, or $R_3$ is connected to an adjacent benzene ring carbon atom to form a 6-membered carbocycle;
A is selected from the group consisting of

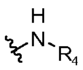, 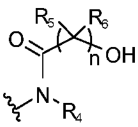, 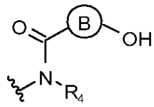, 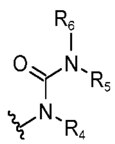,

and

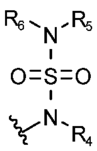;

Ring B is a 3- to 6-membered carbocycle or a 3- to 6-membered heterocycle;
$R_4$ is selected from the group consisting of hydrogen, $C_{1-6}$ alkyl, $C_{1-6}$ haloalkyl, $C_{1-6}$ alkoxyalkyl, $C_{3-6}$ cycloalkyl, and 3- to 6-membered heterocyclyl;
$R_5$ and $R_6$ are each independently selected from the group consisting of hydrogen, $C_{1-6}$ alkyl, $C_{2-6}$ alkenyl, $C_{3-6}$ cycloalkyl, and $C_{1-6}$ haloalkyl; or $R_5$ or $R_6$ together with the carbon atom to which they are attached form a 3- to 6-membered ring;
or, $R_4$ and $R_5$ are connected to form a 4- to 6-membered ring;
n = 1 to 6.

**2.** The compound or pharmaceutically acceptable salt, ester, stereoisomer, polymorph, solvate, N-oxide, isotopically labeled compound, metabolite and prodrug thereof according to claim 1, wherein the compound has the structure of Formula (II):

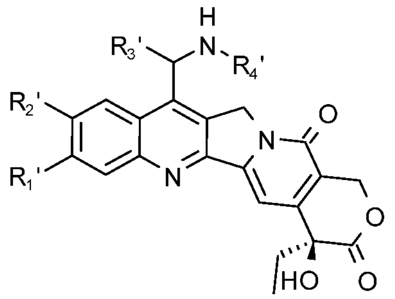

Formula (II)

in Formula (II),

$R_1$' is selected from the group consisting of hydrogen, fluorine, and chlorine;
$R_2$' is selected from the group consisting of methyl, fluorine, chlorine, and hydroxyl;
$R_3$' is selected from the group consisting of hydrogen, $C_{1-6}$ alkyl, deuterated $C_{1-6}$ alkyl, $C_{1-6}$ haloalkyl, $C_{1-6}$ alkoxyalkyl, $C_{3-6}$ cycloalkyl, and 3- to 6-membered heterocyclyl; $R_3$' is preferably hydrogen, $C_{1-6}$ alkyl, deuterated $C_{1-6}$ alkyl, $C_{1-6}$ haloalkyl, or $C_{1-6}$ alkoxyalkyl;
$R_4$' is selected from the group consisting of hydrogen, $C_{1-6}$ alkyl, and $C_{1-6}$ haloalkyl; $R_4$' is preferably hydrogen;
when $R_1$' is fluorine and $R_2$' is methyl, $R_3$' and $R_4$' are not hydrogen at the same time; and
when $R_1$' is fluorine and $R_2$' is methyl, $R_4$' is not $C_{1-6}$ alkyl.

**3.** The compound or pharmaceutically acceptable salt, ester, stereoisomer, polymorph, solvate, N-oxide, isotopically labeled compound, metabolite and prodrug thereof according to claim 1, wherein the compound has the structure of Formula (III):

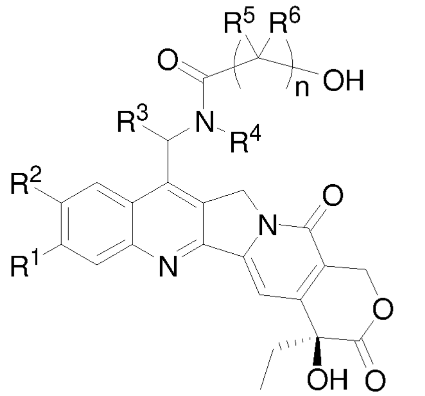

Formula (III)

in Formula (III),

$R^1$ is selected from the group consisting of hydrogen, fluorine, and chlorine;
$R^2$ is selected from the group consisting of hydrogen, methyl, fluorine, chlorine, and hydroxyl;
$R^3$ is selected from the group consisting of hydrogen, $C_{1-6}$ alkyl, $C_{1-6}$ haloalkyl, $C_{1-6}$ alkoxyalkyl, $C_{3-6}$ cycloalkyl, and 3- to 6-membered heterocyclyl, or $R^3$ is connected to an adjacent benzene ring carbon atom to form a 6-membered carbocycle; $R^3$ is preferably hydrogen;
$R_4$ is hydrogen or $C_{1-6}$ alkyl;
$R^5$ and $R^6$ are independently selected from the group consisting of hydrogen, $C_{1-6}$ alkyl, $C_{1-6}$ haloalkyl, $C_{3-6}$ cycloalkyl, $C_{2-6}$ alkenyl, and 3- to 6-membered heterocyclyl, or $R^5$ and $R^6$ together with the carbon atom to which they are attached form a 3- to 6-membered ring;
or, $R^4$ and $R^5$ are connected to form a 4- to 6-membered ring;
n = 1 or 2;

when $R^1$ is fluorine, $R^2$ is methyl, $R^3$ is hydrogen, and $R^4$ is hydrogen, $R^5$ and $R^6$ are not hydrogen at the same time, nor are they together with the carbon atom to which they are attached form a cyclopropyl; and when $R^1$ is fluorine, $R^2$ is methyl, and $R^3$ is hydrogen, $R^4$ is not alkyl.

**4.** The compound or pharmaceutically acceptable salt, ester, stereoisomer, polymorph, solvate, N-oxide, isotopically labeled compound, metabolite and prodrug thereof according to claim 1, wherein the compound has the structure of Formula (IV):

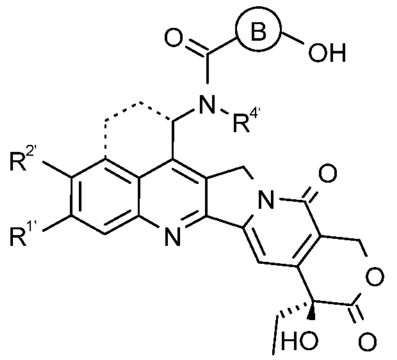

Formula (IV)

in Formula (IV),

$R^{1'}$ is selected from the group consisting of hydrogen, fluorine, and chlorine;
$R^{2'}$ is selected from the group consisting of hydrogen, methyl, fluorine, chlorine, and hydroxyl;
$R^{4'}$ is selected from the group consisting of hydrogen, $C_{1-6}$ alkyl, $C_{3-6}$ cycloalkyl, and $C_{1-6}$ haloalkyl; $R^{4'}$ is preferably hydrogen;
B is selected from the group consisting of $C_{1-6}$ alkylene, 3- to 6-membered carbocycle, and 3- to 6-membered heterocycle;
when $R^{1'}$ is fluorine, $R^{2'}$ is methyl, and the dashed carbocycle exists, $R^{4'}$ is neither hydrogen nor $C_{1-6}$ alkyl; and when $R^{2'}$ is methyl, $R^{1'}$ is fluorine, and the dashed carbocycle does not exist, Ring B is not a 3-membered carbocycle.

**5.** The compound or pharmaceutically acceptable salt, ester, stereoisomer, polymorph, solvate, N-oxide, isotopically labeled compound, metabolite and prodrug thereof according to claim 1, the compound has the structure as follows:

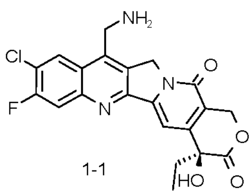
1-1

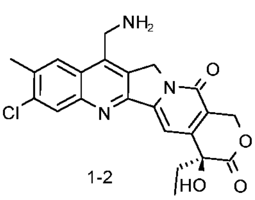
1-2

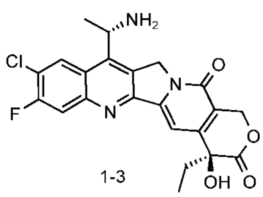
1-3

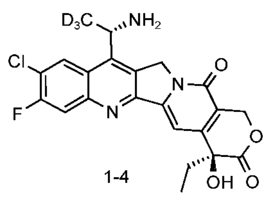
1-4

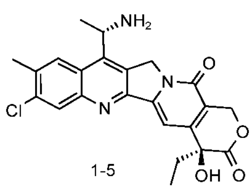
1-5

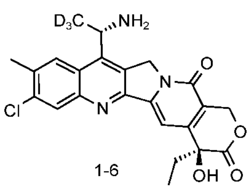
1-6

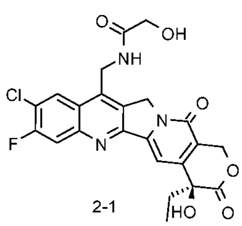
2-1

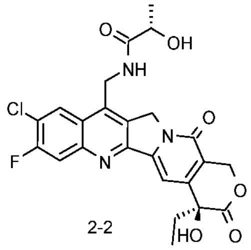
2-2

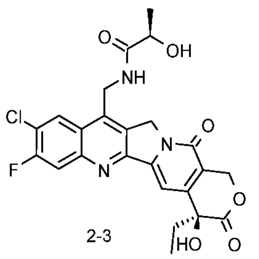
2-3

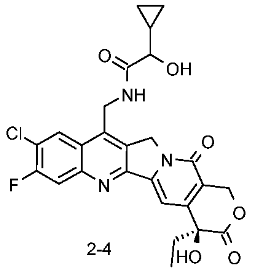
2-4

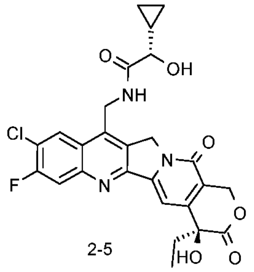
2-5

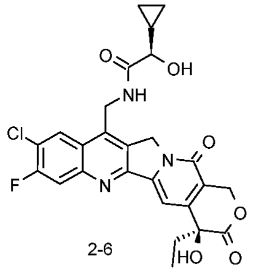
2-6

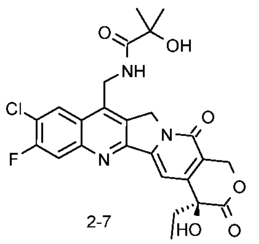
2-7

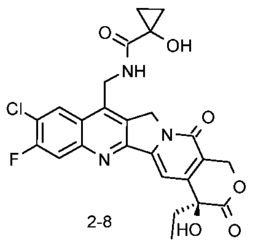
2-8

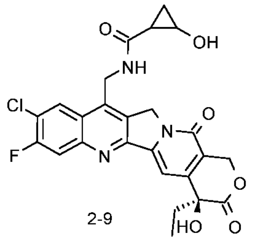
2-9

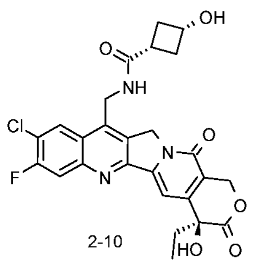
2-10

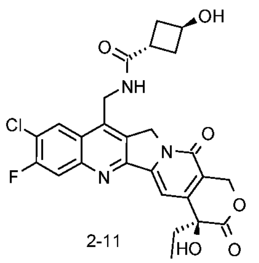
2-11

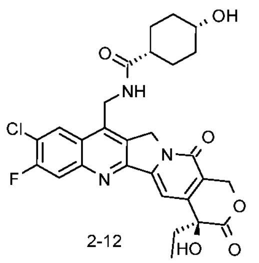
2-12

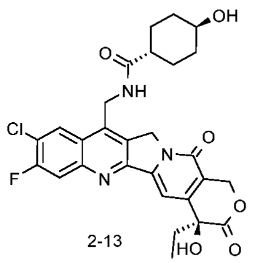
2-13

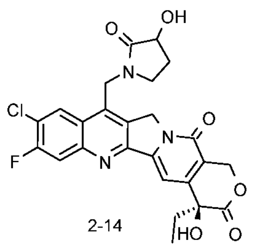
2-14

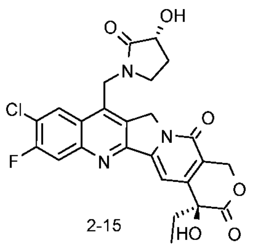
2-15

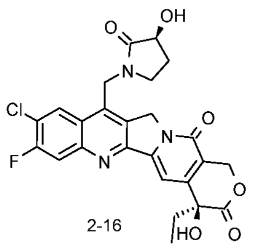
2-16

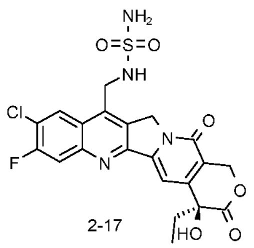
2-17

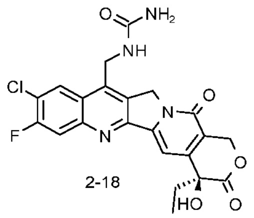
2-18

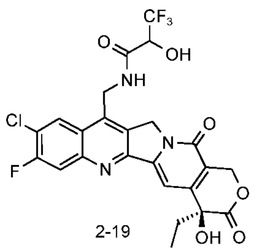
2-19

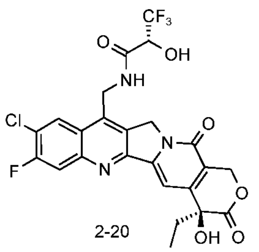
2-20

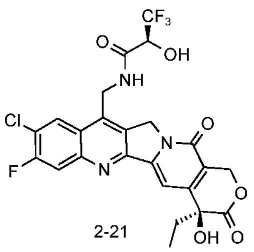
2-21

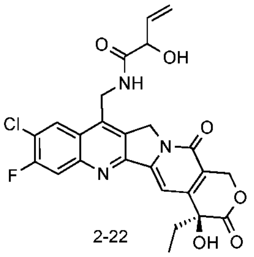
2-22

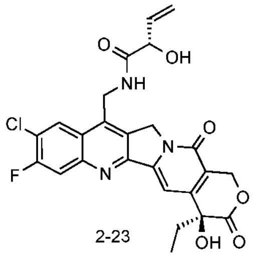
2-23

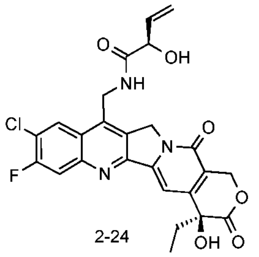
2-24

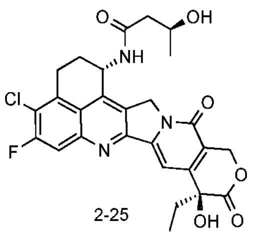
2-25

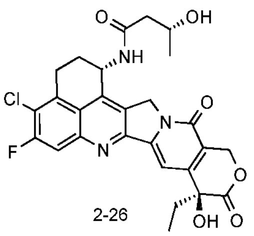
2-26

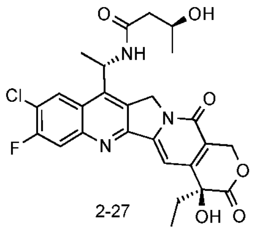
2-27

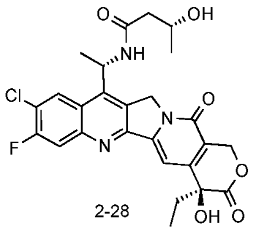
2-28

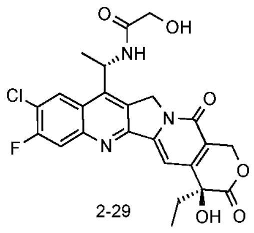
2-29

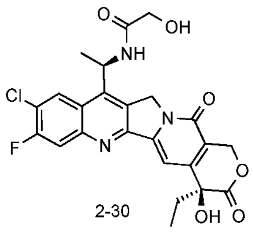
2-30

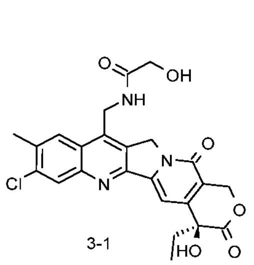
3-1

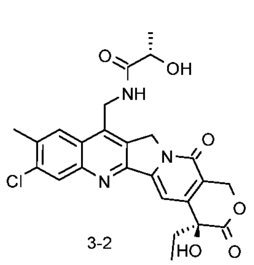
3-2

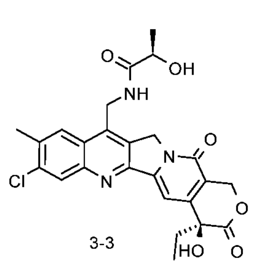
3-3

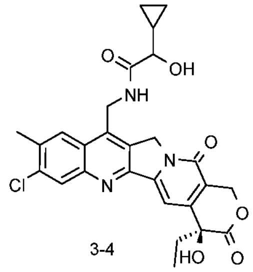
3-4

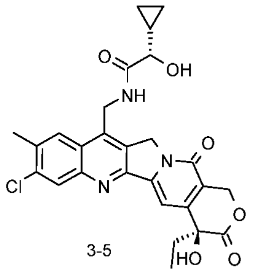
3-5

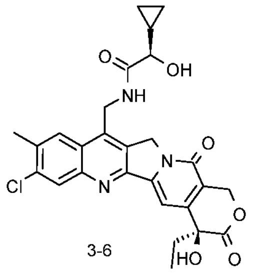
3-6

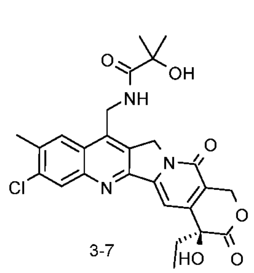
3-7

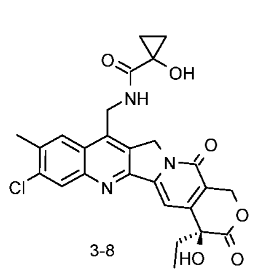
3-8

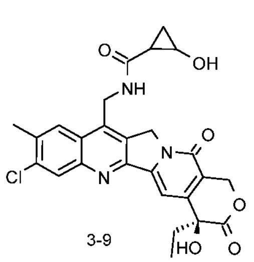
3-9

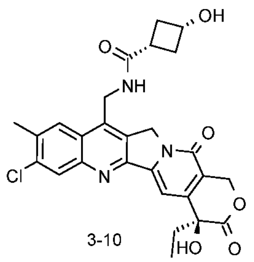
3-10

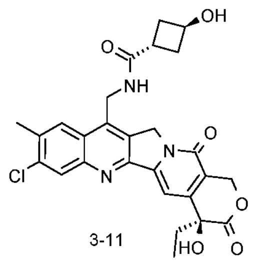
3-11

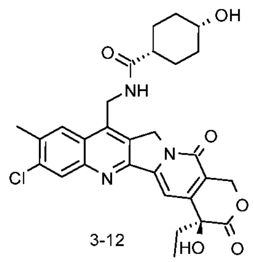
3-12

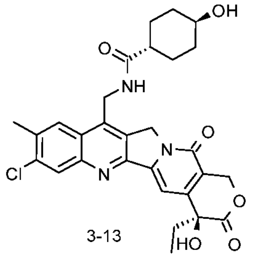
3-13

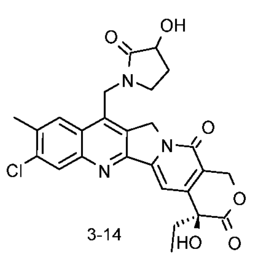
3-14

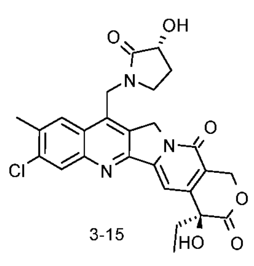
3-15

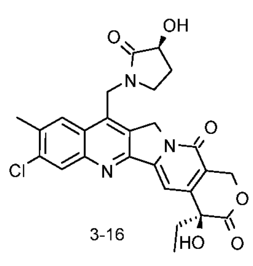
3-16

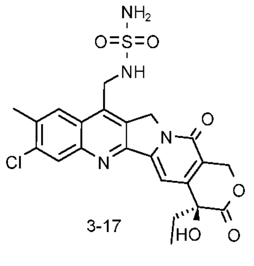
3-17

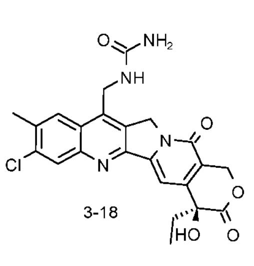
3-18

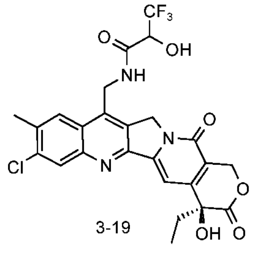
3-19

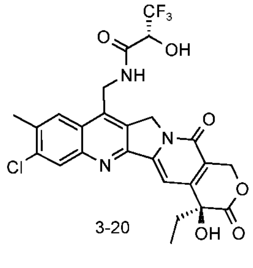
3-20

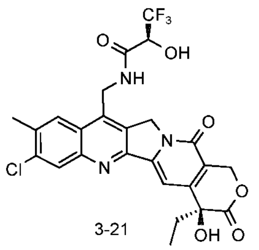
3-21

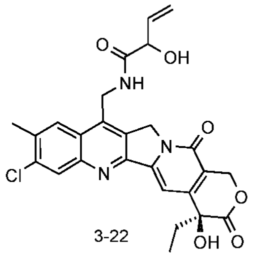
3-22

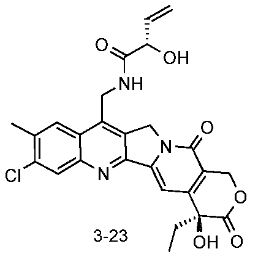
3-23

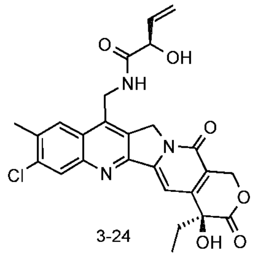
3-24

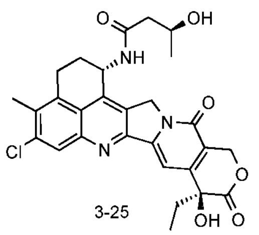
3-25

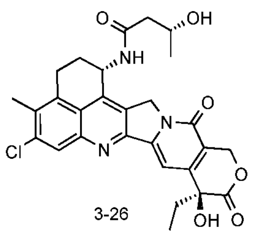
3-26

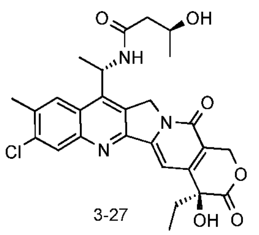
3-27

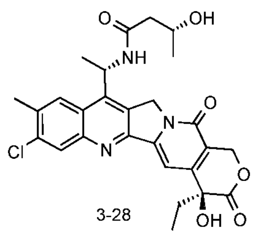
3-28

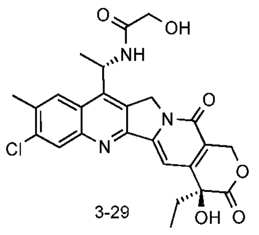
3-29

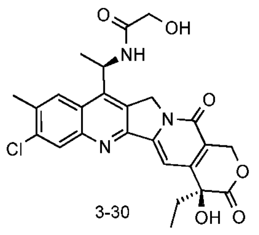
3-30

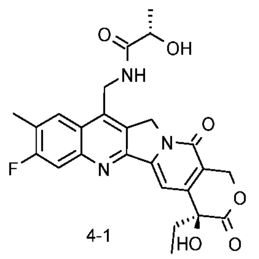
4-1

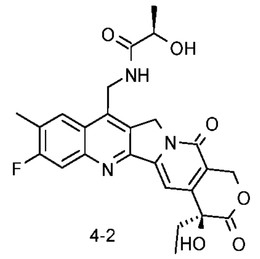
4-2

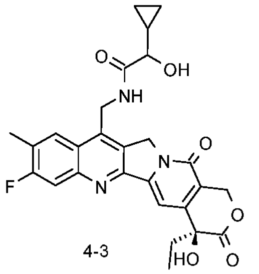
4-3

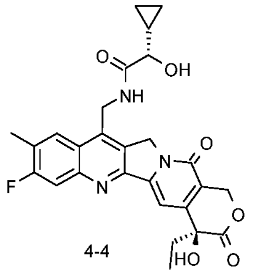
4-4

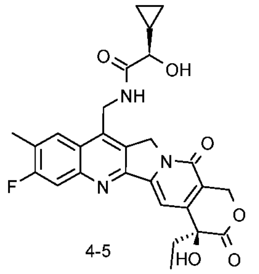
4-5

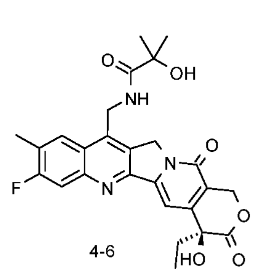
4-6

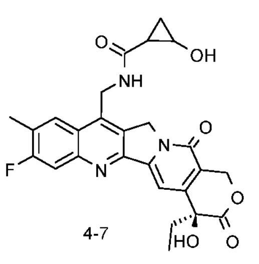
4-7

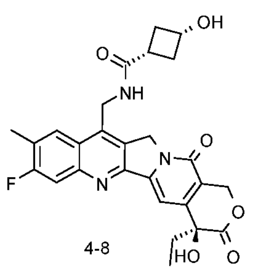
4-8

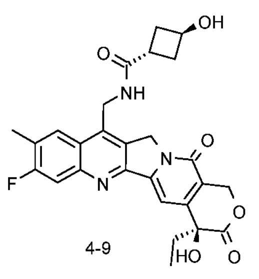
4-9

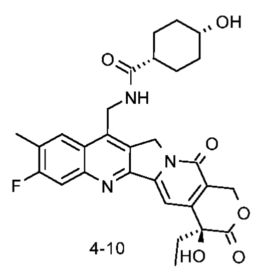
4-10

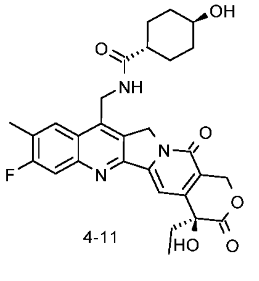
4-11

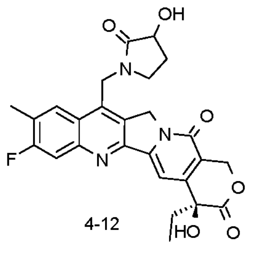
4-12

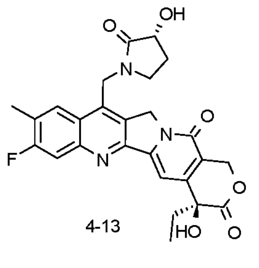
4-13

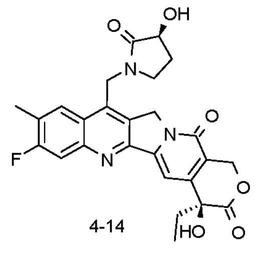
4-14

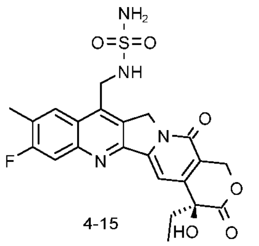
4-15

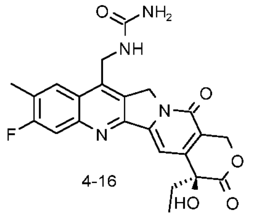
4-16

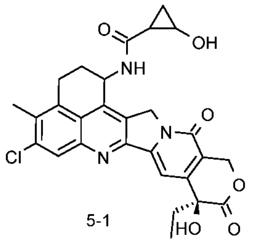
5-1

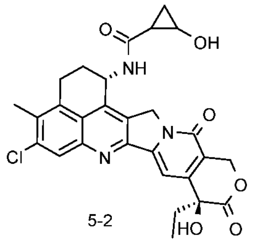
5-2

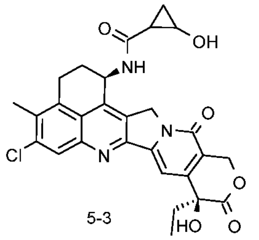
5-3

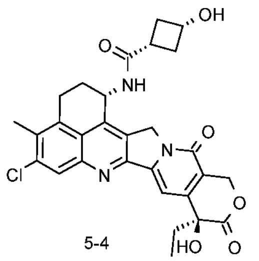
5-4

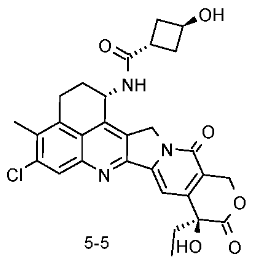
5-5

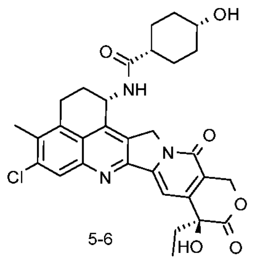
5-6

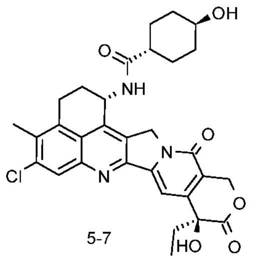
5-7

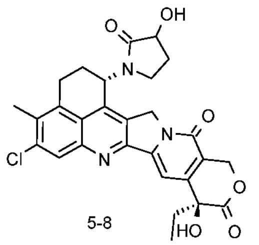
5-8

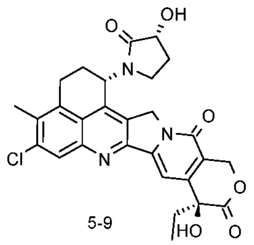
5-9

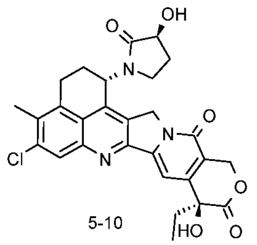
5-10

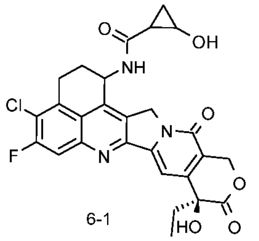
6-1

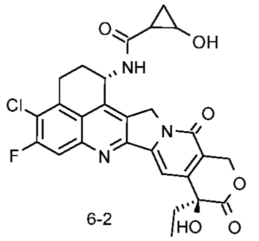
6-2

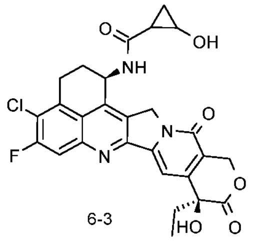
6-3

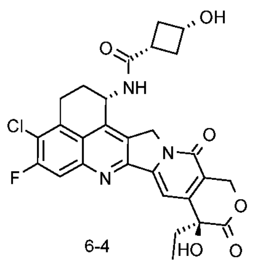
6-4

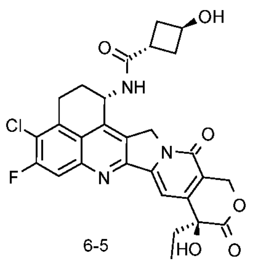
6-5

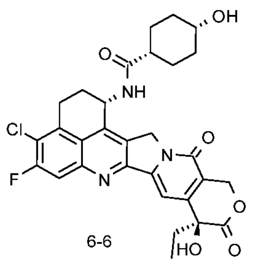
6-6

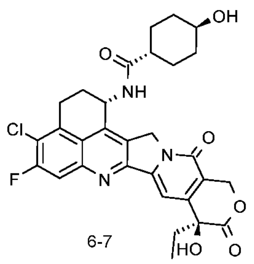
6-7

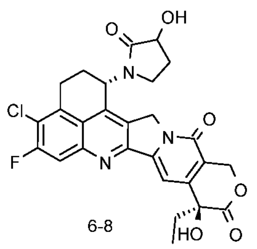
6-8

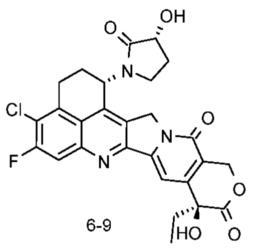
6-9

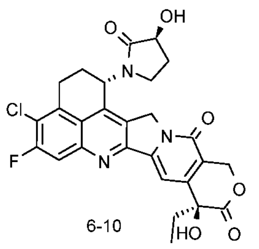
6-10

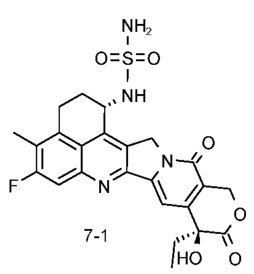
7-1

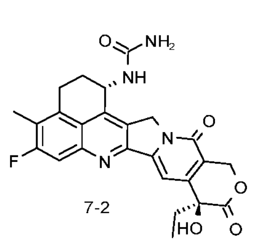
7-2

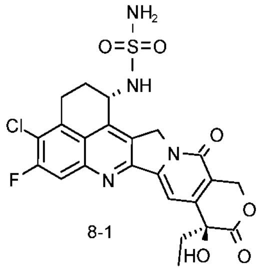
8-1
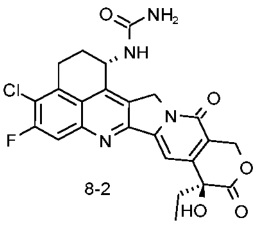
8-2
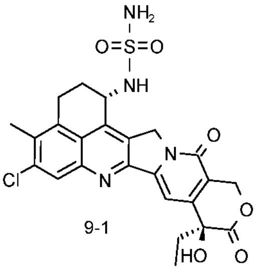
9-1
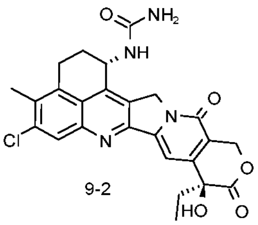
9-2
.

**6.** A compound represented by Formula (V) or a pharmaceutically acceptable salt, ester, stereoisomer, polymorph, solvate, N-oxide, isotopically labeled compound, metabolite and prodrug thereof, wherein the compound has the structure as follows:

M-L-E-D Formula(V)

wherein,

M represents a linking site connecting an antibody or antigen-binding fragment thereof;
L represents a linker connecting the linking site M and E;
E represents a structural fragment connecting L and D;
D represents a structural fragment of a cytotoxic drug;
preferably, M is selected from the group consisting of the following structures:

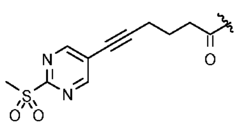 , 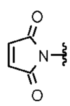 , 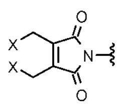 , 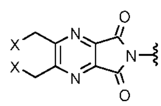 , 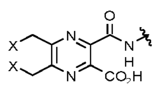 , and 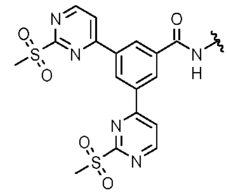 ,

wherein, X is selected from leaving groups, such as chlorine, bromine, -OMs, OTs, and OTf;
preferably, M is selected from the group consisting of the following structures:

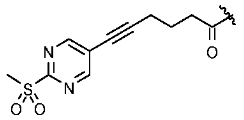 and 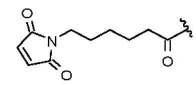 ;

preferably, L is a structure constituted of one or more selected from the group consisting of: $C_{1-6}$ alkylene, -N(R')-, carbonyl, -O-, Val, Cit, Phe, Lys, D-Val, Leu, Gly, Ala, Asn, Val-Cit, Val-Ala, Val-Lys, Val-Lys(Ac), Phe-Lys, Phe-Lys(Ac), D-Val-Leu-Lys, Gly-Gly-Arg, Ala-Ala-Asn, Ala-Ala-Ala, Val-Lys-Ala, Val-Lys-Gly, Gly-Gly-Gly, Gly-Gly-Phe-Gly, Gly-Gly-Gly-Gly-Gly,

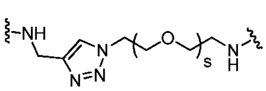 , 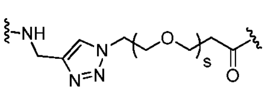 , 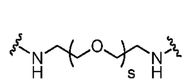 , 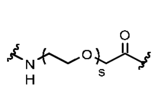 , 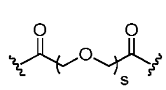 ,

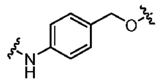 , 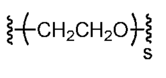 , 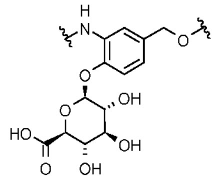 , and 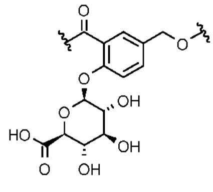 ;

wherein R' represents hydrogen, $C_{1-6}$ alkyl or an alkyl containing $-(CH_2CH_2O)_r-$; r is an integer selected from 1 to 10; s is an integer selected from 1 to 20;

preferably, L is selected from the group consisting of the following structures:

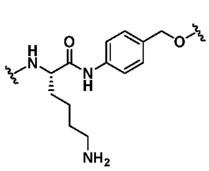
,
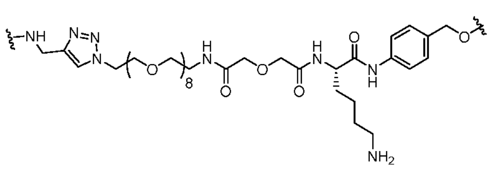
,
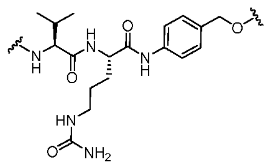
,

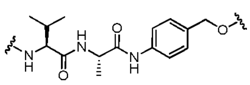
,,
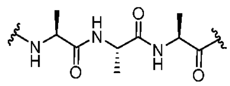
,
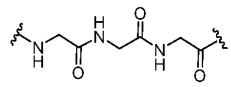
,
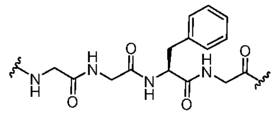
,

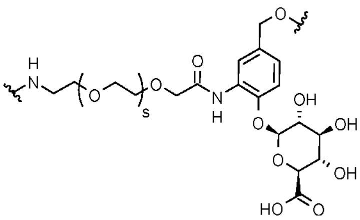
, and
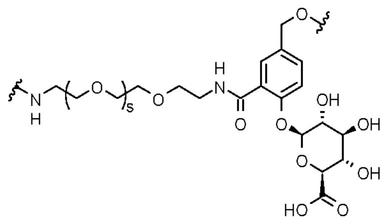
;

wherein s is an integer selected from 1 to 20;
preferably, E is selected from the group consisting of a single bond, $-NH-CH_2-$,

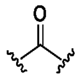
,

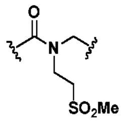
,
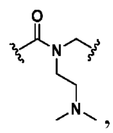
, and
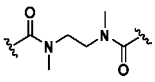
;

preferably, E is $-NH-CH_2-$;
preferably, the cytotoxic drug is selected from the compound according to any one of claims 1 to 5;
preferably, the cytotoxic drug is selected from the compound according to claim 5; preferably, D is selected from the structure formed by removing a hydrogen atom from the compound according to any one of claims 1 to 5;
preferably, D is selected from the structure formed by removing a hydrogen atom from the compound according to claim 5;
preferably, D is selected from the group consisting of the following structures:

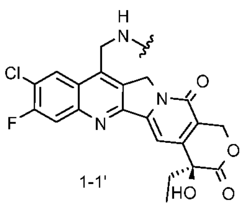
1-1'

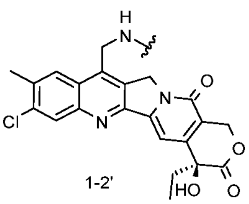
1-2'

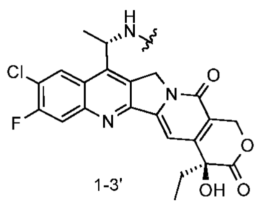
1-3'

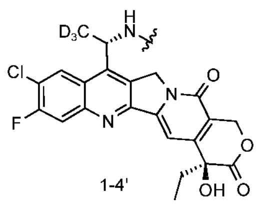
1-4'

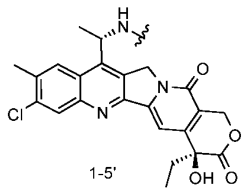
1-5'

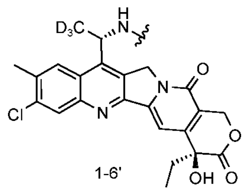
1-6'

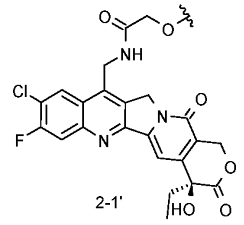
2-1'

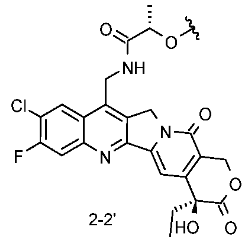
2-2'

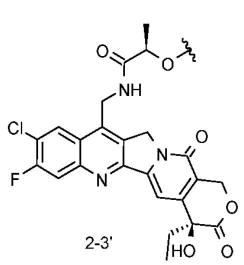
2-3'

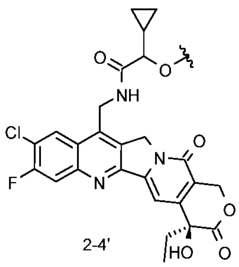
2-4'

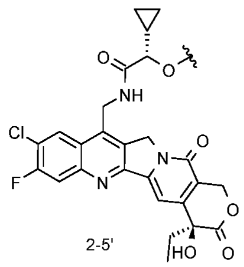
2-5'

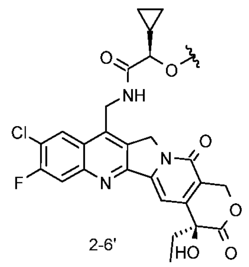
2-6'

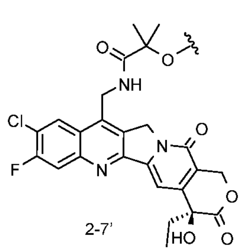
2-7'

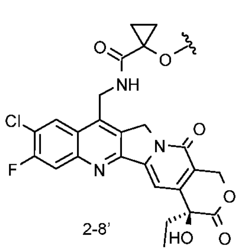
2-8'

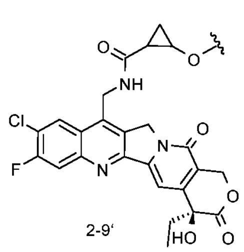
2-9'

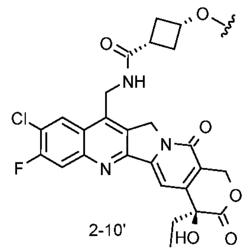
2-10'

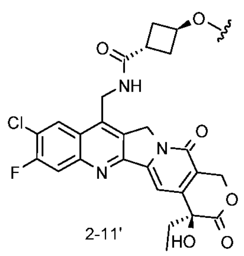
2-11'

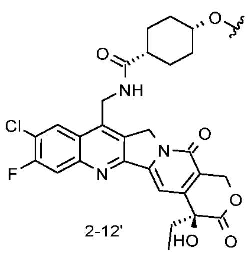
2-12'

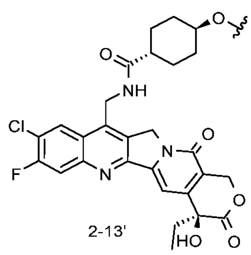
2-13'

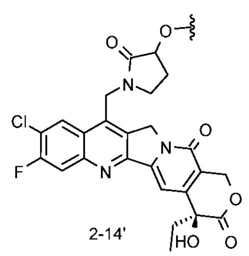
2-14'

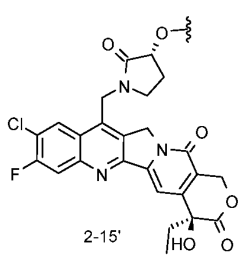
2-15'

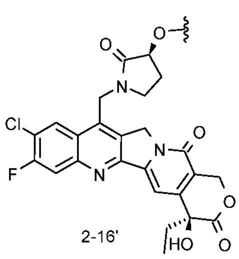
2-16'

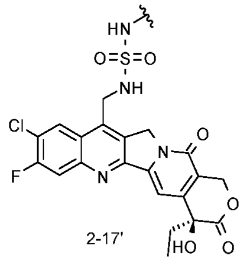
2-17'

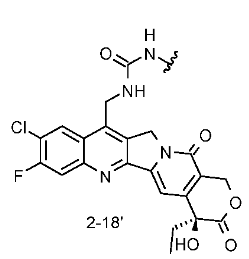
2-18'

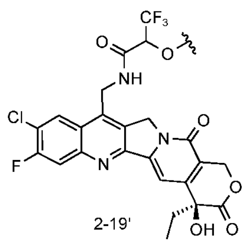
2-19'

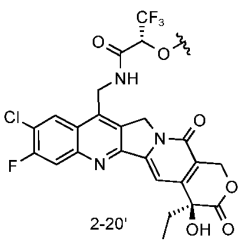
2-20'

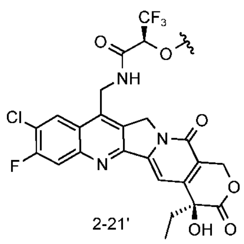
2-21'

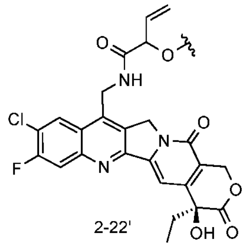
2-22'

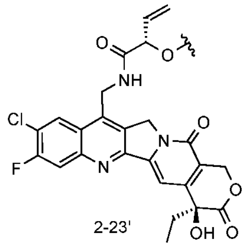
2-23'

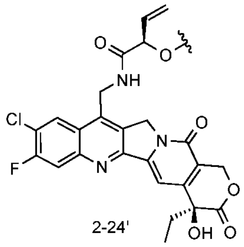
2-24'

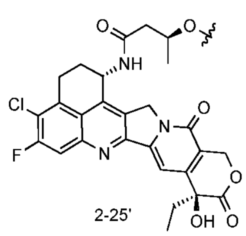
2-25'

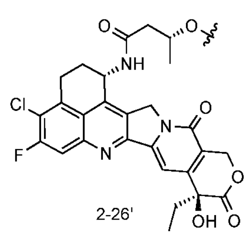
2-26'

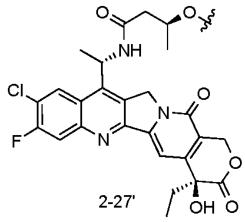
2-27'

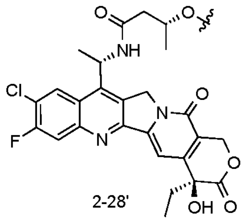
2-28'

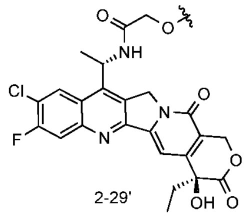
2-29'

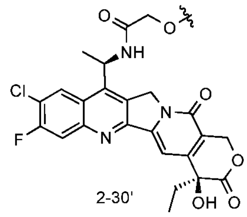
2-30'

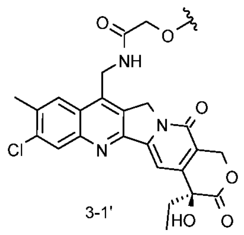
3-1'

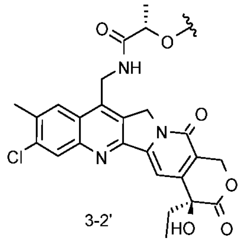
3-2'

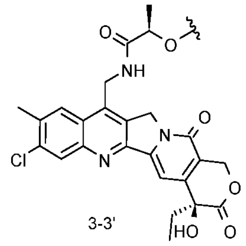
3-3'

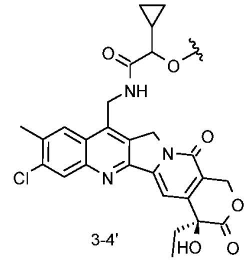
3-4'

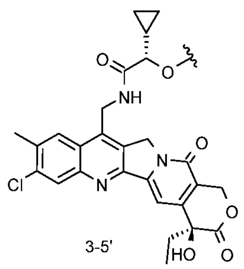
3-5'

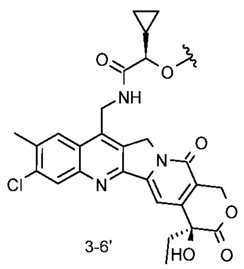
3-6'

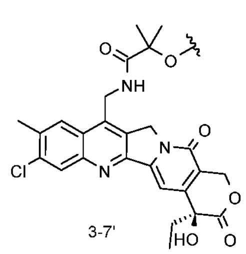
3-7'

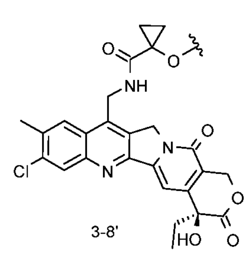
3-8'

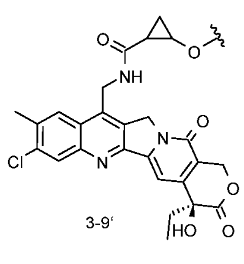
3-9'

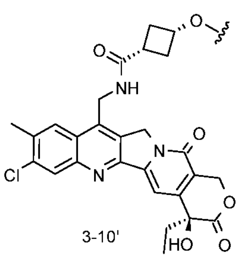
3-10'

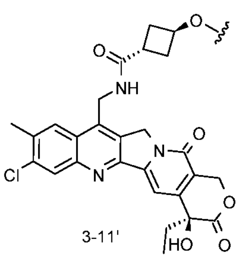
3-11'

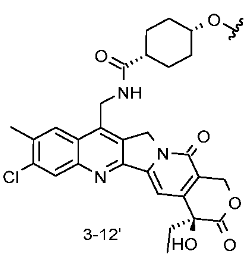
3-12'

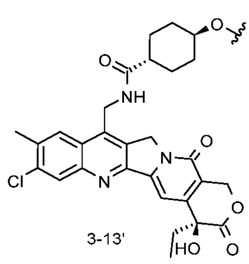
3-13'

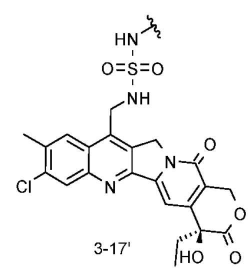
3-17'

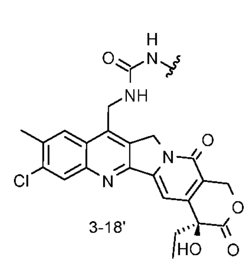
3-18'

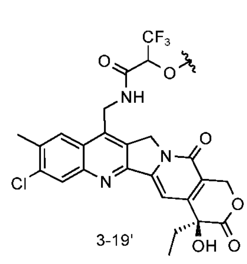
3-19'

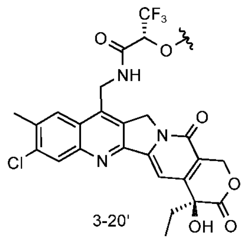
3-20'

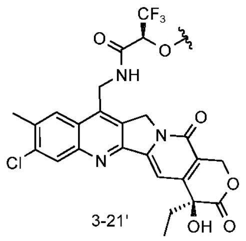
3-21'

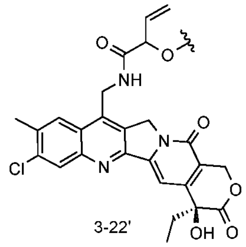
3-22'

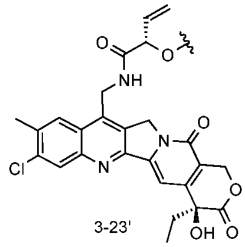
3-23'

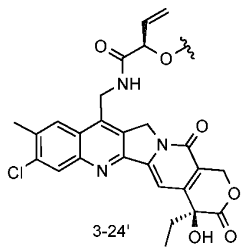
3-24'

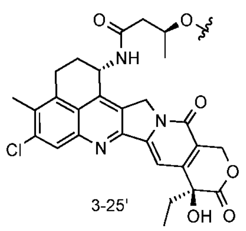
3-25'

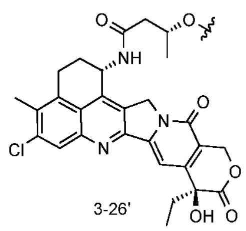
3-26'

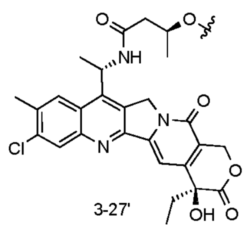
3-27'

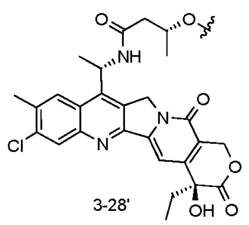
3-28'

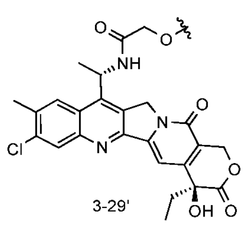
3-29'

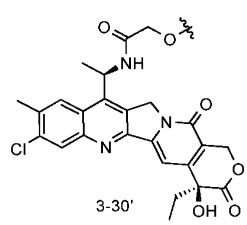
3-30'

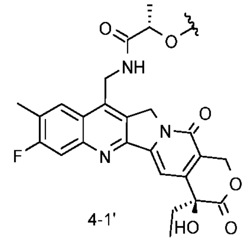
4-1'

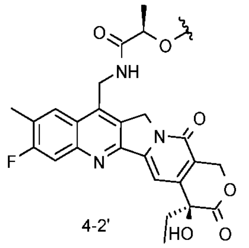
4-2'

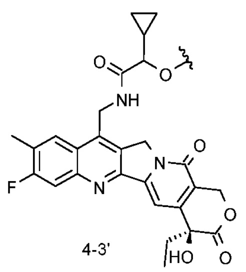
4-3'

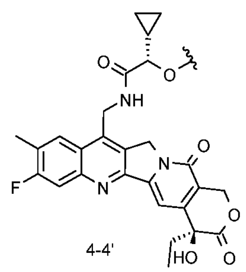
4-4'

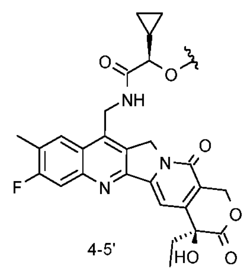
4-5'

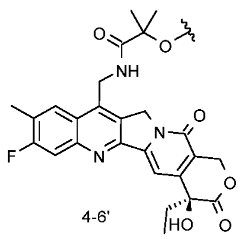
4-6'

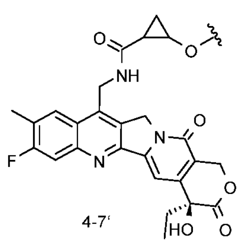
4-7'

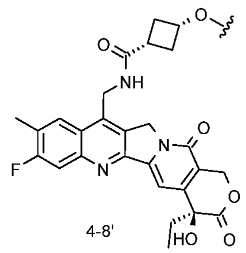
4-8'

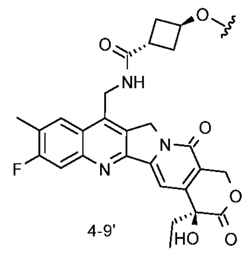
4-9'

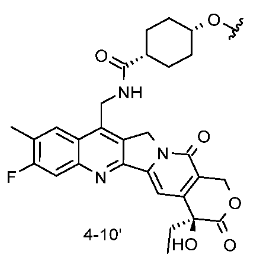
4-10'

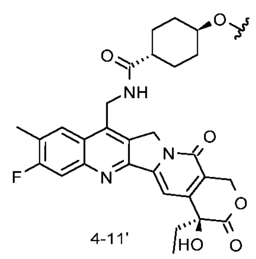
4-11'

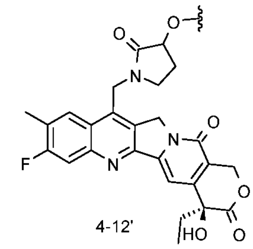
4-12'

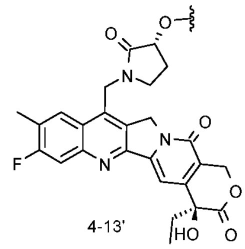
4-13'

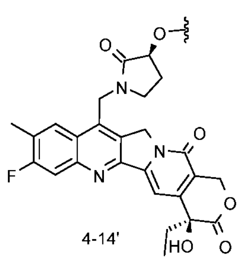
4-14'

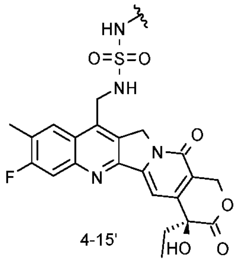
4-15'

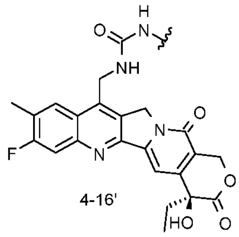
4-16'

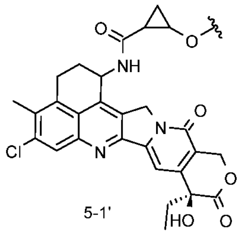
5-1'

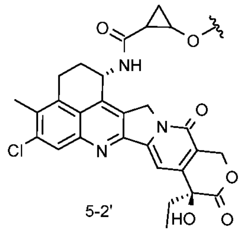
5-2'

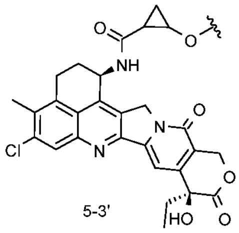
5-3'

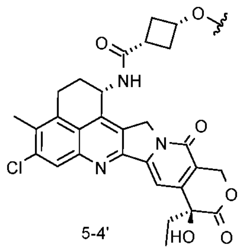
5-4'

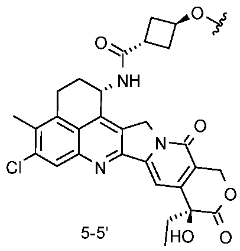
5-5'

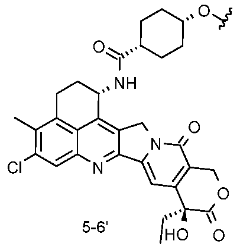
5-6'

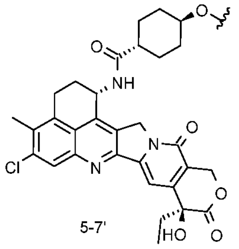
5-7'

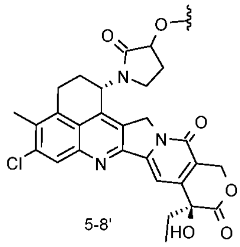
5-8'

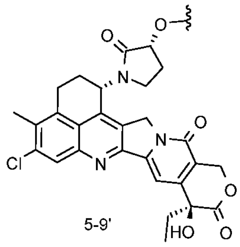
5-9'

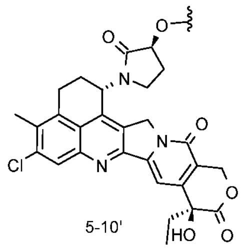
5-10'

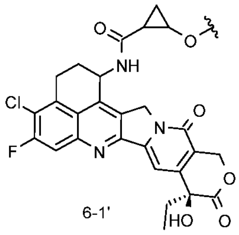
6-1'

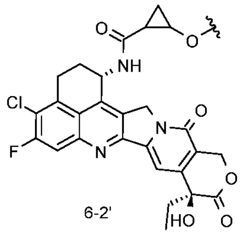
6-2'

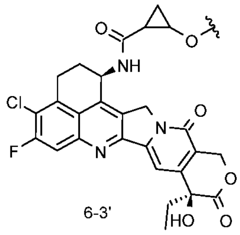
6-3'

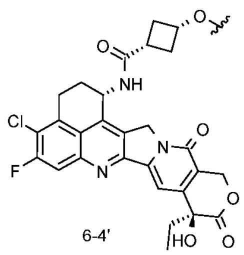
6-4'

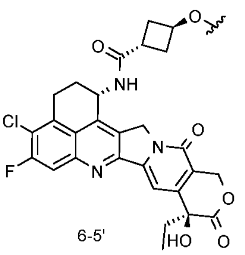
6-5'

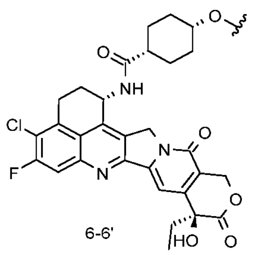
6-6'

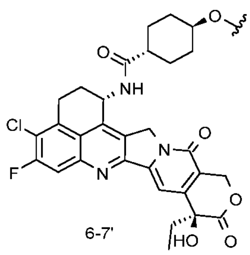
6-7'

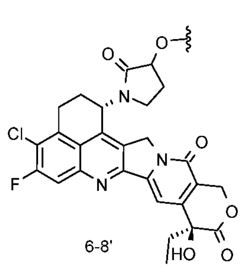
6-8'

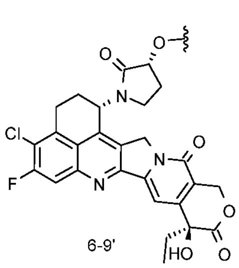
6-9'

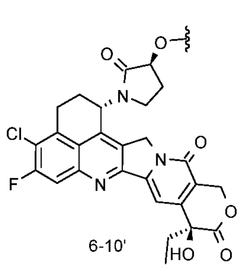
6-10'

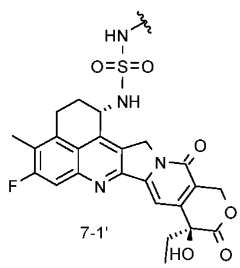
7-1'

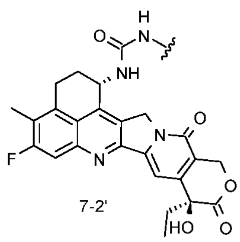
7-2'

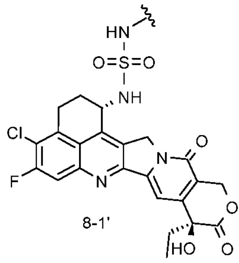
8-1'

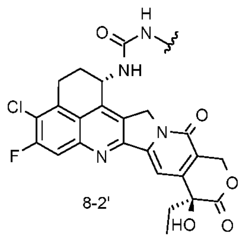
8-2'

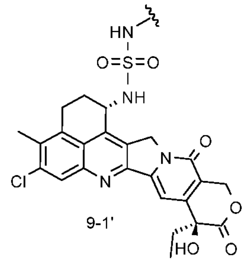
9-1'

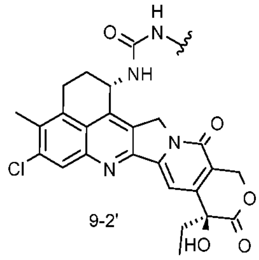
9-2' ;

preferably, D is selected from the group consisting of the following structures:

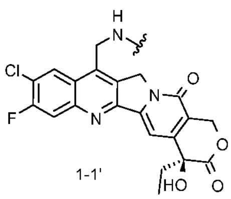
1-1'

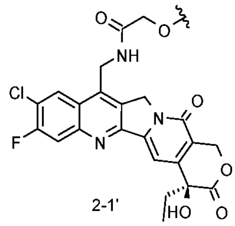
2-1'

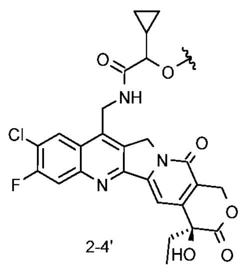
2-4'

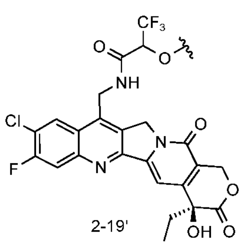
2-19'

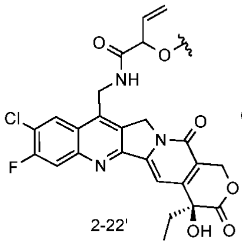
2-22'

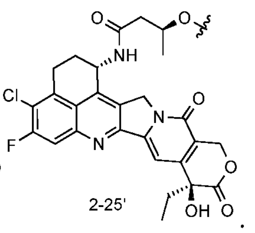
2-25' .

**7.** The compound or pharmaceutically acceptable salt, ester, stereoisomer, polymorph, solvate, N-oxide, isotopically labeled compound, metabolite and prodrug thereof according to claim 6, wherein the compound has the structure as follows:

A-1:

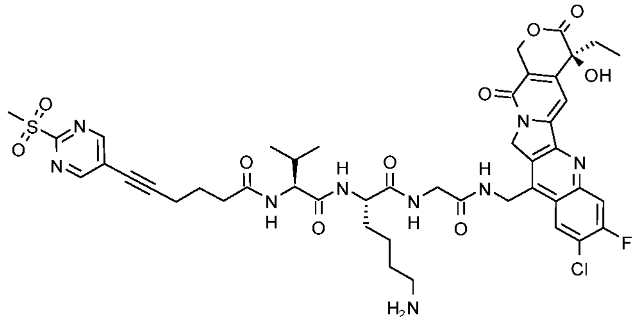

A-2:

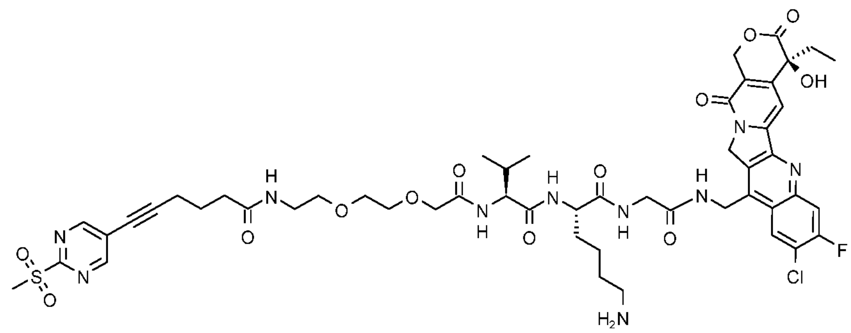

A-3:

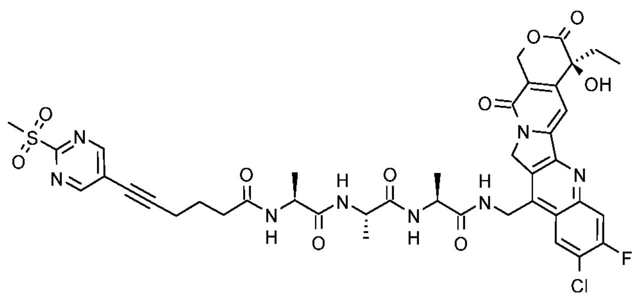

A-4:

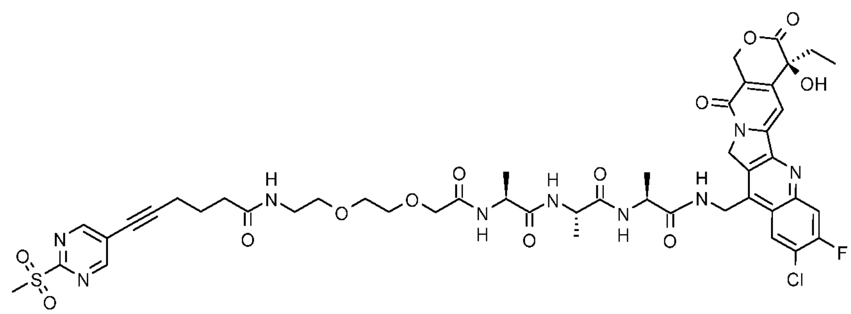

A-5:

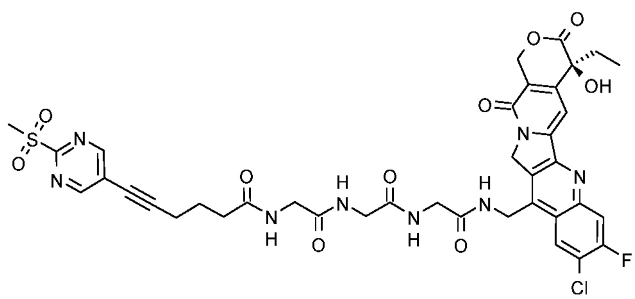

A-6:

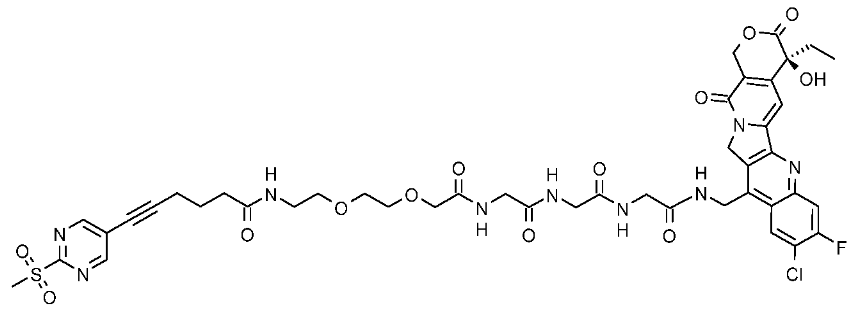

A-7:

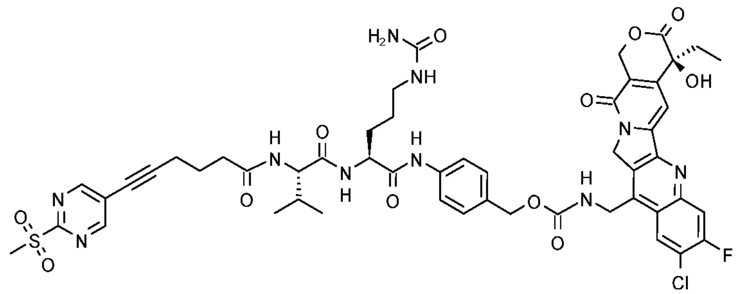

A-8:

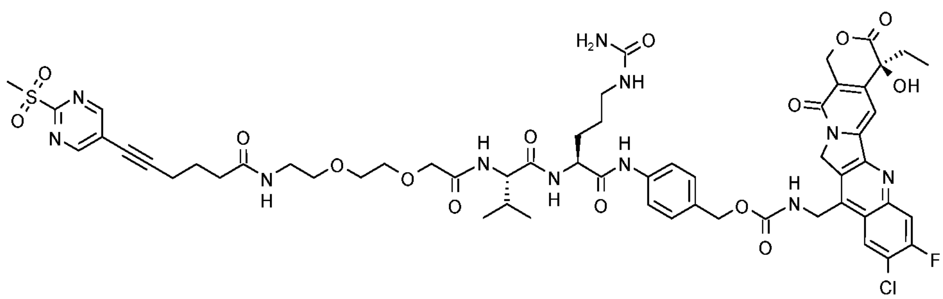

A-9:

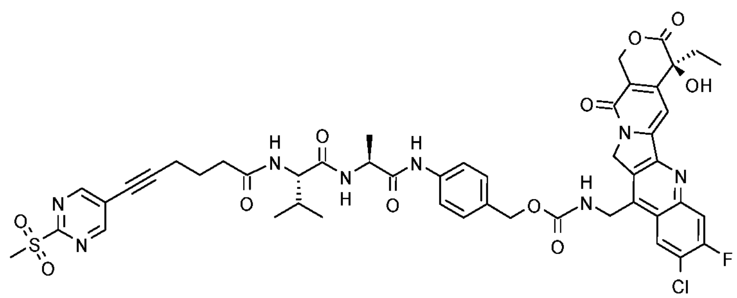

A-10:

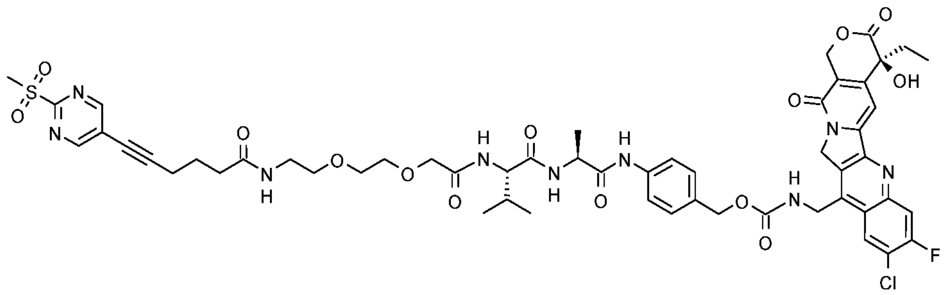

A-11:

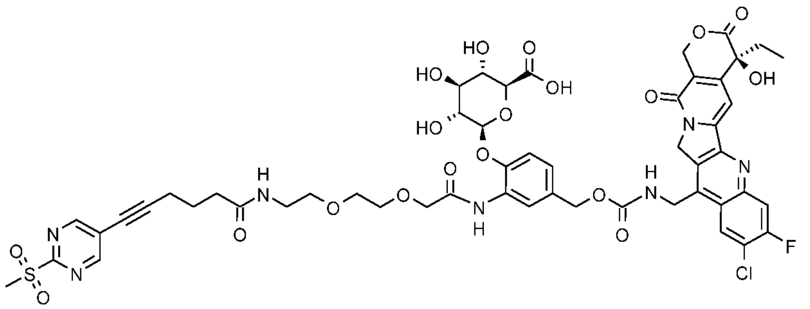

A-12:

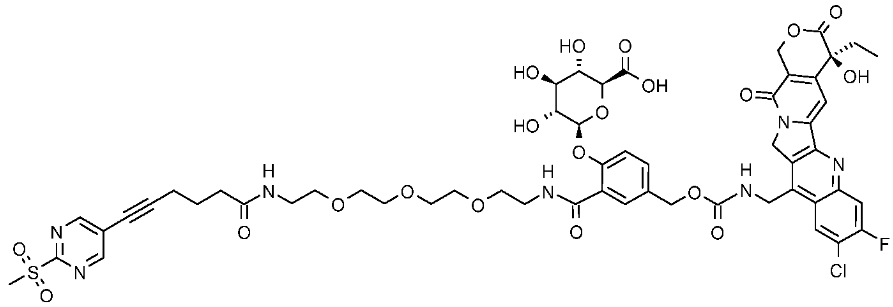

B-1:

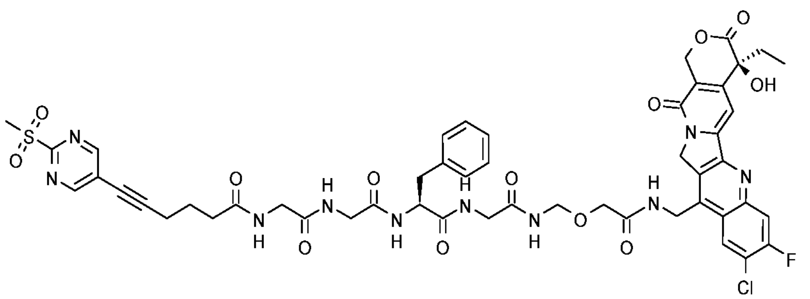

B-2:

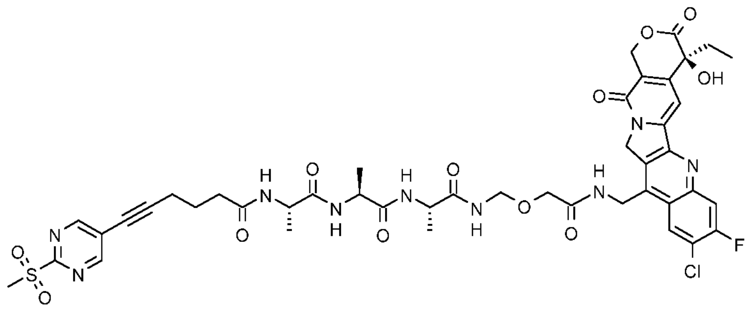

B-3:

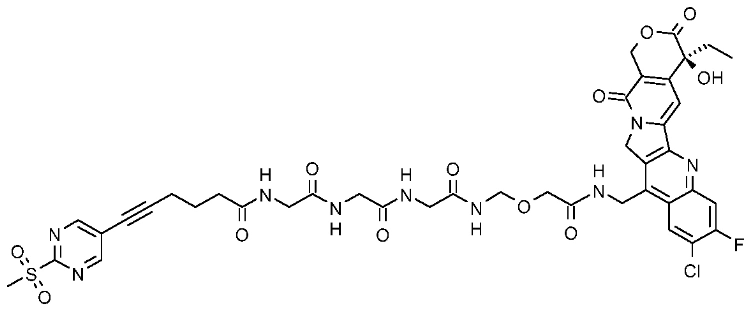

B-4:

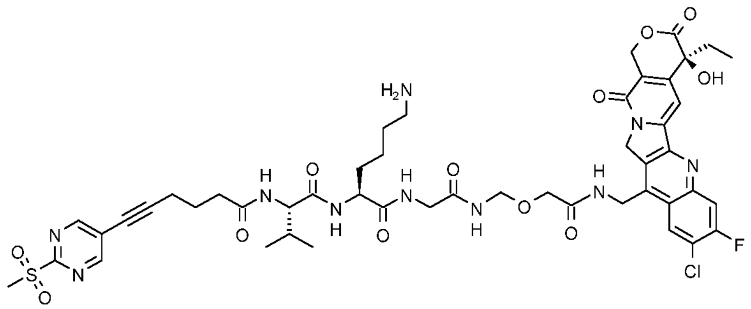

B-5:

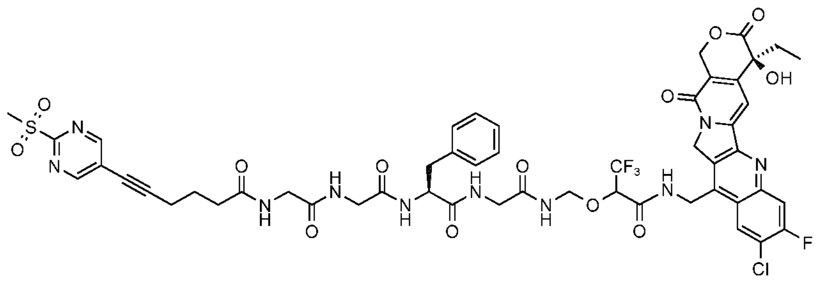

B-6:

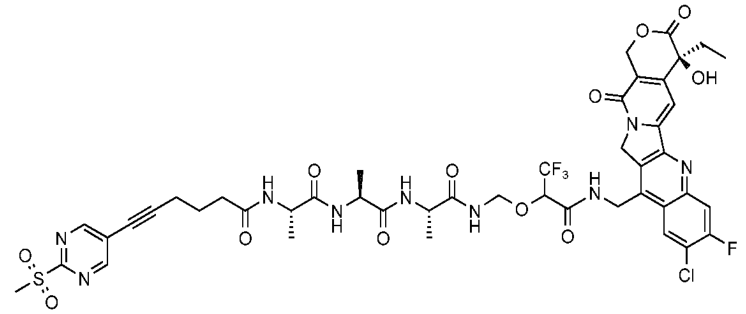

B-7:

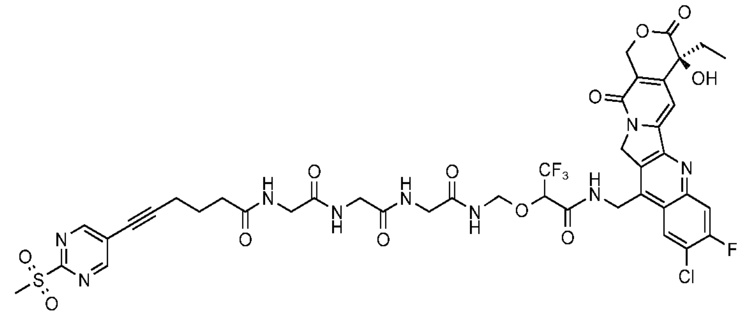

B-8:

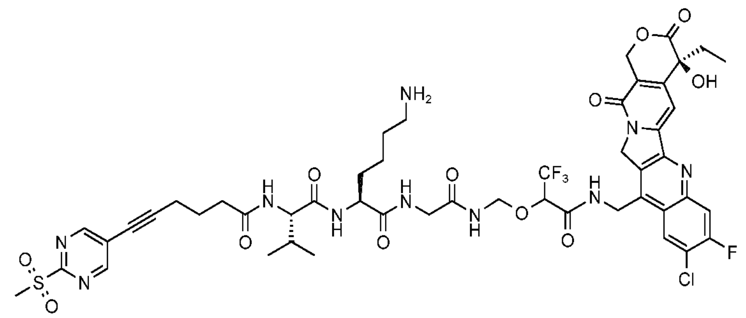

B-9:

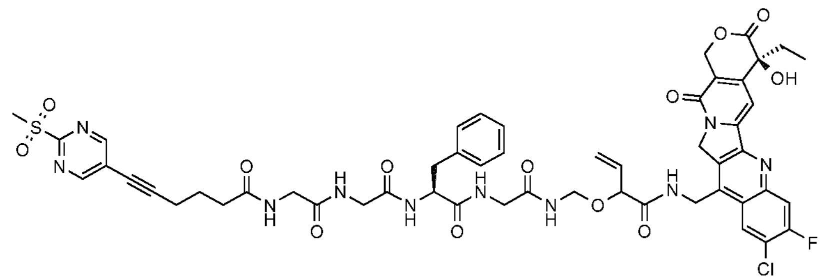

B-10:

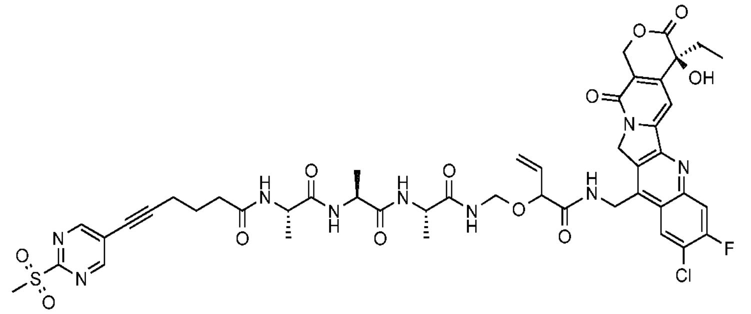

B-11:

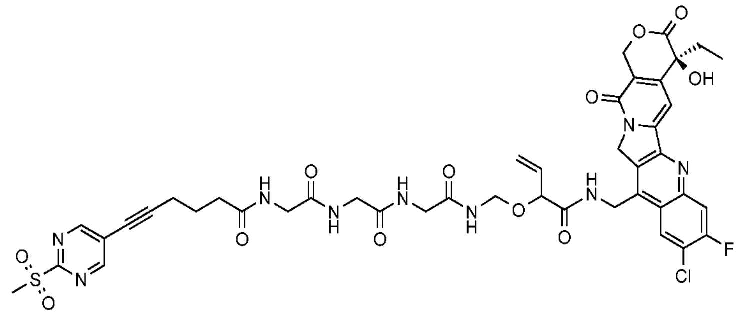

B-12:

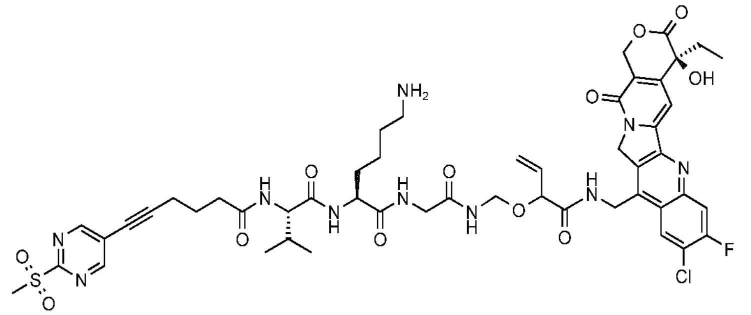

C-1:

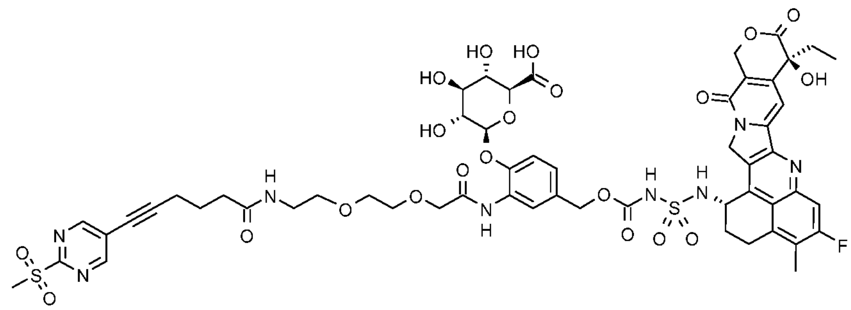

C-2:

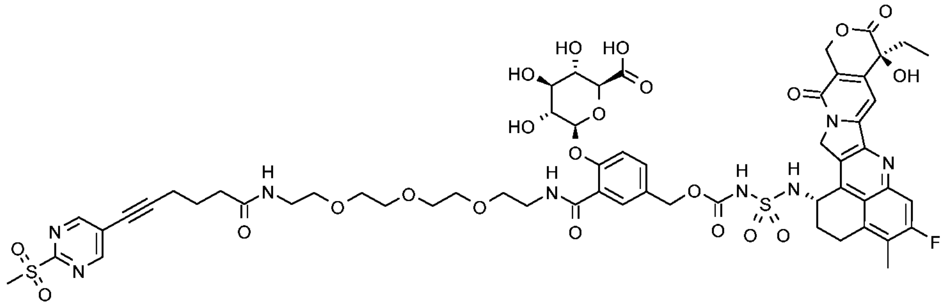

C-3:

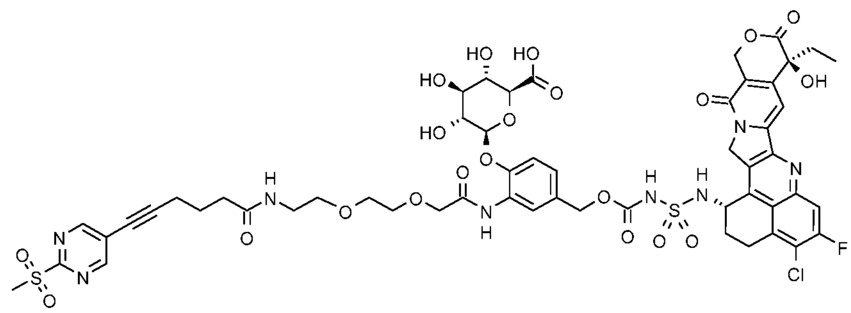

C-4:

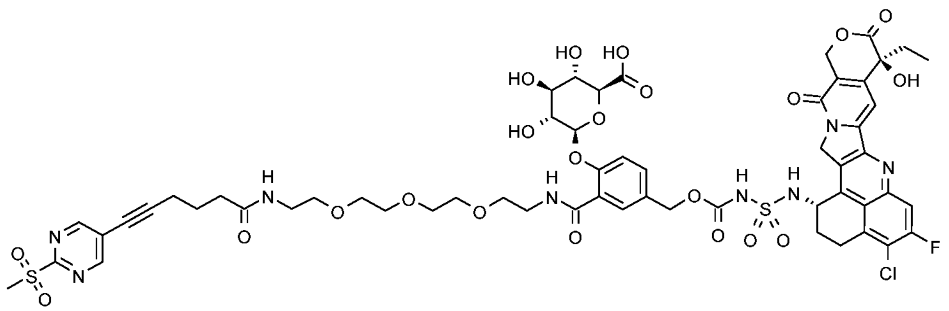

C-5:

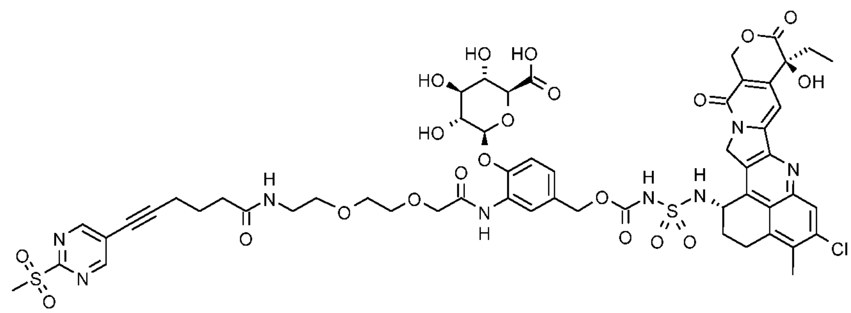

C-6:

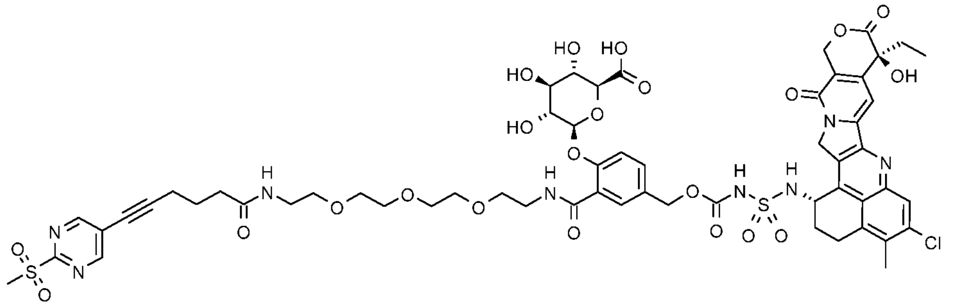

C-7:

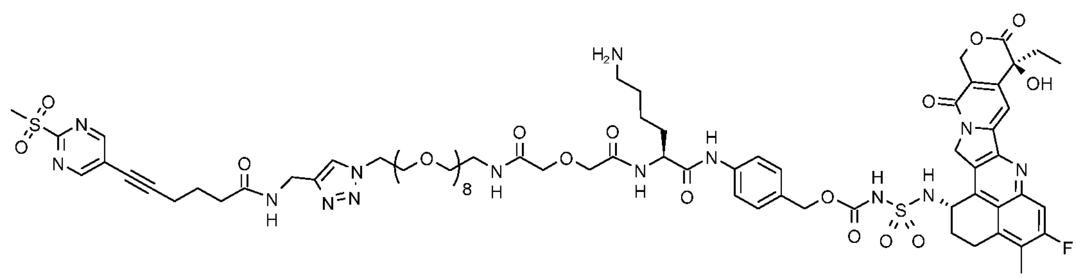

C-8:

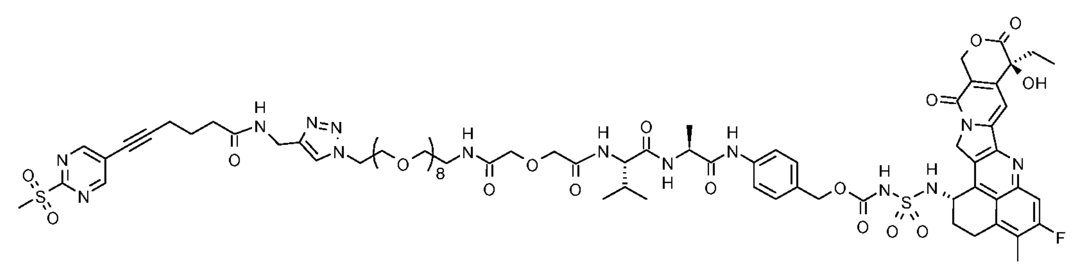

C-9:

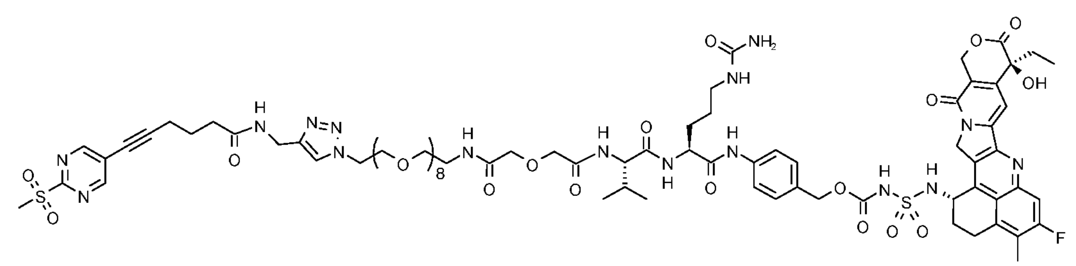

C-10:

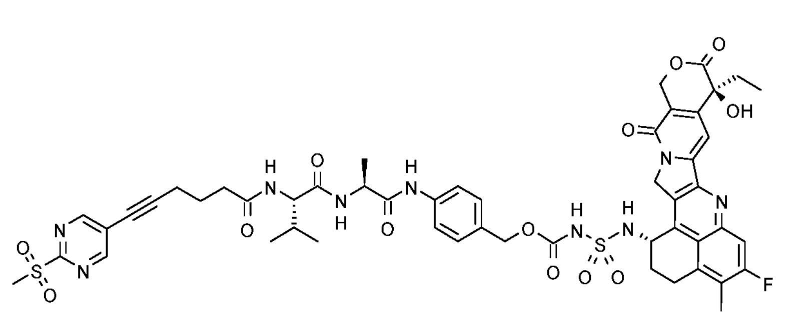

C-11:

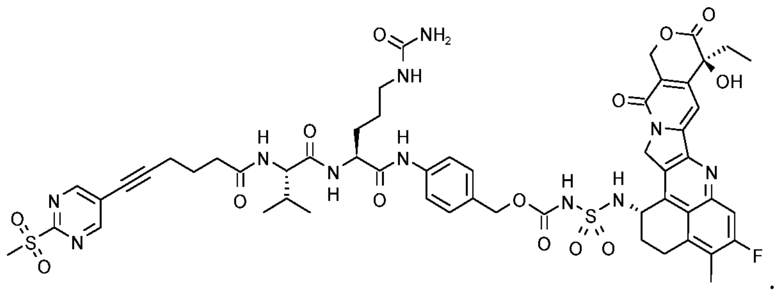

**8.** An antibody-drug conjugate (ADC) represented by Formula (VI):

Ab-(M-L-E-D)$_x$ Formula (VI)

wherein,

Ab represents an antibody;
M represents a linking site connecting the antibody or an antigen-binding fragment thereof;
L represents a linker connecting the linking site M and E;
E represents a structural fragment connecting L and D;
D represents a structural fragment of a cytotoxic drug;
x is 1 to 10;
M, L, E and D are as described in any one of claims 1 to 7;
preferably, Ab-(M-L-E-D)$_x$ is selected from the group consisting of the following structures:

ADC A-1:

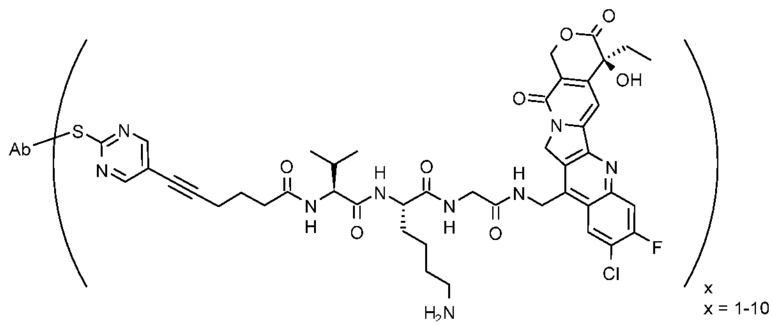

ADC A-2:

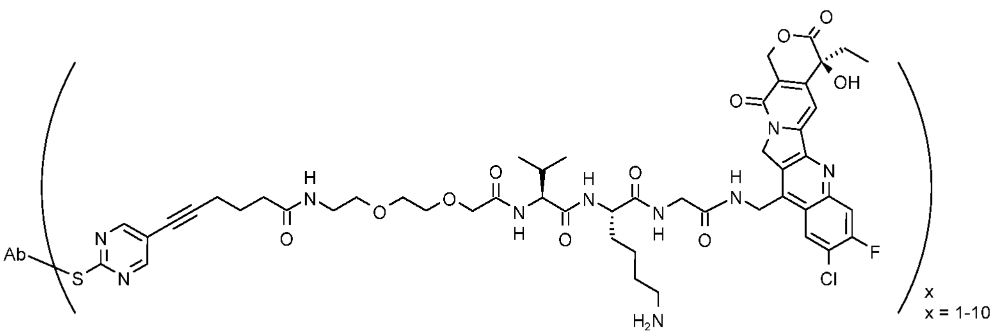

ADC A-3:

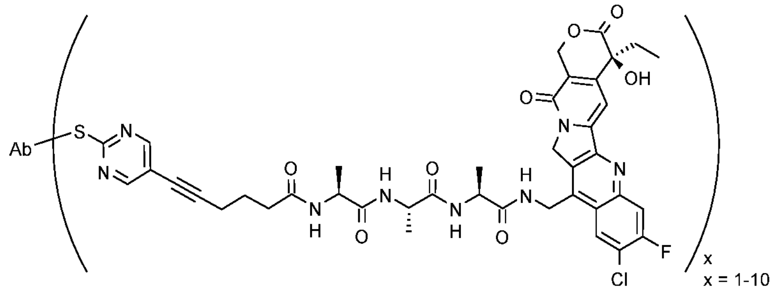

ADC A-4:

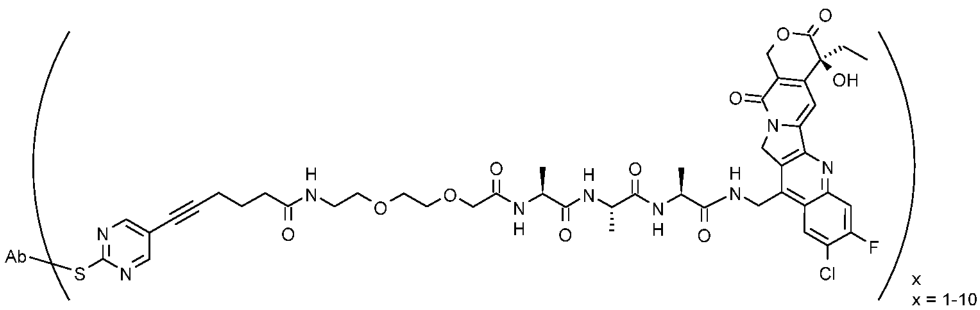

ADC A-5:

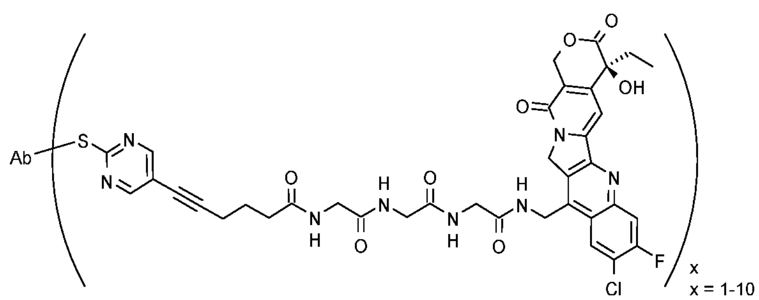

ADC A-6:

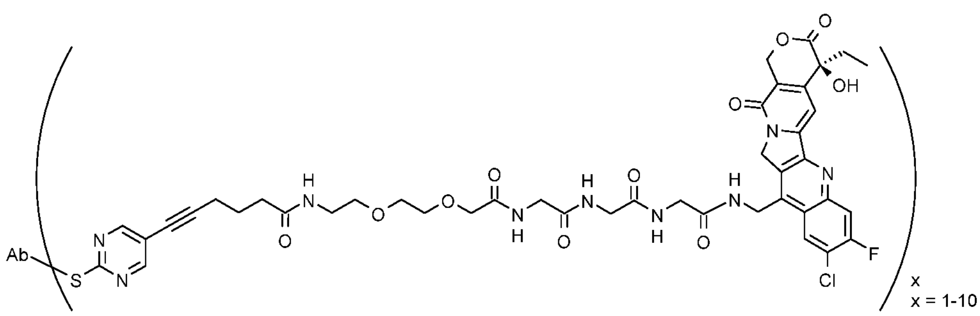

ADC A-7:

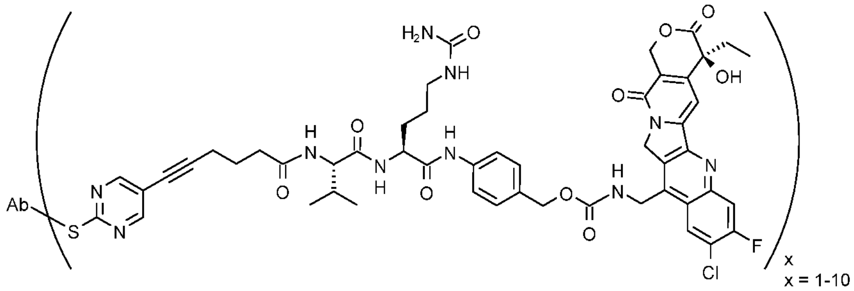

ADC A-8:

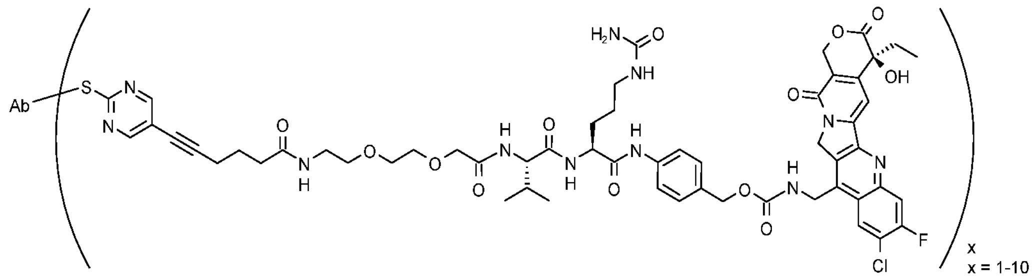

ADC A-9:

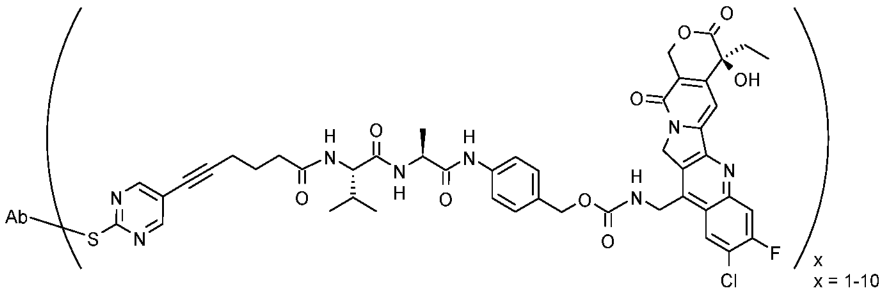

ADC A-10:

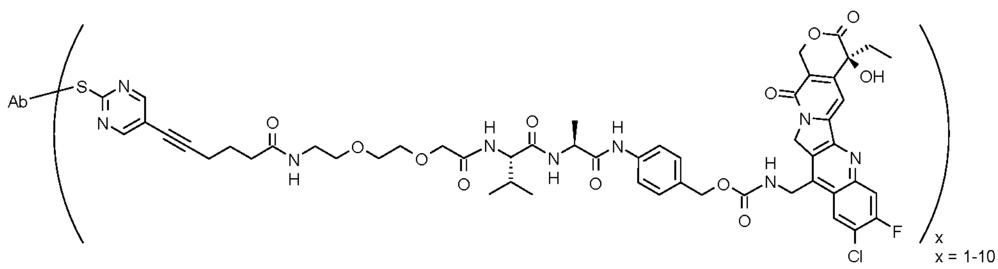

ADC A-11:

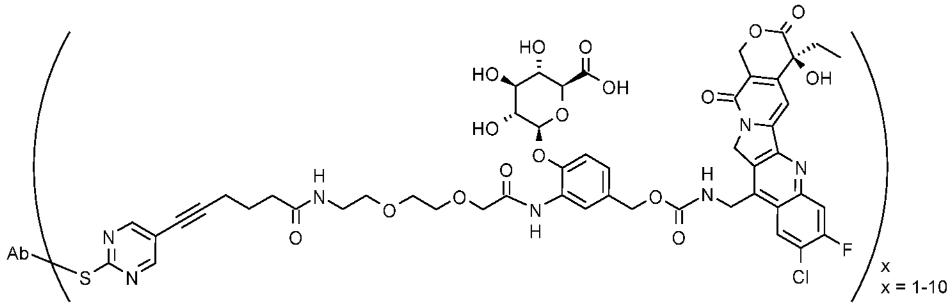

ADC A-12:

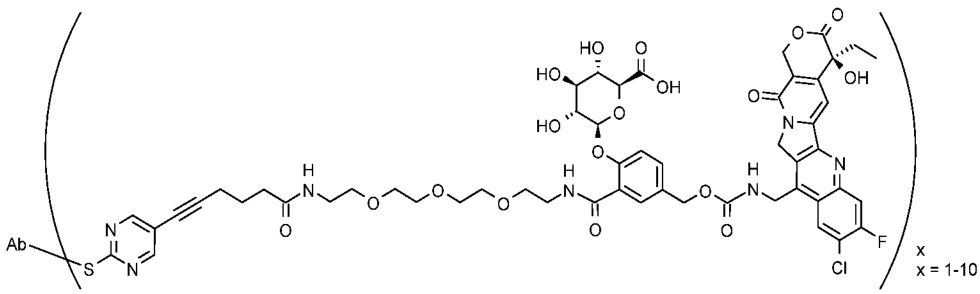

ADC B-1:

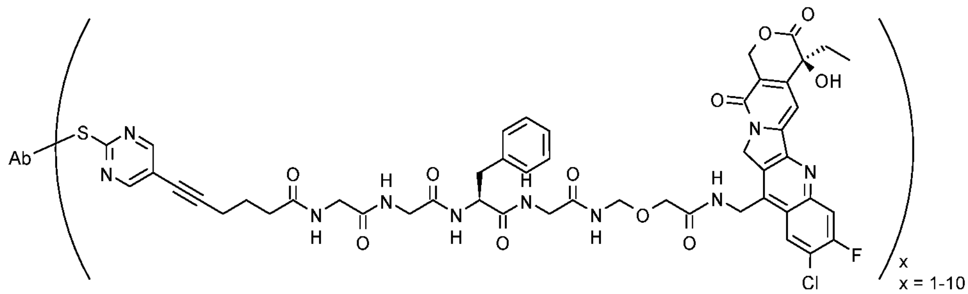

ADC B-2:

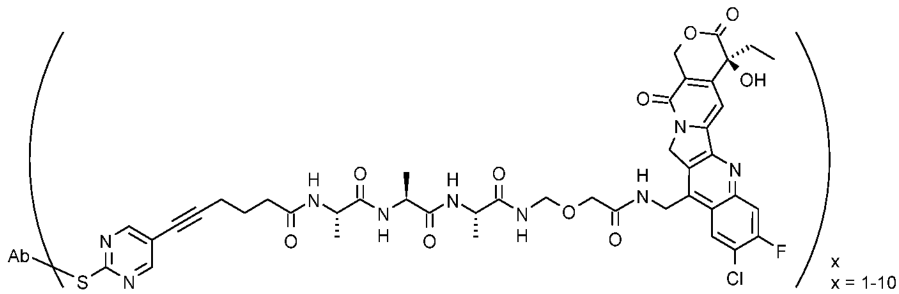

ADC B-3:

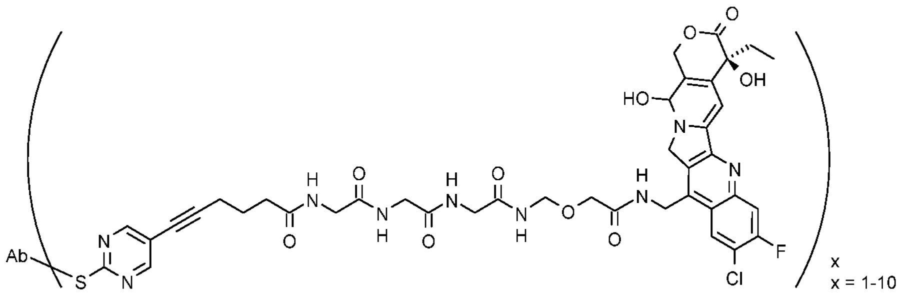

ADC B-4:

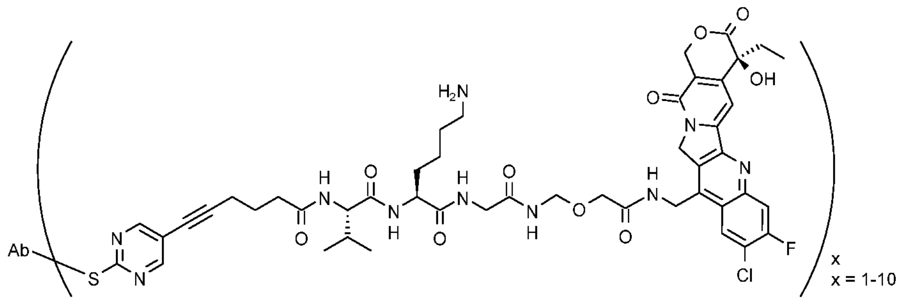

ADC B-5:

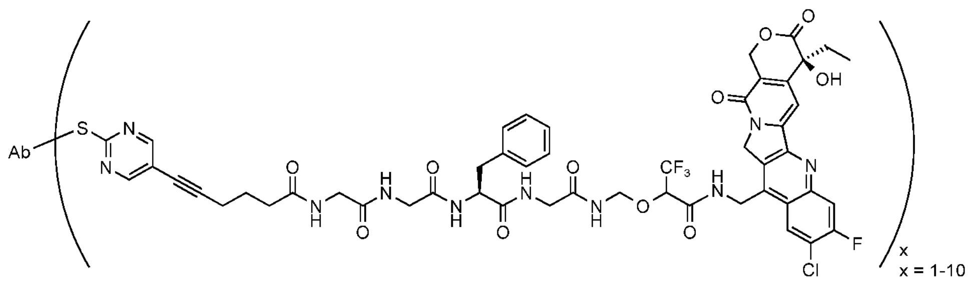

ADC B-6:

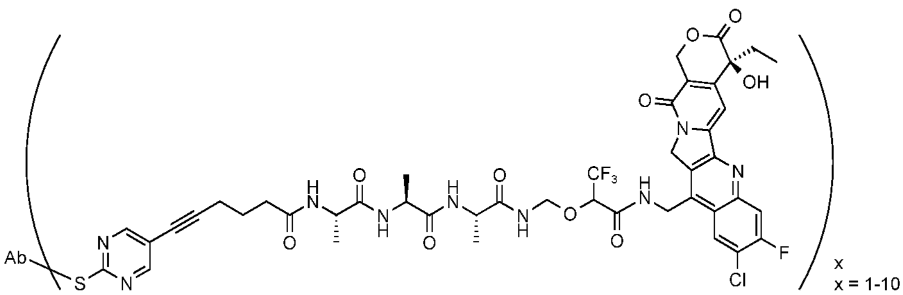

ADC B-7:

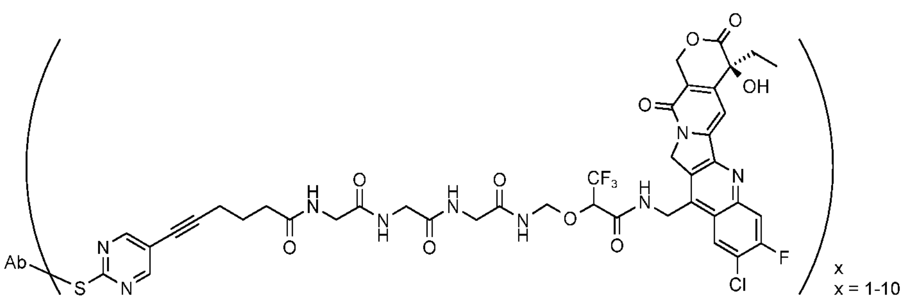

ADC B-8:

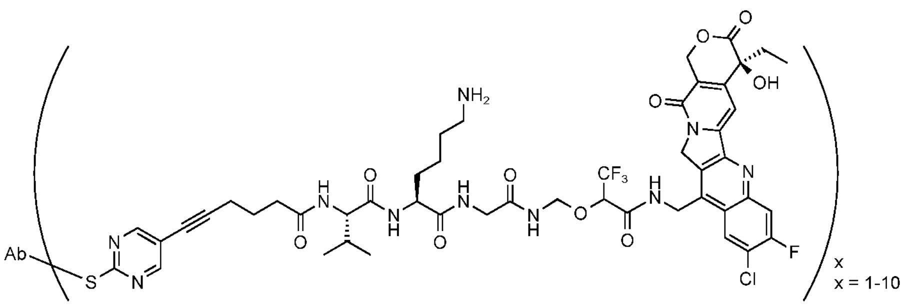

ADC B-9:

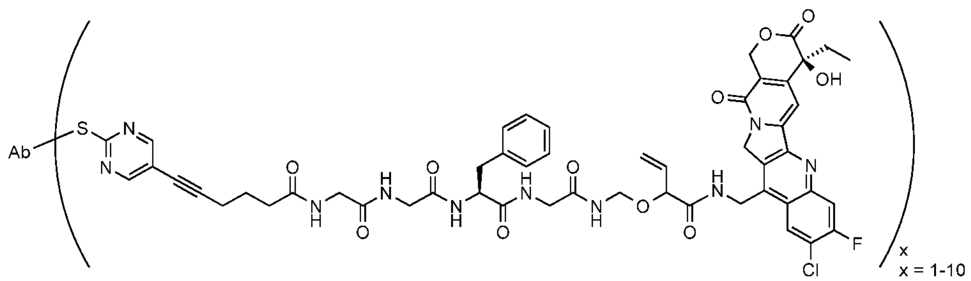

ADC B-10:

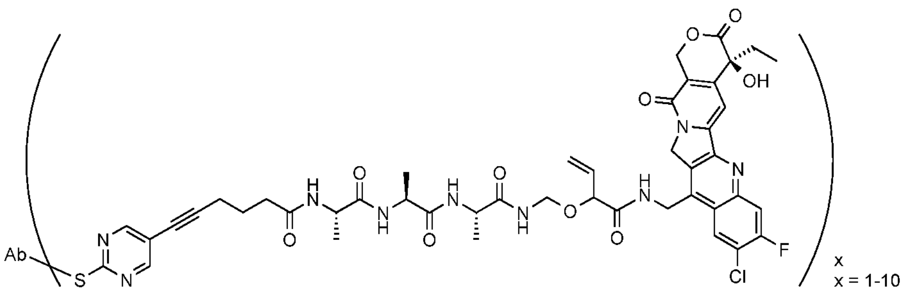

ADC B-11:

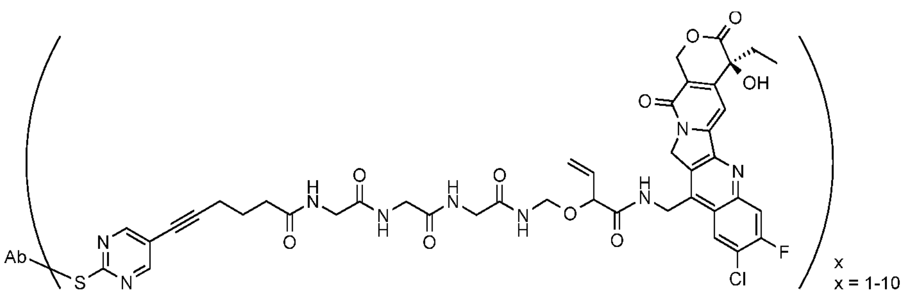

ADC B-12:

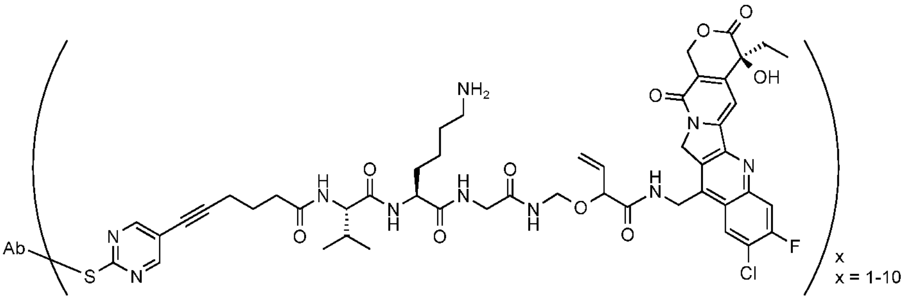

ADC C-1:

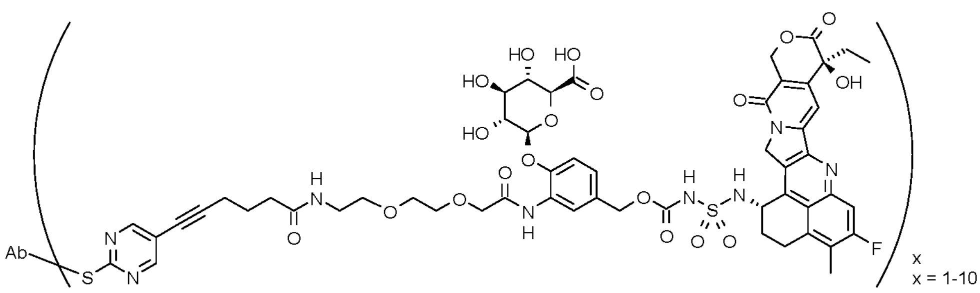

ADC C-2:

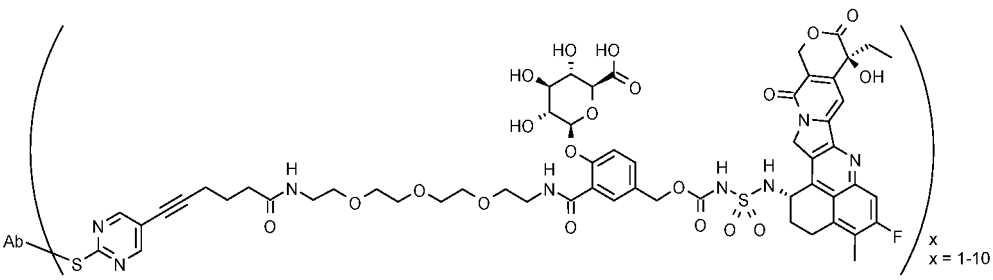

ADC C-3:

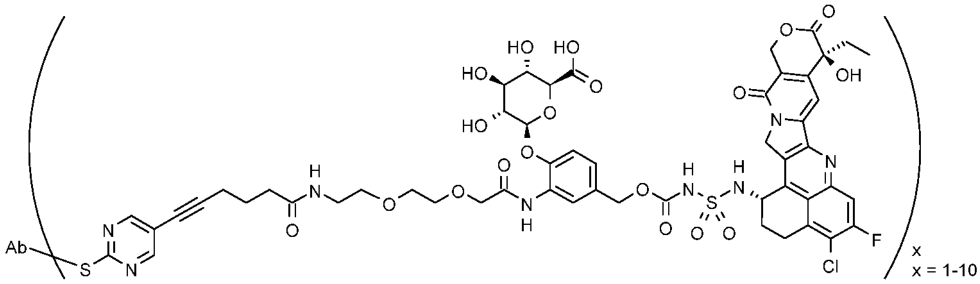

ADC C-4:

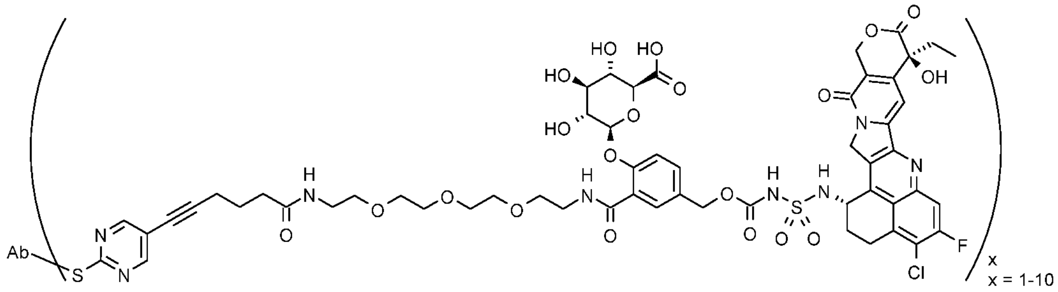

ADC C-5:

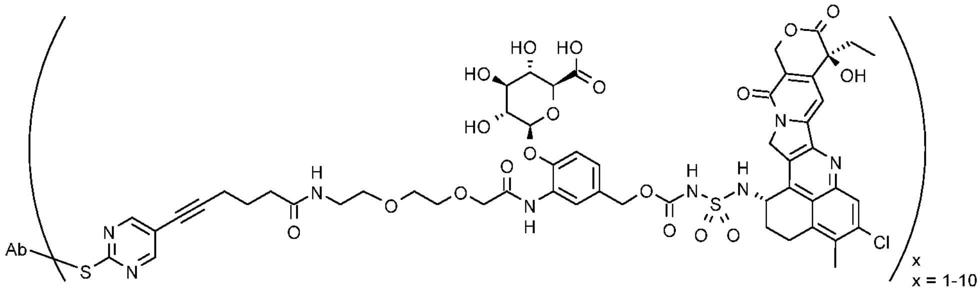

ADC C-6:

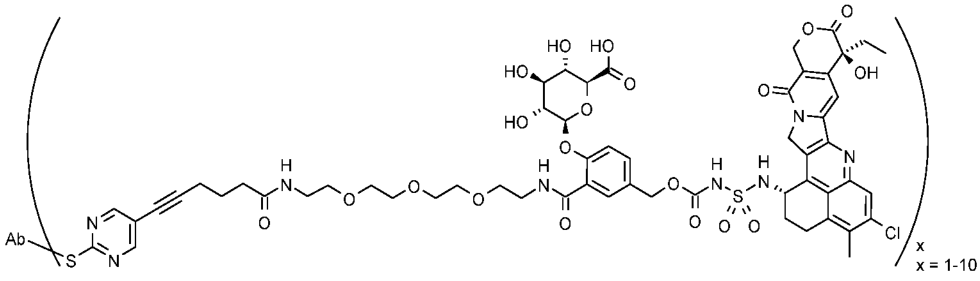

ADC C-7:

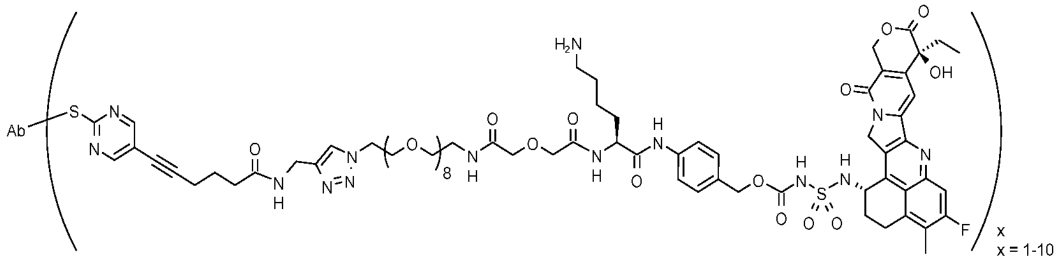

ADC C-8:

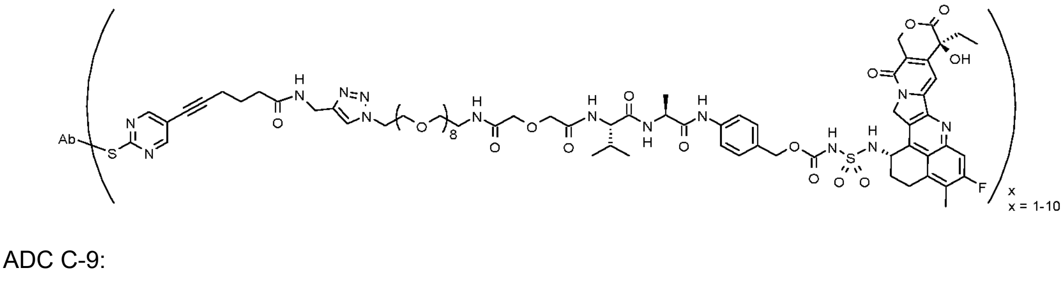

ADC C-9:

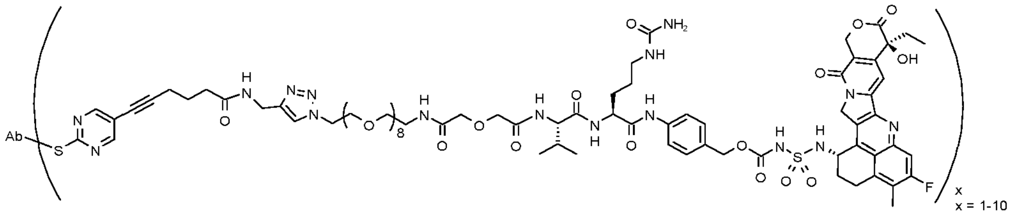

ADC C-10:

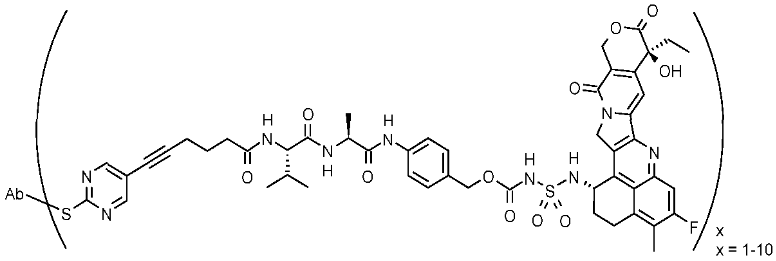

ADC C-11:

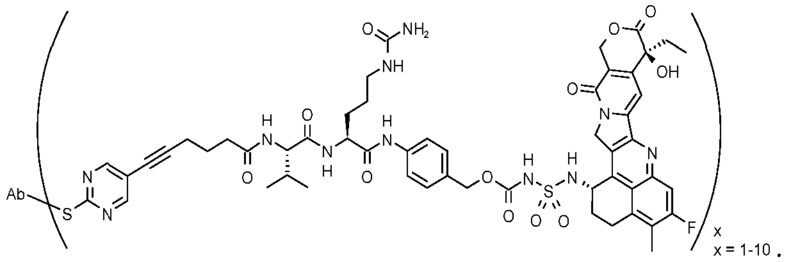

**9.** An antibody-drug conjugate (ADC), which has the structure as follows:

Trastuzumab A-11:

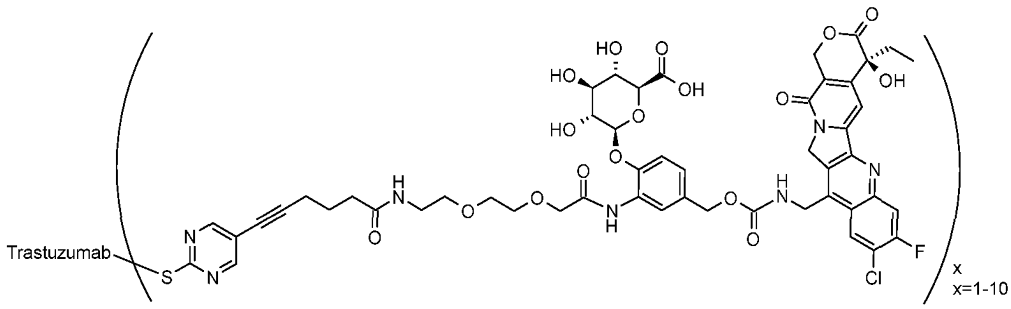

Trastuzumab B-1:

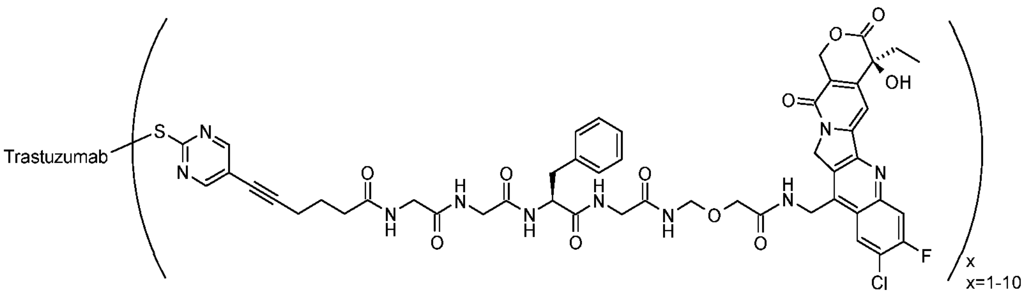

Trastuzumab B-2:

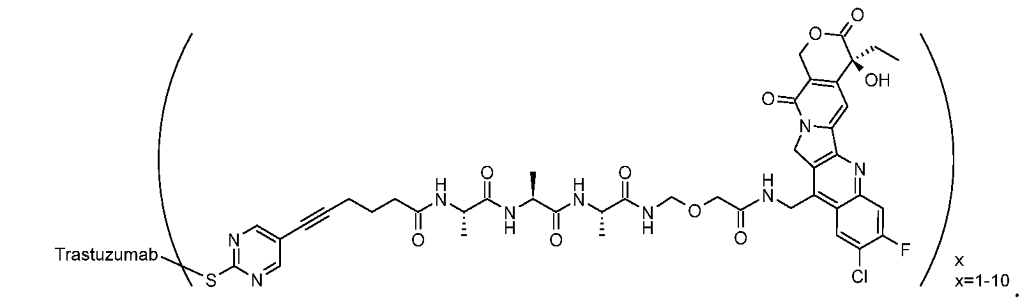

10. A composition comprising one or more of the following antibody-drug conjugates, wherein the composition has a DAR value (drug-antibody ratio) of 7.5 to 8.5, preferably 7.5 to 8.0, further preferably 8.0:
Trastuzumab A-11:

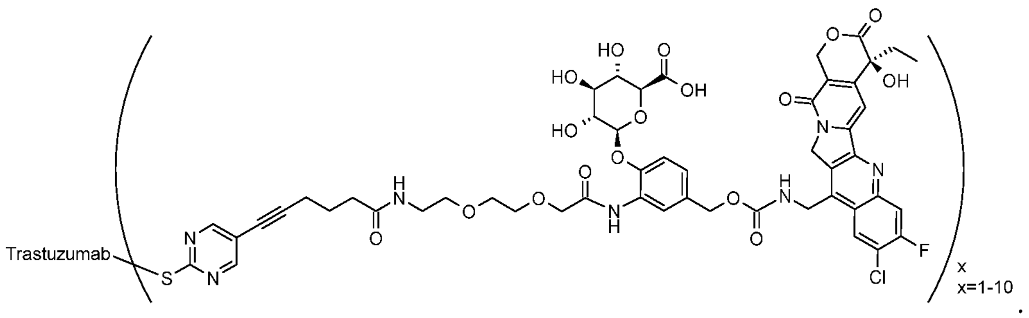

11. A composition comprising one or more of the following antibody-drug conjugates, wherein the composition has a DAR value (drug-antibody ratio) of 7.5 to 8.5, preferably 7.5 to 8.0, further preferably 7.96:
Trastuzumab B-1:

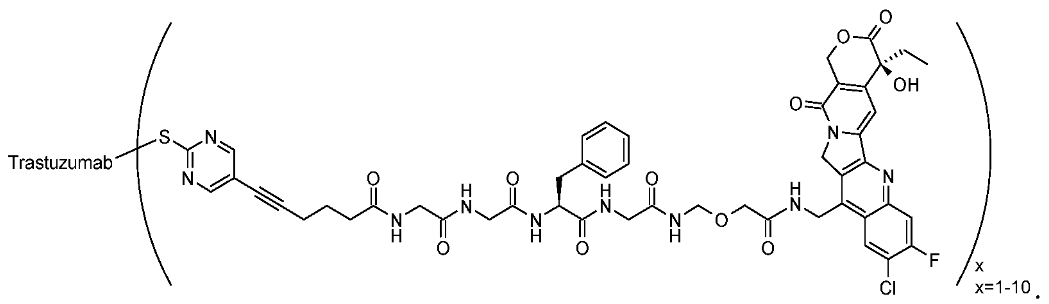

12. A composition comprising one or more of the following antibody-drug conjugates, wherein the composition has a DAR value (drug-antibody ratio) of 7.5 to 8.5, preferably 7.5 to 8.0, further preferably 7.95:
Trastuzumab B-2:

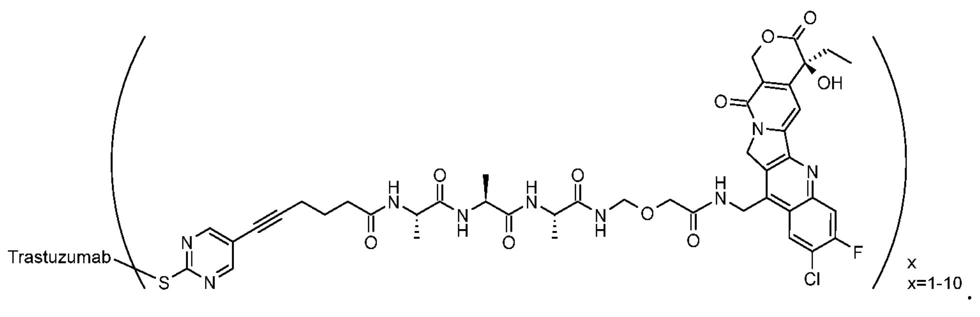

**13.** A pharmaceutical composition, which comprises the compound or pharmaceutically acceptable salt, ester, stereoisomer, polymorph, solvate, N-oxide, isotopically labeled compound, metabolite or prodrug thereof according to any one of claims 1 to 7, or the antibody-drug conjugate according to any one of claims 8 to 9, or the composition according to any one of claims 10 to 12, and one or more pharmaceutically acceptable carriers.

**14.** A kit, which comprises:

a) a first therapeutic agent, which is at least one of the compound or pharmaceutically acceptable salt, ester, stereoisomer, polymorph, solvate, N-oxide, isotopically labeled compound, metabolite or prodrug thereof according to any one of claims 1 to 7, the antibody-drug conjugate according to any one of claims 8 to 9, the composition according to any one of claims 10 to 12, or the pharmaceutical composition according to claim 13;
b) an optional second therapeutic agent, which is at least one additional therapeutic agent, or
an optional second pharmaceutical composition, which is a pharmaceutical composition containing an additional therapeutic agent; and
c) an optional packaging and/or instruction.

**15.** Use of the compound or pharmaceutically acceptable salt, ester, stereoisomer, polymorph, solvate, N-oxide, isotopically labeled compound, metabolite and prodrug thereof according to any one of claims 1 to 9, the antibody-drug conjugate according to any one of claims 8 to 9, the composition according to any one of claims 10 to 12, the pharmaceutical composition according to claim 13, or the kit according to claim 14 in the manufacture of a medicament for treating a disease associated with abnormal cell proliferation;

preferably, the disease is a tumor, such as an advanced solid tumor;
preferably, the tumor is selected from the group consisting of lung cancer, squamous cell carcinoma, bladder cancer, gastric cancer, ovarian cancer, peritoneal cancer, pancreatic cancer, breast cancer, head and neck cancer, cervical cancer, endometrial cancer, rectal cancer, liver cancer, kidney cancer, esophageal adenocarcinoma, squamous cell esophageal carcinoma, prostate cancer, female reproductive tract cancer, carcinoma in situ, lymphoma, neurofibroma, thyroid cancer, bone cancer, skin cancer, brain cancer, colon cancer, testicular cancer, gastrointestinal stromal tumor, mast cell tumor, multiple myeloma, melanoma, glioma, or sarcoma.

**16.** A method for treating a disease associated with abnormal cell proliferation, which comprises the following steps: administrating to an individual in need thereof with a therapeutically effective amount of the compound or pharmaceutically acceptable salt, ester, stereoisomer, polymorph, solvate, N-oxide, isotopically labeled compound, metabolite and prodrug thereof according to any one of claims 1 to 9, or the antibody-drug conjugate according to any one of claims 8 to 9, or the composition according to any one of claims 10 to 12, or the pharmaceutical composition according to claim 13, or the kit according to claim 14;

preferably, the disease is a tumor, such as an advanced solid tumor;
preferably, the tumor is selected from the group consisting of lung cancer, squamous cell carcinoma, bladder cancer, gastric cancer, ovarian cancer, peritoneal cancer, pancreatic cancer, breast cancer, head and neck cancer, cervical cancer, endometrial cancer, rectal cancer, liver cancer, kidney cancer, esophageal adenocarcinoma, squamous cell esophageal carcinoma, prostate cancer, female reproductive tract cancer, carcinoma in situ, lymphoma, neurofibroma, thyroid cancer, bone cancer, skin cancer, brain cancer, colon cancer, testicular cancer, gastrointestinal stromal tumor, mast cell tumor, multiple myeloma, melanoma, glioma, or sarcoma.

**17.** The compound or pharmaceutically acceptable salt, ester, stereoisomer, polymorph, solvate, N-oxide, isotopically labeled compound, metabolite and prodrug thereof according to any one of claims 1 to 8, or the antibody-drug conjugate according to any one of claims 8 to 9, or the composition according to any one of claims 10 to 12, or the pharmaceutical composition according to claim 13, or the kit according to claim 14, for use in treating a disease associated with abnormal cell proliferation;

preferably, the disease is a tumor, such as an advanced solid tumor;
preferably, the tumor is selected from the group consisting of lung cancer, squamous cell carcinoma, bladder cancer, gastric cancer, ovarian cancer, peritoneal cancer, pancreatic cancer, breast cancer, head and neck cancer, cervical cancer, endometrial cancer, rectal cancer, liver cancer, kidney cancer, esophageal adenocarcinoma, squamous cell esophageal carcinoma, prostate cancer, female reproductive tract cancer, carcinoma in situ, lymphoma, neurofibroma, thyroid cancer, bone cancer, skin cancer, brain cancer, colon cancer, testicular cancer, gastrointestinal stromal tumor, mast cell tumor, multiple myeloma, melanoma, glioma, or sarcoma.

**INTERNATIONAL SEARCH REPORT**

International application No. **PCT/CN2023/092019**

**A. CLASSIFICATION OF SUBJECT MATTER**

C07D491/22(2006.01)i; C07D491/147(2006.01)i; A61K31/437(2006.01)i; A61P35/00(2006.01)i

According to International Patent Classification (IPC) or to both national classification and IPC

**B. FIELDS SEARCHED**

Minimum documentation searched (classification system followed by classification symbols)

IDC:C07D,A61K,A61P

Documentation searched other than minimum documentation to the extent that such documents are included in the fields searched

Electronic data base consulted during the international search (name of data base and, where practicable, search terms used)

cntxt, ven, cnki, 万方, WANFANG, registry, caplus, marpat: 喜树碱, camptothecin, cpt, 癌症, 肿瘤, cancer, tumor, structure search

**C. DOCUMENTS CONSIDERED TO BE RELEVANT**

| Category* | Citation of document, with indication, where appropriate, of the relevant passages | Relevant to claim No. |
|---|---|---|
| PX | WO 2022198232 A1 (SEAGEN INC.) 22 September 2022 (2022-09-22)<br>see entire document, in particular description, page 252, table 1 | 1-2, 5-15, 17 |
| PX | WO 2023020605 A1 (JIANGSU SIMCERE PHARM CO., LTD.) 23 February 2023 (2023-02-23)<br>see entire document, in particular description, page 13 | 1-5, 13-15, 17 |
| PX | WO 2023030364 A1 (SHANGHAI BEST LINK BIOSCIENCE LLC) 09 March 2023 (2023-03-09)<br>see entire document, in particular description, pages 17 and 138, and claims 12 and 14 | 1-5, 13-15, 17 |
| PX | WO 2022170971 A1 (MEDILINK THERAPEUTICS SUZHOU CO., LTD.) 18 August 2022 (2022-08-18)<br>see entire document, in particular claims 11, 14, 23, 32 and 49, description, page 79, paragraph 1 and page 169, paragraph 2 | 1-15, 17 |
| X | WO 9638146 A1 (SMITHKLINE BEECHAM CORP. et al.) 05 December 1996 (1996-12-05)<br>see description, page 7, scheme 1 | 1-2 |
| PX | WO 2022246576 A1 (ZYMEWORKS INC.) 01 December 2022 (2022-12-01)<br>see entire document, in particular description, page 51 | 1-15, 17 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **15 August 2023** | **22 August 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
**PCT/CN2023/092019**

**C. DOCUMENTS CONSIDERED TO BE RELEVANT**

| Category* | Citation of document, with indication, where appropriate, of the relevant passages | Relevant to claim No. |
|---|---|---|
| X | WO 2022068878 A1 (DUALITY BIOLOGICS SUZHOU CO., LTD.) 07 April 2022 (2022-04-07)<br>see description, page 68, page 100 | 1-15, 17 |
| A | LI, Wei et al. "Synthesis and Evaluation of Camptothecin Antibody-Drug Conjugates" *ACS Med. Chem. Lett.,* Vol. vol. 10, 06 September 2019 (2019-09-06),<br>pp. 1386-1392 | 1-15, 17 |

Form PCT/ISA/210 (second sheet) (July 2022)

**INTERNATIONAL SEARCH REPORT**

International application No.
**PCT/CN2023/092019**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **16**
because they relate to subject matter not required to be searched by this Authority, namely:

The subject matter of claim 16 relates to a method for treatment of the human or animal body, which falls within the cases set forth in PCT Rule 39.1(iv) for which a search is not required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/092019**

| Patent document cited in search report | | | Publication date (day/month/year) | Patent family member(s) | | | Publication date (day/month/year) |
|---|---|---|---|---|---|---|---|
| WO | 2022198232 | A1 | 22 September 2022 | None | | | |
| WO | 2023020605 | A1 | 23 February 2023 | None | | | |
| WO | 2023030364 | A1 | 09 March 2023 | None | | | |
| WO | 2022170971 | A1 | 18 August 2022 | AU | 2022220512 | A1 | 13 July 2023 |
| | | | | TW | 202241521 | A | 01 November 2022 |
| WO | 9638146 | A1 | 05 December 1996 | US | 5663177 | A | 02 September 1997 |
| | | | | JPH | 11506453 | A | 08 June 1999 |
| | | | | EP | 0871447 | A1 | 21 October 1998 |
| | | | | EP | 0871447 | A4 | 23 June 1999 |
| WO | 2022246576 | A1 | 01 December 2022 | CA | 3177067 | A1 | 01 December 2022 |
| WO | 2022068878 | A1 | 07 April 2022 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210521215
- WO 2022253035 A
- WO 2022166762 A1


### Non-patent literature cited in the description


- *Cancer Res.*, 1989, vol. 49, 6365
- *Nature Review Cancer.*, 2006, vol. 6, 789
- *Med. Res. Rev.*, 2015, vol. 35, 753
- **STAHL** ; **WERMUTH**. Handbook of Pharmaceutical Salts: Properties, Selection, and Use. Wiley-VCH, 2002
- Design of Prodrug. Elsevier, 1985
- Protective Groups in Organic Chemistry. Plenum Press, 1973
- **T.W. GREENE** ; **P.G.M. WUTS**. Protective Groups in Organic Synthesis. John Wiley & Sons, 1991